(19) Europäisches Patentamt
European Patent Office
Office européen des brevets

(11) **EP 2 942 027 B1**

(12) **EUROPÄISCHE PATENTSCHRIFT**

(45) Veröffentlichungstag und Bekanntmachung des Hinweises auf die Patenterteilung:
**26.06.2019 Patentblatt 2019/26**

(21) Anmeldenummer: **14004411.6**

(22) Anmeldetag: **28.06.2013**

(51) Int Cl.:
*A61B 34/30* (2016.01)    *A61B 46/10* (2016.01)
*A61B 90/00* (2016.01)    *A61B 34/00* (2016.01)
*A61B 17/00* (2006.01)

(54) **CHIRURGISCHE INSTRUMENTENANORDNUNG UND ANTRIEBSSTRANGANORDNUNG FÜR EIN, INSBESONDERE ROBOTERGEFÜHRTES, CHIRURGISCHES INSTRUMENT UND CHIRURGISCHES INSTRUMENT**

SURGICAL INSTRUMENTATION AND DRIVETRAIN ASSEMBLY FOR A SURGICAL INSTRUMENT, IN PARTICULAR A ROBOT-CONTROLLED INSTRUMENT AND SURGICAL INSTRUMENT

SYSTEME D'INSTRUMENT CHIRURGICAL ET DE TRANSMISSION POUR UN INSTRUMENT CHIRURGICAL, NOTAMMENT ROBOTISE ET INSTRUMENT CHIRURGICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.07.2012 DE 102012013242**
**11.03.2013 DE 102013004230**
**14.03.2013 DE 102013004487**
**28.03.2013 DE 102013005493**
**06.05.2013 DE 102013007761**

(43) Veröffentlichungstag der Anmeldung:
**11.11.2015 Patentblatt 2015/46**

(62) Dokumentnummer(n) der früheren Anmeldung(en) nach Art. 76 EPÜ:
**13739941.6 / 2 869 779**

(73) Patentinhaber: **KUKA Deutschland GmbH**
**86165 Augsburg (DE)**

(72) Erfinder:
• **Lohmeier, Sebastian, Dr.**
**80639 München (DE)**
• **Schober, Wolfgang**
**86554 Pöttmes (DE)**
• **Brudniok, Sven, Dr.**
**86853 Langerringen (DE)**

(74) Vertreter: **Schlotter, Alexander Carolus Paul et al**
**Wallinger Ricker Schlotter Tostmann**
**Patent- und Rechtsanwälte Partnerschaft mbB**
**Zweibrückenstrasse 5-7**
**80331 München (DE)**

(56) Entgegenhaltungen:
**WO-A2-2010/123231    WO-A2-2011/037394**
**DE-A1-102009 060 987    DE-A1-102010 027 248**
**US-A- 2 227 727    US-A1- 2008 140 088**
**US-A1- 2011 277 775    US-B1- 6 594 552**
**US-B1- 6 656 196**

**Beschreibung**

Ein Aspekt der vorliegenden Erfindung betrifft ein, insbesondere robotergeführtes, chirurgisches Instrument mit einem Instrumentenschaft mit wenigstens einem Freiheitsgrad und einer Antriebseinheit zu dessen Aktuierung sowie einen Instrumentenschaft und eine Antriebseinheit.

Aus der WO 2011/143022 A1 ist ein robotergeführtes chirurgisches Instrument mit vier Antriebseinheiten bekannt, die tortenstückartig an einer Basisplatte angeordnet sind und jeweils mehrere Antriebsmodule aufweisen. Die Antriebsmodule weisen jeweils mehrere verschieb- oder drehbare Abtriebsglieder zum Aktuieren von Eintriebsgliedern eines mit der Antriebseinheit verbundenen Instrumentenschaftes auf.

Die Antriebseinheiten sind jeweils gegen die Basisplatte aktiv teleskopierbar, um ihren Instrumentenschaft durch eine gemeinsame Führungskanüle ein- bzw. auszufahren. Die Abtriebsglieder stehen zu den Eintriebsgliedern hin distal bzw. in einer Koppelrichtung vor, die zur Längsachse des Instrumentenschaftes parallel ist, und sind in dieser distalen Richtung elastisch vorgespannt, um einen spielfreien Kontakt sicherzustellen.

Aus der WO 2010/123231 A2 ist eine Kopplungsstruktur zur Montage eines chirurgischen Instruments an einem Adapter bekannt, der an einer Stirnseite eines Roboterarms angeordnet ist, wobei die Kopplungsstruktur ein tafelartiges Gehäuse, das eine vorbestimmte Dicke aufweist und an der Stirnseite des Roboterarms montiert ist, eine Schnittstelleneinheit, die in dem Bereich der vorbestimmte Dicke des Gehäuses ausgebildet und mit dem Adapter gekoppelt ist, eine Mehrzahl von Antreibern, die an der Schnittstelleneinheit montiert sind, um durch Antriebskräfte aktuiert zu werden, die durch den Adapter übertragen werden, einen mit dem Gehäuse gekoppelten Schaft, der ein mit den Antreibern verbundenes Leistungsübertragungsmittel darin aufnimmt, und einen Endeffektor aufweist, der mit dem Ende des Schafts gekoppelt und mit dem Leistungsübertragungsmittel verbunden ist, um für Chirurgie erforderliche Operationen in Übereinstimmung mit den Operationen der Antreiber auszuführen. WO2011/037394 offenbart einen Instrumentenschaft, der über einen Adapter an einem Roboterarm gehalten wird.

Eine Aufgabe eines Aspekts der vorliegenden Erfindung ist es, ein verbessertes chirurgisches Instrument zur Verfügung zu stellen.

Diese Aufgabe wird durch ein chirurgisches Instrument mit den Merkmalen des Anspruchs 4 gelöst. Anspruch 1 stellt einen Instrumentenschaft für ein chirurgisches Instrument unter Schutz. Die Unteransprüche betreffen vorteilhafte Weiterbildungen.

Ein weiterer Aspekt der vorliegenden Offenbarung betrifft eine chirurgische Instrumentenanordnung, ein Manipulatorchirurgiesystem mit einer solchen manipulatorgeführten Instrumentenanordnung sowie ein Verfahren zum Bestücken eines Manipulators eines solchen.

Beispielsweise aus der EP 1 015 068 A1 ist ein Manipulatorchirurgiesystem mit einem manipulatorgeführten chirurgischen Instrument bekannt, dessen Freiheitsgrade durch eine Antriebsstranganordnung im Manipulator aktuiert werden, was insbesondere in Hinblick auf Sterilitätsanforderungen die Anbindung des Instruments an den Manipulator erschwert.

Die DE 10 2009 060 987 A1 offenbart ein chirurgisches Manipulatorinstrument mit einer eigenen Antriebseinheit zur Aktuierung von Freiheitsgraden des Instruments, das eine mechanische Schnittstelle mit einem Kupplungselement aufweist, das in eine Hinterschnitt-Ausbuchtung eines weiteren Kupplungselements eingreift, ohne auf Sterilitätsanforderungen einzugehen.

Eine Aufgabe eines Aspekts der vorliegenden Offenbarung ist es, ein verbessertes chirurgisches Instrument zur Verfügung zu stellen.

Ein weiterer Aspekt der vorliegenden Offenbarung betrifft eine Antriebsstranganordnung zur Aktuierung wenigstens eines Freiheitsgrades eines Endeffektors eines, insbesondere robotergeführten, chirurgischen Instruments, ein Antriebsmodul und einen Instrumentenschaft eines solchen Instruments, ein Instrument mit einem solchen Instrumentenschaft und/oder Antriebsmodul, eine Manipulatoranordnung mit wenigstens einem solchen Instrument, welches durch einen Manipulator geführt ist, sowie ein Verfahren und ein Steuermittel zum Steuern eines solchen Instruments, insbesondere seines Antriebs und/oder eines manuellen Teleoperationsmittels.

Ein robotergeführtes minimalinvasives chirurgisches Instrument weist allgemein einen Instrumentenschaft auf. Bei durch einen Trokar teileingeführtem Instrumentenschaft kann ein distales bzw. intrakorporales Instrumentenschaftende noch in maximal vier Freiheitsgraden durch den Roboter bewegt werden (drei Drehachsen durch den Trokarpunkt und eine Translation in Richtung der Schaftachse).

Um in einem minimalinvasiven Operationsgebiet mehr Freiheitsgrade zur Verfügung zu haben, ist es aus der WO 2009/079301 A1 bekannt, einen Endeffektor gelenkig an dem distalen Instrumentenschaftende zu lagern und durch eine Antriebsstranganordnung zusätzlich zu aktuieren. Beispielsweise kann so eine Klemme geschlossen oder eine Endoskop-Optik umorientiert werden.

Um einem Teleoperateur, der den Chirurgieroboter bedient, eine haptische Rückmeldung aus dem Operationsgebiet zu geben, schlägt die WO 2009/079301 A1 vor, zwischen Instrumentenschaft und Endeffektorlager einen sechsachsigen Kraft-Momenten-Sensor anzuordnen.

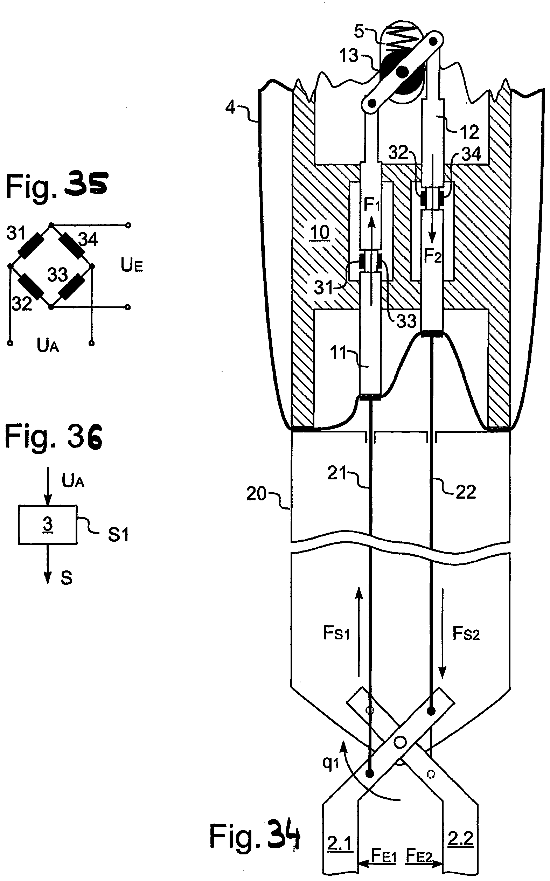

Ein Nachteil dieser Lösung lässt sich anschaulich anhand der Fig. 34 erläutern: dort ist, wie nachfolgend näher beschrieben, ein Instrumentenschaft 20 gezeigt, an dem ein Endeffektor in Form einer Klemme mit zwei Klingen 2.1, 2.2 angeordnet ist. Die Klinge 2.1 ist durch zwei gegensinnige Antriebsstränge 21, 22 in ihrem Drehfreiheitsgrad $q_1$ gegen den Instrumentenschaft verstellbar, die Klinge 2.2 entsprechend. Greift die Klemme an einem Lumen (nicht dargestellt) an, wirken auf sie die Reaktionskräfte $F_{E1}$ bzw. $F_{E2}$. Diese Kräfte bewirken in der in Fig. 34 dargestellten Konstellation keine Kräfte im Instrumentenschaft 20, da ihre Resultierende verschwindet. Entsprechend kann ein Kraft-Momenten-Sensor, wie ihn die WO 2009/079301 A1 vorschlägt, einem Teleoperateur keine Rückmeldung über die von der Klemme ausgeübten Kräfte vermitteln, da er unabhängig von den tatsächlich aufgeprägten Klemmkräften $F_{E1}$, $F_{E2}$ keine Kräfte oder Momente im Instrumentenschaft erfasst.

Eine Aufgabe eines Aspekts der vorliegenden Offenbarung ist es, ein verbessertes chirurgisches Instrument zur Verfügung zu stellen und/oder dessen Steuerung zu verbessern.

Ein weiterer Aspekt der vorliegenden Erfindung betrifft ein, insbesondere robotergeführtes und/oder minimalinvasives, chirurgisches Instrument sowie ein Antriebsmodul und einen Instrumentenschaft eines solchen Instruments und ein Verfahren zu deren Verbinden.

Beispielsweise aus der WO 2011/143022 A1 ist ein robotergeführtes minimalinvasives Instrument mit einem Instrumentenschaft bekannt, der durch einen Roboter durch eine natürliche oder künstliche kleine Öffnung in den Patienten eingeführt wird. Zur Aktuierung intrakorporaler Freiheitsgrade, insbesondere eines Endeffektors, ist ein extrakorporales Antriebsmodul lösbar mit dem Instrumentenschaft verbunden.

Eine Aufgabe eines Aspekts der vorliegenden Offenbarung ist es, ein vorteilhaftes chirurgisches Instrument zur Verfügung zu stellen.

Nach einem Aspekt der vorliegenden Offenbarung weist eine, insbesondere manipulatorgeführte, chirurgische Instrumentenanordnung eine modulare motorische Antriebseinheit auf, die eine Abtriebsanordnung mit einem oder mehreren Abtriebselementen aufweist. Unter einem Abtriebselement wird vorliegend insbesondere ein ein- oder mehrteiliges Element bzw. Bauteil verstanden, welches durch einen Motor, insbesondere einen Elektromotor, der Antriebseinheit direkt oder indirekt akuier- bzw. in einer Verstellrichtung verstellbar und dazu vorgesehen ist, einen Freiheitsgrad des Instruments zu aktuieren. Die Antriebseinheit kann in einer Ausführung kabellos oder kabelbasiert mit Energie versorgt und/oder gesteuert werden.

Die Instrumentenanordnung weist weiter einen Instrumentenschaft auf, der in einer Ausführung dazu vorgesehen ist, teilweise in einen Patienten eingeführt zu werden, insbesondere durch eine Öffnung zur minimalinvasiven Chirurgie, insbesondere auch Endoskopie. Der Instrumentenschaft kann ganz oder teilweise steif oder flexibel ausgebildet sein und/oder einen Endeffektor aufweisen, insbesondere ein Skalpell, eine Schere, Zange, Klemme, ein optisches Aufnahme- und/oder Beleuchtungsmittel, insbesondere ein Lichtleiter-Ende, ein CCD-Chip (sogenanntes "chip-on-the-tip Endoskop), eine LED oder dergleichen. Insofern kann insbesondere auch ein aktuierbares, insbesondere abwinkelbares, Endoskop einen Instrumentenschaft im Sinne der vorliegenden Offenbarung darstellen. Ein Instrumentenschaft im Sinne der vorliegenden Erfindung weist allgemein einen oder mehrere Freiheitsgrade auf, insbesondere einen oder mehrere Freiheitsgrade zum Positionieren, insbesondere Orientieren, und/oder zum Betätigen eines Endeffektors. In einer Weiterbildung weist er zwei, drei oder mehr Freiheitsgrade, insbesondere Drehfreiheitsgrade, zum Orientieren und/oder einen oder mehrere, insbesondere maximal einen, Freiheitsgrad zum Betätigen, insbesondere Öffnen bzw. Schließen, eines Endeffektors auf. Zur Aktuierung weist er eine Antriebsanordnung mit einem oder mehreren Eintriebselementen auf. Unter einem Eintriebselement wird vorliegend insbesondere ein ein- oder mehrteiliges Element bzw. Bauteil verstanden, welches durch ein zugeordnetes Abtriebselement der Antriebseinheit direkt oder indirekt akuier- bzw. in einer Verstellrichtung verstellbar und dazu vorgesehen ist, einen Freiheitsgrad des Instruments zu aktuieren. Es kann hierzu insbesondere uni- oder bidirektional mit dem Endeffektor gekoppelt sein, in einer Ausführung mechanisch, insbesondere mittels eines oder mehrerer Seilzüge, Stangen, Zahnräder, hydraulisch, pneumatisch oder dergleichen, wobei unter einer unidirektionalen Koppelung insbesondere eine Koppelung derart verstanden wird, dass der Freiheitsgrad durch eine Verstellung des Eintriebselements nur in einem Richtungssinn aktuierbar ist, beispielsweise durch einen Seilzug nur in einer Zugrichtung, unter einer bidirektionalen Koppelung entsprechend insbesondere eine Koppelung derart, dass der Freiheitsgrad durch eine Verstellung des Eintriebselements gegensinnig aktuierbar ist, beispielsweise durch eine Schubstange in Zug- und Druckrichtung.

Der Instrumentenschaft ist lösbar mit der Antriebseinheit verbindbar, die Abtriebsanordnung und die Antriebsanordnung mittels einer mechanischen Schnittstelle miteinander koppelbar. In einer Weiterbildung ist der Instrumentenschaft lösbar mit der Antriebseinheit verbunden, die Abtriebsanordnung und die Antriebsanordnung mittels der mechanischen Schnittstelle miteinander gekoppelt. Die Instrumentenanordnung wird dann auch kurz als Instrument bezeichnet. In einer Ausführung können jedoch auch zwei oder mehr verschiedene Antriebseinheiten und/oder zwei oder mehr verschiedene Instrumentenschäfte vorgesehen sein, die wahlweise mit einem Instrumentenschaft bzw. einer Antriebseinheit verbindbar sind und sich insbesondere in der Anzahl der aktuierbaren Freiheitsgrade unterscheiden können. Zur kompakteren Darstellung wird daher vorliegend im Allgemeinen von einer Instrumentenanordnung im Sinne eines Satzes von einer oder mehr Antriebseinheiten und einem oder mehr Instrumentenschäften gesprochen, die lösbar miteinander verbindbar bzw. verbunden sind.

Die Instrumentenanordnung bzw. das Instrument, insbesondere die Antriebseinheit oder der Instrumentenschaft, ist in einer Ausführung lösbar an einem Manipulator befestigt und kann hierzu in einer Weiterbildung eine entsprechende Anbindungsschnittstelle aufweisen. Entsprechend betrifft ein Aspekt der vorliegenden Offenbarung ein Verfahren zum Bestücken eines Manipulators, wobei eine Antriebseinheit und ein Instrumentenschaft lösbar miteinander verbunden und ihre Ab- und die Antriebsanordnung mittels der mechanischen Schnittstelle miteinander gekoppelt werden. Der Manipulator kann in einer Ausführung ein oder mehrere, insbesondere wenigstens sechs, vorzugsweise sieben oder mehr Freiheitsgrade aufweisen, um das Instrument (redundant) zu führen, insbesondere seinen Endeffektor in einem Patienten zu positionieren.

Ein Gesichtspunkt der vorliegenden Offenbarung betrifft die Ausgestaltung der mechanischen Schnittstelle, mittels der Ab- und Antriebsanordnung miteinander koppelbar bzw. gekoppelt sind.

Nach einem Aspekt weist diese Schnittstelle je eine einseitige Bindung zwischen einem oder mehreren Paaren einander zugeordneter Ab- und Eintriebselemente auf. Unter einer einseitigen Bindung bzw. Koppelung wird vorliegend insbesondere, wie in der Mechanik üblich, verstanden, dass ein Ab- und ein Eintriebselement derart miteinander gekoppelt sind, dass eine Bewegung des einen von dem Ab- und dem Eintriebselement in einer Richtung bzw. einem Richtungssinn zwangsgeführt eine Bewegung des anderen von dem Ab- und dem Eintriebselement bewirkt, eine Bewegung des einen von dem Ab- und dem Eintriebselement in der entgegengesetzten Richtung bzw. dem Gegensinn hingegen keine zwangsgeführte Bewegung des anderen von dem Ab- und dem Eintriebselement bewirkt. Insbesondere kann eine einseitige Bindung dadurch gekennzeichnet sein, dass zwischen Ab- und Eintriebselement direkt oder indirekt nur Druckkräfte und keine Zugkräfte übertragbar sind, wobei vorliegend zur kompakteren Darstellung auch antiparallele Kräftepaare, d.h. Drehmomente, verallgemeinernd als Kräfte bezeichnet werden. Eine einseitige Bindung kann entsprechend dadurch gekennzeichnet sein, dass zwischen Ab- und Eintriebselement direkt oder indirekt nur ein Drehmoment in einer Richtung übertragbar ist, in der Gegenrichtung hingegen, wenigstens im Wesentlichen, kein Drehmoment übertragbar ist. Unter einer zweiseitigen Bindung wird vorliegend entsprechend insbesondere verstanden, dass gegensinnige Bewegungen von Ab- oder Eintriebselement zwangsgeführt auf das jeweils andere Element übertragen werden, insbesondere, dass zwischen Ab- und Eintriebselement direkt oder indirekt Druck- und Zugkräfte bzw. gegensinnige Drehmomente übertragbar sind.

Eine solche einseitige Bindung kann vorteilhaft über eine Sterilbarriere hinweg wirken. Insbesondere können in einer Ausführung ein Ab- und ein zugeordnetes Eintriebselement auf gegenüberliegenden Seiten einer Sterilbarriere angeordnet sein und diese kontaktieren, wobei wenigstens eines von dem Ab- und Eintriebselement nicht mit der Sterilbarriere verbunden ist bzw. sich von dieser lösen kann. Auf diese Weise kann einfach und kompakt eine Sterilbarriere zwischen der Antriebseinheit und dem Instrumentenschaft angeordnet werden.

Nach einem Aspekt weist die mechanische Schnittstelle, mittels der Ab- und Antriebsanordnung miteinander koppelbar bzw. gekoppelt sind, je wenigstens eine Aussparung, die in einem Element von einem oder mehreren Paaren einander zugeordneter Ab- und Eintriebselemente ausgebildet ist, und je einen Zapfen auf, der an dem anderen Element dieses Paares ausgebildet und in diese Aussparung einführbar bzw. eingeführt ist. Insbesondere können also ein oder mehrere Abtriebselemente je einen oder mehrere Zapfen und ihnen zugeordnete Eintriebselemente entsprechende Aussparungen aufweisen. Gleichermaßen können ein oder mehrere Eintriebselemente je einen oder mehrere Zapfen und ihnen zugeordnete Abtriebselemente entsprechende Aussparungen aufweisen.

Nach einem Aspekt sind der bzw. die Zapfen in der jeweiligen Aussparung radial, insbesondere elastisch und/oder durch separate Körper, aufweitbar bzw. aufgeweitet, so dass der Zapfen in der Aussparung, insbesondere axial- und/oder drehfest, fixierbar bzw. fixiert ist. In einer Ausführung kann ein Zapfen durch das radiale Aufweiten reibschlüssig in der Aussparung fixiert werden bzw. sein. Zusätzlich oder alternativ kann der Zapfen durch das radiale Aufweiten formschlüssig in der Aussparung fixiert werden bzw. sein. Eine Sterilbarriere kann insbesondere zwischen Zapfen und Aussparung angeordnet, insbesondere geklemmt, sein bzw. werden und so einfach und kompakt zwischen der Antriebseinheit und dem Instrumentenschaft angeordnet werden.

In einer Ausführung ist ein Klemmmittel zum radialen Aufweiten eines oder mehrerer in je eine Aussparung eingeführter Zapfen der mechanischen Schnittstelle vorgesehen. Dieses Klemmmittel kann in einer Weiterbildung manuell oder mechanisch aktuiert werden, insbesondere durch einen eigenen, vorzugsweise elektromotorischen, Klemmmittelantrieb. Es kann insbesondere mechanisch, hydraulisch, pneumatisch und/oder elektromagnetisch aktuiert werden. Beispielsweise kann ein Klemmmittelantrieb derart weg- oder kraftgesteuert werden, dass er nach einem Einführen des Zapfens in die Aussparung, insbesondere durch eine Verstellung bzw. Aktuierung der Antriebsanordnung, diesen radial aufweitet. Der Zapfen kann ein- oder mehrteilig mit dem Ab- bzw. Eintriebselement ausgebildet sein, insbesondere als separater und/oder elastischer Körper, der mit dem übrigen Ab- bzw. Eintriebselement lösbar oder unlösbar, insbesondere stoffschlüssig verbunden, vorzugsweise verklebt sein kann.

Zur elastischen radialen Aufweitung kann der Zapfen, insbesondere dessen elastischer Körper, in einer Weiterbildung aus Kunststoff, insbesondere Polyurethan und/oder Silikon hergestellt sein. Zur nicht-elastischen Aufweitung kann der Zapfen ein oder mehrere separate, insbesondere lamellenartige, Körper aufweisen, die radial verschieblich, insbesondere um eine Achse radial nach außen verschwenkbar oder translatorisch verschiebbar, in dem Zapfen bzw. übrigen Ab- bzw. Eintriebselement geführt sind und durch radiale Verschiebung nach außen, insbesondere durch Verschwenken, dieses im Sinne der vorliegenden Offenbarung radial aufweiten können.

In einer Ausführung kann der Zapfen eine durchgehende oder sacklochartige Innenbohrung aufweisen, die beispielsweise hydraulisch oder pneumatisch mit Druck beaufschlagt wird, um den Zapfen radial aufzuweiten. Ein Stift des Klemmmittels kann eine Durchgangsbohrung in dem Zapfen durchgreifen und auf einer einem Klemmmittelantrieb gegenüberliegenden Seite einen Flansch aufweisen, dessen Durchmesser größer ist als die Durchgangsbohrung. Durch Spannen des Flansches durch den Klemmmittelantrieb zu diesem hin kann der Zapfen zwischen Flansch und Klemmmittelantrieb axial komprimiert werden, so dass dieser sich radial aufweitet. Gleichermaßen kann der Stift eine sich in axialer Richtung radial erweiternde, insbesondere konische, Kontur aufweisen, so dass eine axiale Verschiebung des Stiftes im Zapfen diesen radial aufweitet, insbesondere elastisch oder durch Verschieben von separaten Körpern radial nach außen.

Nach einem anderen Aspekt ist zwischen der Aussparung und dem in diese eingeführten Zapfen ein in radialer Richtung wellenförmiger Spalt ausgebildet, in dem eine Zwischenelementanordnung mit einem oder mehreren Zwischenelementen angeordnet ist, die - insbesondere durch einen fest mit der Antriebseinheit oder dem Instrumentenschaft verbundenen Käfig - radial verschiebbar und axial fest geführt sind. Wird nun der Zapfen (die Aussparung) axial verschoben, verschiebt sich entsprechend auch dessen (deren) wellenförmige, der Aussparung (dem Zapfen) zugewandte Außen(Innen)wand. Dies verstellt formschlüssig die entsprechenden Zwischenelemente in radialer Richtung, die ihrerseits formschlüssig eine entsprechende Axialverschiebung der Aussparung (des Zapfens) erzwingen. Auf diese Weise kann insbesondere eine Axialverschiebung von Zapfen bzw. Aussparung auf die Aussparung bzw. den Zapfen formschlüssig zwangsgeführt übertragen werden. Eine Sterilbarriere kann wiederum insbesondere zwischen Zapfen und Aussparung, insbesondere zwischen Zapfen und Zwischenelementanordnung oder zwischen Zwischenelementanordnung und Aussparung angeordnet, insbesondere geklemmt, sein bzw. werden und so einfach und kompakt zwischen der Antriebseinheit und dem Instrumentenschaft angeordnet werden.

Nach einem Aspekt weist die mechanische Schnittstelle je einen Kipphebel zur Koppelung von einem oder mehreren Paaren von Ab- und Eintriebselementen auf. Unter einem Kipphebel wird vorliegend in fachüblicher Weise insbesondere ein Hebel verstanden, welcher an einer Stelle, insbesondere einem Ende, drehbar gelagert und an einer hiervon axial beabstandeten Stelle, insbesondere einem gegenüberliegenden Ende, formschlüssig durch eine dreh- oder verschiebbare Kulisse zwangsgeführt wird. Eine Sterilbarriere kann insbesondere zwischen Kipphebel und Kulisse angeordnet, insbesondere geklemmt, sein bzw. werden und so einfach und kompakt zwischen der Antriebseinheit und dem Instrumentenschaft angeordnet werden. Insbesondere kann ein Abtriebselement als Kipphebel und ein zugeordnetes Eintriebselement als Kulisse ausgebildet sein. Gleichermaßen kann ein Eintriebselement als Kipphebel und ein zugeordnetes Abtriebselement als Kulisse ausgebildet sein.

In einer Ausführung eines der vorgenannten Aspekte können ein oder mehrere Abtriebselemente der Abtriebsanordnung translatorisch verstellbar geführt bzw. aktuierbar sein. Beispielsweise kann ein Abtriebselement eine Abtriebsachse eines Linearmotors bilden oder mit einer solchen gekoppelt sein. Zusätzlich oder alternativ können ein oder mehrere Eintriebselemente der Antriebsanordnung translatorisch verstellbar geführt bzw. aktuierbar sein. Beispielsweise kann ein Eintriebselement eine Stange, die mit einem Endeffektor gelenkig verbunden ist, bilden oder mit einer solchen gekoppelt sein. Gleichermaßen kann ein Eintriebselement auch beispielsweise mit einem Zugseil zur Aktuierung eines Instrumentenfreiheitsgrades verbunden sein.

Gleichermaßen können ein oder mehrere Abtriebselemente der Abtriebsanordnung rotatorisch verstellbar geführt bzw. aktuierbar sein. Beispielsweise kann ein Abtriebselement eine Abtriebsachse eines Drehmotors bilden oder mit einer solchen gekoppelt sein. Zusätzlich oder alternativ können ein oder mehrere Eintriebselemente der Antriebsanordnung rotatorisch verstellbar geführt bzw. aktuierbar sein. Beispielsweise kann ein Eintriebselement eine Welle sein, auf die ein Zugseil zur Aktuierung eines Instrumentenfreiheitsgrades aufwickelt.

In einer Ausführung eines der vorgenannten Aspekte sind ein oder mehr Abtriebselemente derart mit einem Koppelmittel gekoppelt, dass eine translatorische Bewegung von dem Koppelmittel in eine rotatorische Bewegung von dem Element umgesetzt wird. Gleichermaßen können ein oder mehr Abtriebselemente derart mit einem Koppelmittel gekoppelt sein, dass eine rotatorische Bewegung von dem Koppelmittel in eine translatorische Bewegung von dem Element umgesetzt wird. Zusätzlich oder alternativ können ein oder mehr Eintriebselemente derart mit einem (weiteren) Koppelmittel gekoppelt sein, dass eine translatorische Bewegung von dem Element in eine rotatorische Bewegung von dem Koppelmittel umgesetzt wird. Gleichermaßen können ein oder mehr Eintriebselemente derart mit einem (weiteren) Koppelmittel gekoppelt sein, dass eine rotatorische Bewegung von dem Element in eine translatorische Bewegung von dem Koppelmittel umgesetzt wird.

In einer Weiterbildung kann ein Koppelmittel ein Drehschublager aufweisen, insbesondere ein in einer Kulisse translatorisch verschiebbares Drehgelenk. Zusätzlich oder alternativ kann ein Koppelmittel einen drehbar gelagerten Hebel oder eine drehbar gelagerte Wippe bzw. einen Hebel mit einem Drehlagerpunkt aufweisen, der zwischen zwei Abgriffen, beispielsweise weiteren Drehlagerpunkten, Seilbefestigungen oder dergleichen angeordnet ist. Insbesondere auf diese Weise kann eine rotatorische Bewegung des Koppelmittels mechanisch in eine translatorische Bewegung eines Ab- oder Eintriebselements umgesetzt werden. Zusätzlich oder alternativ kann ein Koppelmittel eine Verzahnung aufweisen, insbesondere zwei ineinandergreifende bzw. miteinander kämmende Verzahnungen, von denen in einer Weiterbildung eine rotatorisch und die andere ebenfalls rotatorisch, insbesondere als kämmende Stirnverzahnung, oder translatorisch, insbesondere als Schnecken- bzw. Zahnstangenverzahnung, beweglich gelagert ist.

In einer Ausführung eines der vorgenannten Aspekte weist der Instrumentenschaft einen Flansch auf, wobei die mechanische Schnittstelle auf einer endeffektorzugewandten Fläche dieses Flansches angeordnet ist. In einer Weiterbildung weist die Antriebseinheit eine entsprechende Aussparung zur Durchführung des Instrumentenschaftes auf, was vorliegend auch als Hinterlader-Anordnung bezeichnet wird. Gleichermaßen kann die mechanische Schnittstelle auf einer endeffektorabgewandten Fläche dieses Flansches angeordnet sein, so dass die Antriebseinheit ebenfalls auf einer endeffektorabgewandten Seite des Instrumentenschaftes angeordnet sein kann, was vorliegend auch als Vorderlader-Anordnung bezeichnet wird. In einer alternativen Gestaltung kann die mechanische Schnittstelle auf einer seitlichen Fläche des Flansches des Instrumentenschaftes angeordnet sein, was vorliegend auch als Seitenlader-Anordnung bezeichnet wird. Insbesondere, wenn die mechanische Schnittstelle eine einseitige Bindung aufweist, können in einer Ausführung ein oder mehrere Abtriebselemente und/oder ein oder mehrere Eintriebselemente entgegen ihrer jeweiligen Verstellrichtung vorgespannt sein, insbesondere durch ein Federmittel. Auf diese Weise kann auch bei einer einseitigen Bindung ein Element dem anderen durch das Federmittel auch entgegen der Bindungsrichtung nachgeführt werden. Auch bei zweiseitigen Bindungen, beispielsweise einem radial aufgeweiteten Zapfen, einer Zwischenelementanordnung oder einem Kipphebel, kann eine Vorspannung eines Ab- oder Eintriebselementes entgegen seiner Verstellrichtung vorteilhaft Spiel reduzieren.

Gleichermaßen kann, insbesondere, wenn zwischen einem Ab- und einem zugeordneten Eintriebselement eine einseitige Bindung ausgebildet ist, ein weiteres Ab- und zugeordnetes Eintriebselement vorgesehen sein, dessen einseitige Bindung ein zu dem einen Ab- und Eintriebselement gegensinnig ist. Mit anderen Worten kann zur gegensinnigen Aktuierung bzw. Aktuierung eines Freiheitsgrades in zwei entgegengesetzten Richtungen je ein Paar gegensinnig wirkender Ab- bzw. Eintriebselemente vorgesehen sein. Hierunter wird vorliegend insbesondere verstanden, dass eine Aktuierung eines Abtriebselementes eines Paares das andere Abtriebselement dieses Paares, insbesondere mechanisch zwangsgeführt, gegensinnig aktuiert bzw. verstellt. Auch bei zweiseitigen Bindungen, beispielsweise einem radial aufgeweiteten Zapfen, einer Zwischenelementanordnung oder einem Kipphebel, kann ein gegensinnig wirkendes weiteres Ab- und Eintriebselement vorgesehen sein, um vorteilhaft eine redundante und präzise Aktuierung des Freiheitsgrades darzustellen.

Insbesondere, wenn durch ein Paar gegensinnig wirkender Ab- bzw. Eintriebselemente eine statische Überbestimmtheit vorliegt, kann in einer Ausführung der vorliegenden Erfindung ein Ausgleichmittel zum Toleranzausgleich vorgesehen sein. Ein solches Toleranzmittel kann insbesondere eine elastische Nachgiebigkeit eines Ab- oder Eintriebselements, insbesondere in dessen Verstellrichtung, aufweisen. Zusätzlich oder alternativ kann auch ein mit einem Ab- oder Eintriebselement gekoppeltes Koppelmittel eine elastische Nachgiebigkeit in Verstellrichtung des Elements aufweisen. Zusätzlich oder alternativ kann auch eine Sterilbarriere, die zwischen Ab- und Eintriebselement angeordnet ist, eine elastische Nachgiebigkeit aufweisen. Eine elastische Nachgiebigkeit kann insbesondere durch ein elastisches Material, welches im normalen Betrieb makroskopische Deformationen zeigt, und/oder durch eine entsprechende Formgebung bzw. biegeweiche Gestaltung, insbesondere eine lokale Materialverdünnung, vorzugsweise Einschnürung, definiert bzw. gebildet sein. Gleichermaßen kann ein Ausgleichsmittel auch eine, insbesondere vorgespannte, in Verstellrichtung verschiebbare Lagerung bzw. Lagerachse, insbesondere eines mit einem Ab- oder Eintriebselement gekoppelten, Koppelmittels aufweisen. Auch ohne statische Überbestimmtheit kann ein solcher Toleranzausgleich vorteilhaft sein, um beispielsweise Montage- oder Fertigungstoleranzen in einer kinematischen Kette auszugleichen.

In einer Ausführung eines der vorgenannten Aspekte kann eine Stirnseite eines Abtriebselements und/oder eine Stirnseite eines Eintriebselements eben ausgebildet sein, insbesondere, um vorteilhaft eine größere Kontaktfläche darzustellen. Gleichermaßen können eine Stirnseite eines Abtriebselements und/oder eine Stirnseite eines Eintriebselements ballig ausgebildet sein, insbesondere, um vorteilhaft einen wohldefinierten Kontaktbereich darzustellen. Zusätzlich oder alternativ kann eine Stirnseite eines Ab- oder Eintriebselements wenigstens einen Vorsprung und eine dieser zugewandten Stirnseite eines damit koppelbaren bzw. gekoppelten Ein- bzw. Abtriebselements eine entsprechende Aussparung aufweisen, in die dieser Vorsprung eingreift.

Ein weiterer Gesichtspunkt der vorliegenden Offenbarung betrifft die Sterilität des Instruments. Hierzu weist nach einem Aspekt, der mit einem oder mehreren der vorstehenden Aspekte bzw. Ausführungen kombiniert sein kann, die Instrumentenanordnung bzw. das Instrument eine Sterilbarriere auf, die dazu vorgesehen ist, die Antriebseinheit, insbesondere luftdicht, zu umhüllen und zwischen der Antriebseinheit und dem Instrumentenschaft angeordnet zu werden, bzw. welche die Antriebseinheit steril umhüllt und zwischen der Antriebseinheit und dem Instrumentenschaft angeordnet ist. Die Sterilbarriere kann in einer Weiterbildung folienartig und/oder als Einmal- bzw. Wegwerfartikel ausgebildet sein.

Nach einem Aspekt weist die Sterilbarriere im Bereich der mechanischen Schnittstelle eine Lose in einer Verstellrichtung der Ab- und Antriebsanordnung auf. Die Lose kann in einer Ausführung durch eine Stulpe gebildet sein, die sich in einer axialen Verstellrichtung erstreckt und bei einer axialen Verstellung von Ab- bzw.

Eintriebselement auf- bzw. abrollt bzw. umstülpt. Unter Lose wird vorliegend verallgemeinernd insbesondere eine Materialreserve der Sterilbarriere verstanden, um, insbesondere translatorische, Verstellbewegungen von Ab- bzw. Eintriebselementen zu kompensieren bzw. mitzumachen, die in einem Verstellzustand gefaltet bzw. gerollt gespeichert und in einem anderen Verstellzustand enfaltet bzw. entrollt ist.

In einer Ausführung kann die Lose vorgespannt ausgebildet sein. Hierunter wird vorliegend insbesondere verstanden, dass die Lose sich bei einer Verstellbewegung bzw. Aktuierung des Ab- bzw. Eintriebselements gegen die Vorspannung elastisch verformt und bei gegensinniger Bewegung zurückstellt. Insofern wird vorliegend zur kompakteren Darstellung insbesondere ein Mehrmaterial, das zur Kompensation einer Stellbewegung eines Ab- bzw. Eintriebselements vorgesehen ist, verallgemeinernd als Lose bezeichnet, die gleichermaßen vorgespannt oder vorspannungsfrei bzw. lose sein kann. In einer Weiterbildung weist die Lose einen Faltenbalg auf, dessen Faltung eine Vorspannung in eine Grundkonfiguration induziert. Die Faltung bzw. der Faltenbalg kann sich in einer Ausführung in Verstellrichtung und/oder transversal bzw. quer zu dieser erstrecken, wodurch entsprechende Grundkonfigurationen und Verformungen dargestellt werden können.

Nach einem Aspekt weist die Sterilbarriere im Bereich der mechanischen Schnittstelle wenigstens eine berührungslose translatorisch verschiebbare Dichtung auf. Diese kann insbesondere als Spalt - oder Labyrinthdichtung ausgebildet und vorzugsweise teleskopierbar sein, d.h. zwei oder mehr axial gegeneinander verschiebbare und abgedichtete Teile, insbesondere Ringe, vorzugsweise konzentrische Ringe, aufweisen. Vorteilhafterweise können durch solche berührungslose translatorische Dichtungen dissipationsärmere Verstellbewegungen dargestellt werden. In einer Weiterbildung erfolgt in einer berührungslosen translatorischen Dichtung eine Kraftübertragung über die Sterilbarriere hinweg und nicht durch diese hindurch.

Wie bereits vorstehend erläutert, kann die Sterilbarriere ein Ausgleichmittel zum Toleranzausgleich, insbesondere eine elastische Nachgiebigkeit aufweisen. In einer Weiterbildung kann sie hierzu insbesondere eine lokale Wandaufdickung in einem Kontaktbereich eines Ab- und/oder Eintriebselements, insbesondere einer einseitigen Bindung, aufweisen, um so mehr elastischen Weg zur Verfügung zu stellen. In einer Weiterbildung die elastische Nachgiebigkeit, insbesondere eine lokale Wandaufdickung, eine höhere Steifigkeit aufweisen als ein sie umgebender Bereich der Sterilbarriere, um das Übertragungsverhalten zu verbessern. Hierzu kann die Sterilbarriere in einer Ausführung der vorliegenden Erfindung im Kontaktbereich eines Ein- und/oder Abtriebselements eine lokale Werkstoffänderung aufweisen, insbesondere lokal einen Werkstoff mit einer höheren oder niedrigeren Steifigkeit als in einer Umgebung des Kontaktbereichs.

Nach einem Aspekt weist die Sterilbarriere im Bereich der mechanischen Schnittstelle wenigstens einen Elementfortsatz auf. Dieser ist in einer Ausführung lösbar mit einer Abtriebselementbasis verbindbar bzw. verbunden, welche die Sterilbarriere zerstörend durchgreift und zusammen mit dem Elementfortsatz ein Abtriebselement bildet. Gleichermaßen kann ein Elementfortsatz lösbar mit einer Eintriebselementbasis verbindbar bzw. verbunden sein, welche die Sterilbarriere zerstörend durchgreift und zusammen mit dem Elementfortsatz ein Eintriebselement bildet. Beispielsweise kann eine sterile, insbesondere sterilisierte Eintriebselementbasis der Antriebsanordnung des sterilen Instrumentenschaftes zerstörend die Sterilbarriere durchgreifen und auf der instrumentenschaftabgewandten Seite mit dem Elementfortsatz verbunden werden, der dann innerhalb der Sterilbarriere bzw. sterilen Hülle mit dem zugeordneten Abtriebselement gekoppelt wird. Gleichermaßen kann ein steriler Elementfortsatz instrumentenschaftseitig steril kontaktierend an der Sterilbarriere angeordnet werden, bevor eine Abtriebselementbasis diese zerstörend durchgreift und auf der instrumentenschaftzugewandten Seite mit dem Elementfortsatz verbunden wird. Auf diese Weise kann jeweils die Sterilität des Instrumentenschaftes bei Ankopplung einer selbst nicht sterilen, durch die Sterilbarriere umhüllten Antriebseinheit gewahrt werden.

Ein weiterer Gesichtspunkt der vorliegenden Offenbarung betrifft die Befestigung von Antriebseinheit und Instrumentenschaft aneinander. Hierzu weist nach einem Aspekt, der mit einem oder mehreren der vorstehenden Aspekte bzw. Ausführungen kombiniert sein kann, die Instrumentenanordnung bzw. das Instrument ein Befestigungselement zur lösbaren Verbindung mit der Antriebseinheit auf, welches dazu vorgesehen ist, vorzugsweise ausschließlich von außen auf einer der Antriebseinheit abgewandten Oberfläche der Sterilbarriere angeordnet zu werden bzw. welches ausschließlich auf einer der Antriebseinheit abgewandten Oberfläche der Sterilbarriere angeordnet ist. Das Befestigungselement kann lösbar, insbesondere form- oder reibschlüssig, oder unlösbar mit dem Instrumentenschaft verbunden, beispielsweise mit diesem verclipst oder integral ausgebildet sein. Die Sterilbarriere ist in einer Ausführung wenigstens im Kontaktbereich mit dem Befestigungselement, vorzugsweise im Bereich der kompletten mechanischen Schnittstelle, geschlossen, insbesondere kann sie zwischen Rastvorsprüngen und/oder -aussparungen von Antriebseinheit und Befestigungselement zerstörungs- bzw. öffnungsfrei geklemmt sein. Auf diese Weise muss bei einer Befestigung des Instrumentenschaftes an der umhüllten Antriebseinheit keine Abdichtung erfolgen. In einer Weiterbildung ist das Befestigungselement entsprechend dichtungsfrei bzw. dichtungslos ausgebildet.

Das Befestigungselement kann insbesondere separat als steriler Einmalartikel oder sterilisierbarer Adapter ausgebildet und an der Antriebseinheit reib- und/oder formschlüssig, insbesondere mittels Clipsverbindung, befestigbar bzw. befestigt sein. Insbesondere in Kombination mit einer einseitigen Bindung können so Befestigungs- und Kopplungsfunktionalität getrennt und auf Befestigungselement und Schnittstelle aufgeteilt werden.

Nach einem Aspekt der vorliegenden Offenbarung weist ein chirurgisches Instrument einen Instrumentenschaft, an dem ein Endeffektor angeordnet ist, und ein Antriebsmodul mit einem Antrieb zur Aktuierung eines oder mehrerer Freiheitsgrade des Endeffektors relativ zum Instrumentenschaft auf. Instrumentenschaft und Antriebsmodul sind in einer Ausführung, insbesondere lösbar, miteinander verbindbar bzw. verbunden. In einer Weiterbildung ist zwischen Instrumentenschaft und Antriebsmodul eine Sterilbarriere angeordnet, insbesondere, um einen schlechter bzw. nicht sterilisierbaren Antrieb gegen ein Operationsumfeld abzuschirmen. Das chirurgische Instrument ist in einer Ausführung ein minimalinvasives chirurgisches Instrument, dessen Instrumentenschaft zum teilweisen Einführen in einen Patienten vorgesehen bzw. eingerichtet ist, insbesondere durch einen Trokar und/oder einen lokalen Zugang, dessen Umfang in einer Ausführung höchstens dem Doppelten des Außenumfangs des einzuführenden Instrumentenschaftteils entspricht.

Das Instrument kann insbesondere ein manipulatorgeführtes Instrument sein. Hierzu weist in einer Ausführung der Instrumentenschaft und/oder das Antriebsmodul eine mechanische und/oder signaltechnische Schnittstelle zum Ankoppeln an einen Manipulator auf. Entsprechend wird nach einem Aspekt der vorliegenden Offenbarung eine Manipulatoranordnung mit einem oder mehreren Manipulatoren, insbesondere sechs- oder mehrachsigen Robotern, unter Schutz gestellt, die ein erfindungsgemäßes chirurgisches Instrument führen.

Der Endeffektor weist einen, zwei oder mehrere translatorische Freiheitsgrade und/oder einen, zwei oder mehrere rotatorische Freiheitsgrade gegenüber bzw. relativ zu dem Instrumentenschaft auf. In einer Ausführung weist der einteilige Endeffektor einen translatorischen oder Drehfreiheitsgrad auf und ist beispielsweise als ausfahrbare Nadel bzw. drehbare Skalpellklinge ausgebildet. In einer anderen Ausführung weist der zweiteilige Endeffektor zwei Drehfreiheitsgrade auf und ist beispielsweise als Schere, Klemme oder dergleichen ausgebildet. Gleichermaßen kann der Endeffektor insbesondere eine Optik zum Aussenden und/oder Empfangen elektromagnetischer Strahlung, insbesondere eine Laseraustritts- oder Endoskop-Linse, und/oder eine Öffnung zum Einsaugen und/oder Ausbringen von Gas und/oder Flüssigkeit aufweisen, die um eine oder mehrere Freiheitsgradachsen verdreht und/oder ein- bzw. ausgefahren werden kann.

Der Antrieb weist in einer Ausführung einen oder mehrere Motoren, insbesondere Elektromotoren, zum Aktuieren des bzw. der Freiheitsgrade des Endeffektors auf. Zusätzlich oder alternativ kann der Antrieb auch elektromagnetische, hydraulische und/oder pneumatische Aktuatoren aufweisen.

Um einen Endeffektor, insbesondere durch einen Antrieb, zu aktuieren, ist nach einem Aspekt der vorliegenden Offenbarung eine Antriebsstranganordnung vorgesehen. Diese kann in einer Ausführung als instrumentenschaftseitige Antriebsstranganordnung an, insbesondere in, einem Instrumentenschaft eines, insbesondere minimalinvasiven und/oder manipulatorgeführten, chirurgischen Instruments angeordnet sein. Zusätzlich oder alternativ kann eine erfindungsgemäße Antriebsstranganordnung als antriebsmodulseitige Antriebsstranganordnung an, insbesondere in, einem Antriebsmodul eines, insbesondere minimalinvasiven und/oder manipulatorgeführten, chirurgischen Instruments angeordnet sein. Entsprechend werden nach einem Aspekt der vorliegenden Erfindung ein Instrumentenschaft und ein Antriebsmodul mit einer erfindungsgemäßen Antriebsstranganordnung unter Schutz gestellt.

Eine Antriebsstranganordnung nach einem Aspekt der vorliegenden Offenbarung weist einen oder mehrere Antriebsstränge zur Aktuierung von einem oder mehreren Freiheitsgraden eines Endeffektors eines chirurgischen Instruments relativ zu einem Instrumentenschaft durch einen Antrieb auf.

Ein Antriebsstrang kann in einer Ausführung, wenigstens im Wesentlichen, nur Zugkräfte übertragen bzw. als biegeweicher Antriebsstrang, insbesondere als Zugseil bzw. -kabel ausgebildet sein. In einer anderen Ausführung kann ein Antriebsstrang Druckkräfte übertragen, insbesondere, wenigstens im Wesentlichen, nur Druckkräfte oder Zug- und Druckkräfte, insbesondere als Druckstab bzw. -stange bzw. Stößel. Gleichermaßen kann ein Antriebsstrang in einer Ausführung auch oder, wenigstens im Wesentlichen, nur Drehmomente übertragen und/oder eine Übersetzung und/oder ein Getriebe aufweisen. In einer Ausführung ist ein Antriebsstrang als Vollwelle oder Hohlwelle oder als Vollstab oder Hohlstab ausgebildet. Allgemein überträgt ein Antriebsstrang im Sinne der vorliegenden Offenbarung, insbesondere mechanisch, Kräfte und/oder Bewegungen zwischen dem Antrieb und dem Endeffektor, insbesondere, um diesen in einem Freiheitsgrad gegenüber dem Instrumentenschaft zu aktuieren.

Nach einem Aspekt der vorliegenden Offenbarung ist an der Antriebsstranganordnung eine Messanordnung zum Erfassen einer Last in einem oder mehreren, insbesondere allen Antriebssträngen angeordnet.

Dadurch können vorzugsweise direkt eine oder mehrere sogenannte aktive bzw. generalisierte Lasten erfasst werden, die in dem bzw. den Freiheitsgraden des Endeffektors wirken. Unter einer generalisierten oder Minimalkraft wird vorliegend insbesondere in fachüblicher Weise eine Last verstanden, die bei einer, gegebenenfalls virtuellen, Bewegung im Freiheitsgrad physikalische, gegebenenfalls virtuelle, Arbeit leistet. Beispielsweise ist die generalisierte Kraft in einem Drehfreiheitsgrad ein Drehmoment um die Drehfreiheitsgradachse. Entsprechend kann eine Last im Sinne der vorliegenden Offenbarung insbesondere eine Kraft, ein antiparalleles Kräftepaar bzw. ein Drehmoment, eine Spannung, insbesondere eine Zug-, Druck- und/oder Biegespannung, und/oder eine, insbesondere elastische, Deformation infolge solcher Kräfte, Momente bzw. Spannungen, insbesondere eine Dehnung bzw. Kompression umfassen, insbesondere sein.

Dies kann wiederum anschaulich am Beispiel der Fig. 34 erläutert werden: die Klemmkraft $F_{E1}$ bewirkt ein Drehmoment um die Drehlagerachse der Klinge 2.1 mit dem Drehfreiheitsgrad $q_1$. Dieses wiederum bewirkt entsprechende Lasten $F_{S1}$, $F_{S2}$ in den instrumentenschaftseitige Antriebssträngen 21, 22 und diese ihrerseits Lasten $F_1$, $F_2$ in antriebsmodulseitigen Antriebssträngen 11, 12. Man erkennt, dass durch eine Messanordnung an den Antriebssträngen 21, 22 und/oder an den Antriebssträngen 11, 12 direkt die aktiven bzw. generalisierten Lasten erfasst werden, die in den Freiheitsgraden des Endeffektors wirken und so insbesondere einem Teleoperateur eine vorteilhafte Rückmeldung aus einem Operationsgebiet vermitteln können. Natürlich kann in einer Ausführung das chirurgische Instrument zusätzlich eine Messanordnung zum Erfassen einer Last in dem Instrumentenschaft, insbesondere zwischen Instrumentenschaft und Endeffektor(lager) und/oder zwischen Instrumentenschaft und Antriebsmodul aufweisen, um zusätzlich zu den aktiven auch sogenannte passive Lasten zu erfassen, insbesondere Stütz- bzw. Lagerlasten. Drückt beispielsweise die Klemme der Fig. 34 in der dargestellten Konstellation mit ihren Spitzen vertikal nach unten, resultieren hieraus reine Lagerlasten in den Drehgelenken der Klingen 2.1, 2.2, die über eine solche zusätzliche Messanordnung zum Erfassen einer Last in dem Instrumentenschaft 20 erfasst und weiterverarbeitet, insbesondere dem Teleoperateur vermittelt werden können.

In einer Ausführung kann ein weiterer Vorteil darin liegen, dass die Messanordnung zum Erfassen wenigstens einer Last in der Antriebsstranganordnung an der Antriebsstranganordnung und somit vorzugsweise im Inneren des Instrumentenschafts oder an, insbesondere in, einem Antriebsmodul angeordnet ist und so eine vorteilhafte, insbesondere geschützte und/oder vom Operationsgebiet bzw. Endeffektor entfernte, insbesondere extrakorporale und/oder antriebsnahe Messstelle zur Verfügung gestellt werden kann.

In einer Ausführung der vorliegenden Offenbarung weist die Antriebsstranganordnung zwei oder mehr, insbesondere gegensinnige, Antriebsstränge zur Aktuierung desselben Freiheitsgrades des Endeffektors auf. Dies ist exemplarisch in Fig. 34 veranschaulicht: dort wird die Klinge 2.1 in ihrem Freiheitsgrad $q_1$ durch die gegensinnigen Antriebstränge 21, 22, beispielsweise zwei Zugseile oder Druckstangen, aktuiert, die wiederum translatorisch durch die gegensinnigen Antriebstränge 11, 12 aktuiert werden, zum Beispiel Stößel, die von einem Elektromotor 13 gegensinnig aktuiert werden.

Die Messanordnung weist in einer Ausführung wenigstens ein Messmittel auf, welches an einem der Antriebsstränge zum Erfassen einer Last in diesem Antriebsstrang angeordnet ist. In einer Weiterbildung weist die Messanordnung ein erstes Messmittel, welches an einem ersten Antriebsstrang zum Erfassen einer Last in diesem Antriebsstrang angeordnet ist, und ein zweites Messmittel auf, welches an einem zweiten, insbesondere gegensinnigen, Antriebsstrang zum Erfassen einer Last in diesem Antriebsstrang angeordnet ist, wobei derselbe Freiheitsgrad des Endeffektors durch den ersten und zweiten Antriebsstrang aktuierbar ist.

In einer Ausführung weist die Antriebsstranganordnung einen ersten Antriebsstrang zum Aktuieren eines ersten Freiheitsgrades des Endeffektors und einen weiteren ersten Antriebsstrang zum Aktuieren eines weiteren Freiheitsgrades des Endeffektors auf. In einer Weiterbildung kann die Antriebsstranganordnung einen zweiten Antriebsstrang zum Aktuieren des ersten Freiheitsgrades des Endeffektors und/oder einen weiteren zweiten Antriebsstrang zum Aktuieren des weiteren Freiheitsgrades des Endeffektors aufweisen. Natürlich kann der Endeffektor noch weitere Freiheitsgrade und entsprechende erste und gegebenenfalls zweite Antriebsstränge aufweisen.

In einer Weiterbildung weist die Messanordnung ein erstes Messmittel auf, welches an dem ersten Antriebsstrang zum Aktuieren des ersten Freiheitsgrades des Endeffektors zum Erfassen einer Last in diesem Antriebsstrang angeordnet ist. Zusätzlich oder alternativ weist die Messanordnung ein zweites Messmittel auf, welches an dem zweiten, insbesondere gegensinnigen, Antriebsstrang zum Aktuieren des ersten Freiheitsgrades des Endeffektors zum Erfassen einer Last in diesem Antriebsstrang angeordnet ist. Zusätzlich oder alternativ weist die Messanordnung ein weiteres erstes Messmittel auf, welches an dem weiteren ersten Antriebsstrang zum Aktuieren des weiteren Freiheitsgrades des Endeffektors zum Erfassen einer Last in diesem Antriebsstrang angeordnet ist. Zusätzlich oder alternativ weist die Messanordnung ein weiteres zweites Messmittel auf, welches an dem weiteren zweiten, insbesondere gegensinnigen, Antriebsstrang zum Aktuieren des weiteren Freiheitsgrades des Endeffektors zum Erfassen einer Last in diesem Antriebsstrang angeordnet ist.

In einer Ausführung sind zwei oder mehr Messmittel, die an zwei, insbesondere gegensinnigen, Antriebssträngen zum Aktuieren desselben Freiheitsgrades des Endeffektors angeordnet sind, signaltechnisch miteinander verknüpft. Sie können insbesondere leitungstechnisch miteinander verschaltet oder, insbesondere in einem Steuermittel, rechentechnisch miteinander verknüpft sein bzw. werden, insbesondere additiv bzw. subtraktiv.

Hierdurch kann in einer Ausführung insbesondere eine gemeinsame Last, insbesondere eine Vorspannung, in zwei Antriebssträngen zum Aktuieren desselben Freiheitsgrades, wenigstens im Wesentlichen, signaltechnisch kompensiert und so vorzugsweise direkt die resultierende aktive bzw. generalisierte Last ermittelt werden. Allgemein sind in einer Ausführung ein erstes und ein zweites Messmittel, die an zwei, insbesondere gegensinnigen, Antriebssträngen zum Aktuieren desselben Freiheitsgrades des Endeffektors angeordnet sind, kompensatorisch miteinander verknüpft.

Unter einer kompensatorischen Verknüpfung wird insbesondere eine Verknüpfung der Signale des ersten und zweiten Messmittels derart verstanden, dass eine vorgegebene Last, insbesondere eine Last in einer vorgegebenen Richtung, wenigstens im Wesentlichen kompensiert wird bzw. das gemeinsame, verknüpfte Signal des ersten und zweiten Messmittels, wenigstens im Wesentlichen, unabhängig von einer Last ist, die sowohl durch das erste als auch das zweite Messmittel erfasst wird.

Insbesondere hierzu können das erste und zweite Messmittel, die an einem ersten bzw. zweiten Antriebsstrang zum Aktuieren desselben Freiheitsgrades angeordnet sind, in zwei Zweigen einer Wheatstoneschen Brückenschaltung miteinander verknüpft sein, insbesondere in zwei Zweigen einer Wheatstoneschen Halbbrückenschaltung, die vorzugsweise in Reihe zwischen einer Brücken-Eingangs- bzw. Speisespannung liegen. In einer Weiterbildung kann die Messanordnung ein drittes Messmittel, welches, insbesondere dem ersten Messmittel gegenüberliegend, an dem ersten Antriebsstrang zum Erfassen einer Last in diesem Antriebsstrang angeordnet ist, und ein viertes Messmittel aufweisen, welches, insbesondere dem zweiten Messmittel gegenüberliegend, an dem zweiten Antriebsstrang zum Erfassen einer Last in diesem Antriebsstrang angeordnet ist, wobei das erste Messmittel in einem ersten Zweig, das zweite Messmittel in einem zweiten Zweig, insbesondere in Reihe mit dem ersten Messmittel zwischen einer Brücken-Eingangs- bzw. Speisespannung, das dritte Messmittel in einem dritten Zweig, insbesondere parallel zu dem zweiten Messmittel zwischen der Speise- bzw. Erregerspannung, und das vierte Messmittel in einem vierten Zweig, insbesondere parallel zu dem ersten Messmittel zwischen der Speisespannung, einer elektrischen Schaltung, insbesondere einer Wheatstoneschen Vollbrückenschaltung miteinander verknüpft sind. Hierdurch können in einer Ausführung nicht nur gemeinsame Lasten, sondern auch Ungleichförmigkeiten, insbesondere Biegemomente, in demselben Antriebsstrang, wenigstens im Wesentlichen, bereits signaltechnisch kompensiert werden. Zusätzlich oder alternativ können erfasste Lasten so signaltechnisch verstärkt werden, insbesondere, indem einander entsprechende Lastkomponenten, die von verschiedenen Messmitteln erfasst werden, sich durch die Verknüpfung addieren.

Ein Messmittel der Messanordnung kann in einer Ausführung einen oder mehrere Dehnungsmesser zum, insbesondere elektrischen, magnetischen, optischen und/oder akustischen, Erfassen einer mechanischen Last aufweisen. Hierbei kann es sich insbesondere um, vorzugsweise folienartige, Dehnmessstreifen, deren Widerstand sich vorzugsweise mit ihrer elastischen Dehnung ändert, Halbleiterdehnungsmesser, optische, vorzugsweise faserartige, Dehnungsmesser, insbesondere auf Bragg oder Fabry-Perot-Technologie basierende Dehnungsmesser wie FBG-Dehnungsmesser ("optic fiber Bragg grating"), akustische Dehnungsmesser wie insbesondere sogenannte SAW-Dehnungsmesser ("strain sensing surface acoustic wave"), piezoelektrische oder magnetoelastische Signalgeber oder dergleichen handeln.

In einer Ausführung sind ein oder mehr Messmittel der Messanordnung, wenigstens im Wesentlichen, zum Erfassen einer axialen Zug- und/oder Drucklast in einem Antriebsstrang an diesem Antriebsstrang angeordnet. Beispielsweise kann ein Dehnmessstreifen, wenigstens im Wesentlichen, in Längsrichtung an einem Zugseil oder Druckstab angeordnet bzw. ausgerichtet sein.

In einer Ausführung sind ein oder mehr Messmittel der Messanordnung, wenigstens im Wesentlichen, in einer Aussparung eines Antriebsstrangs angeordnet. Hierdurch kann in einer Ausführung die Messanordnung geschützt werden. Zusätzlich oder alternativ kann so ein Hervorragen der Messanordnung über eine Außenkontur des bzw. der Antriebsstränge reduziert, insbesondere verhindert werden, was die Handhabung, insbesondere den Betrieb und/oder die Montage erleichtern kann.

Zusätzlich oder alternativ kann der Antriebsstrang im Bereich eines oder mehrerer Messmittel eine Wandstärkenreduzierung aufweisen, insbesondere durch die vorstehend erläuterte Aussparung. Hierdurch kann in einer Ausführung die Sensitivität der Messanordnung erhöht werden. In einer Ausführung kann der Antriebsstrang im Bereich eines oder mehrerer Messmittel zur Wandstärkenreduzierung einen Hohlraum, insbesondere eine Hohlraumerweiterung aufweisen. In einer Weiterbildung kann der Antriebsstrang eine, vorzugsweise dünnwandige, Hülse aufweisen, auf der außen- und/oder innenseitig ein oder mehr Messmittel der Messanordnung angeordnet sind. Die Hülse kann stoffschlüssig mit weiteren Teilen, insbesondere Stäben oder Wellen mit Vollquerschnitt, zu dem Antriebsstrang verbunden, insbesonder verschweißt oder - klebt, werden.

In einer Ausführung ist eine Antriebsstranganordnung an, insbesondere in, einem Antriebsmodul eines chirurgischen Instruments angeordnet, mit dem ein Instrumentenschaft, der einen Endeffektor aufweist, insbesondere lösbar, verbindbar ist. Die antriebsmodulseitige Antriebsstranganordnung kann insbesondere eine mechanische Schnittstelle zum Ankoppeln einer instrumentenschaftseitigen Antriebsstranganordnung zum Aktuieren des Endeffektors aufweisen. Ein antriebsmodulseitiger Antriebsstrang kann insbesondere eine Welle eines Elektromotors des Antriebs aufweisen oder mit dieser, insbesondere gelenkig, gekoppelt sein. Durch eine antriebsmodulseitige Messanordnung können in vorteilhafter Weise Lasten weit entfernt von dem Endeffektor, insbesondere extrakorporal, vorzugsweise hinter einer Sterilbarriere bzw. in einem sterilen Gehäuse des Antriebsmoduls, erfasst werden.

Zusätzlich oder alternativ kann in einer Ausführung eine Antriebsstranganordnung an, insbesondere in, einem Instrumentenschaft eines chirurgischen Instruments mit einem Endeffektor angeordnet sein, mit dem ein Antriebsmodul, das einen Antrieb aufweist, insbesondere lösbar, verbindbar ist. Die instrumentenschaftseitige Antriebsstranganordnung kann insbesondere eine mechanische Schnittstelle zum Ankoppeln einer antriebsmodulseitigen Antriebsstranganordnung aufweisen, die mit dem Antrieb gekoppelt ist. Durch eine instrumentenschaftseitige Messanordnung können vorzugsweise Lasten in der Nähe des Endeffektors unmittelbar erfasst werden.

Eine antriebsmodulseitige Antriebsstranganordnung und eine instrumentenschaftseitige Antriebsstranganordnung, von denen an wenigstens einer eine Messanordnung zum Erfassen von Lasten in dieser Antriebsstranganordnung angeordnet ist, sind in einer Ausführung der vorliegenden Offenbarung über eine Schnittstelle koppelbar bzw. lösbar gekoppelt.

Sie können in einer Ausführung translatorisch koppelbar bzw. gekoppelt sein. Hierunter wird vorliegend insbesondere verstanden, dass ein antriebsmodulseitiger und ein damit gekoppelter instrumentenschaftseitiger Antriebsstrang an der Schnittstelle translatorisch beweglich sind bzw. bewegt werden, um einen Freiheitsgrad des Endeffektors zu aktuieren, wobei diese translatorische Bewegung im weiteren antriebsmodulseitigen und/oder instrumentenschaftseitigen Antriebsstrang in eine rotatorische Bewegung umgesetzt werden bzw. sein kann. Gleichermaßen können ein antriebsmodulseitiger und ein damit gekoppelter instrumentenschaftseitiger Antriebsstrang an der Schnittstelle rotatorisch gekoppelt sein bzw. werden, wobei diese rotatorische Bewegung in der Schnittstelle im weiteren antriebsmodulseitigen und/oder instrumentenschaftseitigen Antriebsstrang in eine translatorische Bewegung umgesetzt werden bzw. sein kann.

In einer Ausführung sind eine antriebsmodulseitige Antriebsstranganordnung und eine instrumentenschaftseitige Antriebsstranganordnung, von denen an wenigstens einer eine Messanordnung zum Erfassen von Lasten in dieser Antriebsstranganordnung angeordnet ist, über eine Schnittstelle einseitig koppelbar bzw. lösbar gekoppelt. Hierunter wird vorliegend insbesondere verstanden, dass ein antriebsmodulseitiger und ein damit gekoppelter bzw. koppelbarer instrumentenschaftseitiger Antriebsstrang eine sogenannte einseitige Bindung aufweisen bzw. nur Kräfte oder Momente in einer Richtung übertragen können, insbesondere nur Druckkräfte. In einer Weiterbildung weisen ein antriebsmodulseitiger und ein damit gekoppelter bzw. koppelbarer instrumentenschaftseitiger Antriebsstrang an der Schnittstelle einander gegenüberliegende, vorzugsweise fluchtende, Stößel auf, die verschieblich gelagert sind und aufeinander, wenigstens im Wesentlichen, nur Druckkräfte übertragen.

In einer Ausführung sind eine antriebsmodulseitige und eine damit gekoppelte bzw. koppelbare instrumentenschaftseitige Antriebsstranganordnung über eine, vorzugsweise folienartige und/oder flexible Sterilbarriere gekoppelt. Die Sterilbarriere kann in einer Ausführung translatorische Bewegungen der Antriebsstranganordnungen an der Schnittstelle unter, vorzugweise elastischer, Deformation mitmachen und/oder beweglich, insbesondere verschieblich und/oder drehbar, gelagerte Kupplungselemente aufweisen.

Wie vorstehend erläutert, kann durch die Messanordnung in einer Ausführung vorzugsweise eine aktive bzw. generalisierte Last direkt erfasst und so eine Rückmeldung an einen Teleoperateur verbessert werden. Entsprechend wird nach einem Aspekt der vorliegenden Erfindung ein manuelles Teleoperationsmittel eines chirurgischen Instruments auf Basis einer oder mehrerer durch die Messanordnung erfassten Lasten gesteuert, wobei zur kompakteren Darstellung auch ein Regeln verallgemeinernd als Steuern im Sinne der vorliegenden Erfindung bezeichnet wird. Ein manuelles Teleoperationsmittel kann insbesondere einen oder mehrere Hebel, Handgriffe, Handschuhe, Joysticks oder ein sogenanntes Spiegelinstrument aufweisen, dessen Bewegungen mit den Bewegungen des chirurgischen Instruments, vorzugsweise steuerungstechnisch, gekoppelt sind. Auf Basis von durch die Messanordnung erfassten Lasten kann ein solches Teleoperationsmittel, insbesondere motorisch, aktuiert werden, um dem Teleoperateur eine haptische Rückmeldung über den Operationsvorgang zu vermitteln. Insbesondere können auf den Endeffektor wirkende Kräfte auf Basis von durch die Messanordnung erfasste Lasten auf das Teleoperationsmittel aufgeprägt werden, um dem Teleoperateur ein Kraft-Feedback zu vermitteln.

Zusätzlich oder alternativ können durch die Messanordnung erfasste Lasten auch zum Steuern, insbesondere Regeln, des Antriebs genutzt werden. Beispielsweise kann eine von einem Motor aufzuprägende Sollkraft mit einer Istkraft in einem Antriebsstrang verglichen und auf dieser Basis der Motor geregelt werden.

Entsprechend ist nach einem Aspekt der vorliegenden Offenbarung ein Steuermittel zum Steuern eines chirurgischen Instruments dazu eingerichtet, eine oder mehrere durch die Messanordnung erfasste Lasten weiterzuverarbeiten, insbesondere den Antrieb und/oder ein manuelles Teleoperationsmittel auf Basis von durch die Messanordnung erfassten Lasten zu steuern. Ein Mittel im Sinne der vorliegenden Offenbarung kann hard- und/oder softwaretechnisch ausgebildet sein, insbesondere eine, vorzugsweise mit einem Speicher- und/oder Bussystem daten- bzw. signalverbundene, insbesondere digitale, Verarbeitungs-, insbesondere Mikroprozessoreinheit (CPU) und/oder ein oder mehrere Programme oder Programmmodule aufweisen. Die CPU kann dazu ausgebildet sein, Befehle, die als ein in einem Speichersystem abgelegtes Programm implementiert sind, abzuarbeiten, Eingangssignale von einem Datenbus zu erfassen und/oder Ausgangssignale an einen Datenbus abzugeben. Ein Speichersystem kann ein oder mehrere, insbesondere verschiedene, Speichermedien, insbesondere optische, magnetische, Festkörper- und/oder andere nicht-flüchtige Medien aufweisen. Das Programm kann derart beschaffen sein, dass es die hier beschriebenen Verfahren verkörpert bzw. auszuführen imstande ist, sodass die CPU die Schritte solcher Verfahren ausführen kann und damit insbesondere den Antrieb und/oder das Teleoperationsmittel steuern kann.

Nach einem Aspekt der vorliegenden Offenbarung weist ein chirurgisches Instrument einen Instrumentenschaft und eine damit lösbar verbindbare, insbesondere verbundene, Antriebseinheit auf. Das Instrument ist in einer Ausführung ein robotergeführtes Instrument. Hierzu weist in einer Weiterbildung der Instrumentenschaft und/oder die Antriebseinheit eine, insbesondere mechanische, signal- und/oder energietechnische, insbesondere elektrische, hydraulische und/oder pneumatische, Schnittstelle zum Befestigen an einem Roboter auf. In einer Ausführung ist das Instrument ein minimalinvasives chirurgisches Instrument, dessen Instrumentenschaft zum teilweisen Einführen in einen Patienten durch eine lokale natürliche oder künstliche Öffnung, insbesondere eine Körperöffnung oder einen Trokar, vorgesehen ist.

Ein Instrumentenschaft nach einer Ausführung der vorliegenden Offenbarung weist einen oder mehrere Freiheitsgrade auf. In einer Ausführung weist der Instrumentenschaft ein, insbesondere wenigstens im Wesentlichen zylindrisches, Rohr auf. Ein Freiheitsgrad des Instrumentenschaftes kann dann insbesondere ein Gelenkfreiheitsgrad eines Gelenkes zwischen zwei Rohrstücken oder ein elastischer Freiheitsgrad eines flexiblen Rohres sein. In einer Ausführung weist der Instrumentenschaft einen Endeffektor auf, insbesondere eine Zange, Klemme oder Klammer, ein Skalpell, einen Bohrer, eine Nadel bzw. Kanüle zum Aufnehmen und/oder Einbringen von gasförmigem und/oder flüssigem Fluid und/oder eine Optik zum Aussenden und/oder Empfangen elektromagnetischer Strahlung, insbesondere ein Lichtwellenleiterende eines Endoskops oder Lasers. Ein Freiheitsgrad des Instrumentenschaftes kann dann insbesondere ein Freiheitsgrad des Endeffektors sein, insbesondere ein translatorischer oder rotatorischer Freiheitsgrad gegenüber dem Rohr oder ein funktionaler Freiheitsgrad, insbesondere zum Öffnen bzw. Schließen einer Zange, Klemme, Klammer, Kanüle und/oder Optik oder dergleichen. Ein funktionaler Freiheitsgrad im Sinne der vorliegenden Erfindung kann insbesondere eine Bewegungsmöglichkeit von zwei Endeffektorteilen relativ zueinander beschreiben. In einer Ausführung kann das Rohr einen rotatorischen Freiheitsgrad gegenüber einem proximalen Instrumentengehäuse des Instrumentenschafts aufweisen.

Zum Aktuieren eines Freiheitsgrades weist der Instrumentenschaft ein oder mehrere, insbesondere gegensinnig wirkende, Eintriebsglieder auf. Ein Eintriebsglied ist in einer Ausführung translatorisch bzw. verschiebbar und/oder rotatorisch bzw. drehbar in einer Schnittstelle des Instrumentenschaftes gelagert, um durch eine translatorische bzw. rotatorische Bewegung einen Freiheitsgrad des Instrumentenschaftes zu aktuieren. Es kann hierzu mit einem Rohr(teil) oder Endeffektor des Instrumentenschaftes gekoppelt sein, insbesondere durch eine Druckstange, ein Zugseil bzw. Kabeltrum und/oder ein Getriebe, insbesondere zum Umsetzen einer translatorischen und rotatorischen Bewegung ineinander. In einer Ausführung weist der Instrumentenschaft, insbesondere eine Schnittstelle des Instrumentenschaftes zum Koppeln mit der Antriebseinheit, eine Eintriebsgliedanordnung mit mehreren Eintriebsgliedern auf. In einer Weiterbildung ist wenigstens ein Freiheitsgrad des Instrumentenschaftes durch zwei, insbesondere gegenläufige, Eintriebsglieder gegensinnig aktuierbar, beispielsweise ein verschwenkbarer Endeffektor durch zwei gegenläufige Druckstangen auf- und abschwenkbar.

Eine Antriebseinheit nach einer Ausführung der vorliegenden Offenbarung weist ein Gehäuse und ein oder mehrere Antriebsmodule auf. Wenigstens ein, vorzugsweise alle Antriebsmodule weisen jeweils einen Antrieb und eine Abtriebsgliedanordnung mit einem oder mehreren beweglichen Abtriebsgliedern auf. Der Antrieb kann insbesondere einen elektromagnetischen, hydraulischen oder pneumatischen Dreh- oder Linearmotor aufweisen, insbesondere ein Elektromotor sein.

In einer Ausführung aktuiert der Antrieb genau ein Abtriebsglied. In einer anderen Ausführung aktuiert der Antrieb, insbesondere gegensinnig, zwei Abtriebsglieder. Ein oder mehrere Abtriebsglieder sind in einer Ausführung translatorisch bzw. verschiebbar und/oder rotatorisch bzw. drehbar in einer Schnittstelle des Antriebsmoduls gelagert, um durch eine translatorische bzw. rotatorische Bewegung einen Freiheitsgrad des Instrumentenschaftes zu aktuieren. Ab- und Eintriebsgliedanordnung können in einer Ausführung direkt oder über eine Kupplung einseitig gekoppelt werden bzw. sein. Hierunter wird in fachüblicher Weise verstanden, dass Kräfte nur in einer Aktuierungsrichtung von dem Abtriebs- auf das Eintriebsglied übertragbar sind, während Abtriebs- und Eintriebsglied sich in der entgegengesetzten Richtung voneinander entfernen können. In einer Weiterbildung weist eine Abtriebsgliedanordnung zwei gegenläufige bzw. gegensinnig aktuierte Abtriebsglieder, insbesondere zwei Druckstangen, auf, die einseitig mit entsprechenden gegenläufigen Eintriebsgliedern direkt oder über eine Kupplung koppelbar bzw. gekoppelt sind. In einer anderen Ausführung sind Ab- und Eintriebsgliedanordnung direkt oder über eine Kupplung zweiseitig koppelbar bzw. gekoppelt. Hierunter wird entsprechend verstanden, dass Kräfte in zwei entgegengesetzten Aktuierungsrichtungen von dem Abtriebs- auf das Eintriebsglied übertragbar sind. In einer Weiterbildung weist eine Abtriebsgliedanordnung ein drehbares Abtriebsglied, insbesondere eine Abtriebswelle eines Elektromotors oder Getriebes, auf, das drehfest mit einem entsprechenden drehbaren Eintreibsglied koppelbar bzw. gekoppelt ist. Zur kompakteren Darstellung wird vorliegend auch ein antiparalleles Kräftepaar, d.h. ein Drehmoment, verallgemeinernd als Kraft bezeichnet.

Nach einem Aspekt der vorliegenden Offenbarung sind ein oder mehrere Antriebsmodule in dem, insbesondere geschlossenen, Gehäuse der Antriebseinheit jeweils in einer Koppelrichtung bzw. gegen die Eintriebsgliedanordnung beweglich gelagert und vorgespannt. Die Koppelrichtungen von zwei, vorzugsweise allen, Antriebsmodulen können, wenigstens im Wesentlichen, parallel sein. Gleichermaßen können die Koppelrichtungen von zwei Antriebsmodulen einen Winkel einschließen, dessen Betrag vorzugsweise kleiner als 90°, insbesondere kleiner als 45° ist.

Indem nicht oder nicht nur die einzelnen Abtriebsglieder vorgespannt werden, wie dies die eingangs genannte WO 2011/143022 A1 vorschlägt, sondern nach diesem Aspekt ausschließlich oder zusätzlich das Antriebsmodul in dem Gehäuse und dadurch dessen Abtriebsgliedanordnung als Ganzes, kann in einer Ausführung die Kopplung zwischen Abtriebs- und Eintriebsanordnung verbessert werden. Zusätzlich oder alternativ können das Gewicht, der Bauraum und/oder -aufwand reduziert und/oder die Bedienung verbessert werden.

In einer Ausführung weist ein Antriebsmodul ein hydraulisches, ein pneumatisches und/oder ein elastisches Federmittel, insbesondere wenigstens ein Hydraulik- oder Pneumatikzylinder und/oder eine Druck- und/oder Zugfeder, zum Vorspannen auf, das das Antriebsmodul in dem Gehäuse fesselt und in Koppelrichtung bzw. gegen die Eintriebsgliedanordnung vorgespannt ist. Ein hydraulisches oder pneumatisches Federmittel kann in einer Weiterbildung schaltbare ausgebildet sein, insbesondere in einen drucklosen Zustand, in dem es, wenigstens im Wesentlichen, keine Kraft ausübt. Vorteilhaft erfordert die Verstellung des Antriebsmoduls im Gehäuse nach einem Wegnehmen des Überdrucks eines hydraulischen oder pneumatischen Federmittels keine nennenswerte Bedienkraft.

Zusätzlich oder alternativ kann ein Antriebsmodul in einer Ausführung eine Magnetanordnung zum Vorspannen des Antriebsmoduls aufweisen. Die Magnetanordnung kann einen oder mehrere Permanent- oder Elektromagnete aufweisen, die an dem einen von dem Gehäuse und dem Antriebsmodul angeordnet sind. Das andere von dem Gehäuse und dem Antriebsmodul kann einen oder mehrere weitere Elektromagnete und/oder hart- oder weichmagnetische Bereiche, insbesondere wenigstens einen weiteren Permanentmagneten, aufweisen, der diesem bzw. diesen Permanent- oder Elektromagneten vorzugsweise gegenüberliegt und von diesem magnetisch permanent oder bei Bestromung angezogen oder abgestoßen wird.

In einer Ausführung ist wenigstens ein Permanent- oder Elektromagnet an dem Gehäuse auf einer instrumentenschaftabgewandten Seite angeordnet und, vorzugsweise diesem gegenüberliegend, wenigstens ein weiterer Elektromagnet oder hart- oder weichmagnetischer Bereich, insbesondere wenigstens ein weiterer Permanentmagnet, an dem Antriebsmodul angeordnet. Zusätzlich oder alternativ kann wenigstens ein Permanent- oder Elektromagnet an dem Gehäuse auf einer instrumentenschaftzugewandten Seite angeordnet und, vorzugsweise diesem gegenüberliegend, wenigstens ein weiterer Elektromagnet oder hart- oder weichmagnetischer Bereich, insbesondere wenigstens ein weiterer Permanentmagnet, an dem Antriebsmodul angeordnet sein. Zusätzlich oder alternativ kann wenigstens ein Permanent- oder Elektromagnet an dem Antriebsmodul auf einer instrumentenschaftabgewandten Seite angeordnet und, vorzugsweise diesem gegenüberliegend, wenigstens ein weiterer Elektromagnet oder hart- oder weichmagnetischer Bereich, insbesondere wenigstens ein weiterer Permanentmagnet, an dem Gehäuse angeordnet sein. Zusätzlich oder alternativ kann wenigstens ein Permanent- oder Elektromagnet an dem Antriebsmodul auf einer instrumentenschaftzugewandten Seite angeordnet und, vorzugsweise diesem gegenüberliegend, wenigstens ein weiterer Elektromagnet oder hart- oder weichmagnetischer Bereich, insbesondere wenigstens ein weiterer Permanentmagnet, an dem Gehäuse angeordnet sein. Durch magnetische Anziehung bzw. Abstoßung zwischen diesen kann das Antriebsmodul in dem Gehäuse gegen die Eintriebsgliedanordnung vorgespannt werden.

Während bei einer Vorspannung durch ein Federmittel, beispielsweise infolge der Entspannung einer mechanischen Feder oder einer Volumenvergrößerung eines Hydraulik- oder Pneumatikvolumens, die Vorspannkraft mit zunehmender Verstellung des Antriebsmoduls im Gehäuse abnimmt, kann eine (elektro)magentische Vorspannung bei zunehmender Verstellung des Antriebsmoduls im Gehäuse vorteilhafterweise zunehmen.

In einer Weiterbildung weist die Magnetanordnung einen oder mehrere wahlweise, insbesondere gesteuert, bestrombare Elektromagnete auf. Auf diese Weise kann die Vorspannung wahlweise, insbesondere gesteuert aufgeprägt werden. Zur kompakteren Darstellung wird vorliegend auch ein Regeln, d.h. die Vorgabe einer Steuergröße auf Basis einer erfassten Ist-Größe, verallgemeinernd als Steuern bezeichnet.

In einer Ausführung weist die Magnetanordnung ein oder mehrere, vorzugsweise unmagnetische, Abstandselemente auf, die einen direkten Kontakt zwischen einem Elektro- oder Permanetmagneten an einem von dem Gehäuse der Antriebseinheit und dem Antriebsmodul mit einem weich- oder hartmagnetischen Bereich, insbesondere einem (weiteren) Permanetmagneten an dem anderen von dem Gehäuse der Antriebseinheit und dem Antriebsmodul verhindern, um so einen magnetischen Kurzschluss zu vermeiden, zu dessen Lösen eine übermäßige Kraft erforderlich wäre.

Beim oder nach dem Koppeln von Ab- und Eintriebsanordnung bzw. Antriebseinheit und Instrumentenschaft muss nach dem vorstehend erläuterten Aspekt die Vorspannung des Antriebsmoduls aufgebaut werden.

Dies kann in einer Ausführung, wie vorstehend erläutert, durch wahlweises Bestromen eines oder mehrerer Elektromagnete der Magnetanordnung erfolgen. Auf diese Weise muss ein Bedienpersonal, insbesondere bei den hohen Anforderungen im OP-Betrieb, vorteilhaft allenfalls geringe Kräfte zum Koppeln von Antriebseinheit und Instrumentenschaft aufbringen.

Zusätzlich oder alternativ kann in einer Ausführung eine, insbesondere mechanische und/oder magnetische, Rückzuganordnung zum Zurückziehen des Antriebsmoduls gegen die Vorspannung vorgesehen sein. So kann in einer Ausführung eine Magnetanordnung wahlweise aktiviert werden, um das Antriebsmodul unter (weiterem) Vorspannen eines Federmittels von der Eintriebsanordnung weg zu ziehen. Wird die Bestromung der Magnetanordnung, vorzugsweise gesteuert, beispielsweise linear, reduziert, baut das Federmittel die Vorspannung auf.

Eine Weiterbildung basiert auf der Idee, den Arbeitsbereich des Antriebs des Antriebsmoduls aufzuteilen in einen Aktuierbereich, in dem der Antrieb die Abtriebsgliedanordnung zum Aktuieren eines Freiheitsgrads des Instrumentenschaftes aktuiert, und einem hiervon verschiedenen Rückzugbereich, in dem der Antrieb die Rückzuganordnung aktuiert. Beide Bereiche können insbesondere durch einen mechanischen Anschlag für ein Abtriebsmittel des Antriebs voneinander getrennt sein, wobei das Abtriebsmittel das Antriebsmodul gegen die Vorspannung verschiebt, wenn es sich an dem mechanischen Anschlag abstützt.

Indem der Antrieb über den Aktuierbereich hinaus verstellt wird, kann so durch entsprechende Steuerung des Antriebs das Antriebsmodul vorzugsweise motorisch gegen die Vorspannung zurückgezogen werden, was, wie vorstehend erläutert, das Koppeln von Instrumentenschaft und Antriebseinheit vorteilhaft vereinfacht.

In einer vorteilhaften Weiterbildung weist die Antriebseinheit eine Antriebsmodul-Verriegelungsanordnung zum Verriegeln des zurückgezogenen Antriebsmoduls auf. Diese kann insbesondere mechanisch, vorzugsweise form- und/oder reibschlüssig, und/oder (elektro)magnetisch und/oder pneumatisch ausgebildet sein. In einer exemplarischen Ausführung kann ein Riegel verstellt werden und das Antriebsmodul gegen eine vorspannungsinduzierte Verstellung in Koppelrichtung sichern. Auf diese Weise kann das (stärker vorgespannte) Antriebsmodul bzw. dessen Abtriebsanordnung in einer Ausführung auch, wenn Antriebseinheit und Instrumentenschaft miteinander verbunden werden bzw. sind, von der Eintriebsanordnung beabstandet werden.

Nach einem Aspekt der vorliegenden Offenbarung schließt die Koppelrichtung, in der das Antriebsmodul in dem Gehäuse der Antriebseinheit beweglich gelagert und vorgespannt ist, mit einer Längsachse des Instrumentenschaftes einen Winkel ein, dessen Betrag größer ist als 0°, insbesondere größer als 45°. In einer Ausführung beträgt der Winkel, wenigstens im Wesentlichen, 90° bzw. die Koppelrichtung ist, wenigstens im Wesentlichen, senkrecht bzw. orthogonal zur Längsachse des Instrumentenschaftes.

Indem die Koppelrichtung nicht parallel zur Längsachse des Instrumentenschaftes ist, wie dies in der eingangs genannten WO 2011/143022 A1 der Fall ist, sondern nach diesem Aspekt mit der Längsachse einen von Null verschiedenen, insbesondere rechten, Winkel einschließt, interferieren in einer Ausführung vorteilhaft Deformationen des Instrumentenschaftes nicht oder nur weniger mit der Vorspannung, da deren Kraftrichtungen nicht übereinstimmen. Auf diese Weise kann vorzugsweise insbesondere eine Longitudinalschwingung im Instrumentenschaft von der Vorspannung des Antriebsmoduls, wenigstens teilweise, entkoppelt und diese so verbessert werden.

Die Koppelrichtung kann in einer Ausführung, wenigstens im Wesentlichen, mit einer Aktuierungsrichtung der Ab- und/oder Eintriebsgliedanordnung übereinstimmen. Unter einer Koppelrichtung wird insbesondere eine Bewegungsrichtung verstanden, in der ein Ab- bzw. Eintriebsglied beweglich gelagert und vorgespannt ist bzw. wird, um mit einem entsprechenden Ein- bzw. Abtriebsglied zu koppeln. Unter einer Aktuierungsrichtung wird insbesondere eine Bewegungsrichtung verstanden, in der ein Ab- bzw. Eintriebsglied beweglich ist, um einen Freiheitsgrad des Instrumentenschaftes zu aktuieren. Sind beispielsweise ein Ab- und ein damit gekoppeltes Eintriebsglied als einseitig gekoppelte Druckstangen bzw. -stößel ausgebildet, stellt die Längsachsenrichtung des Stößelpaares, in der der Ab- gegen den Eintriebsstößel vorgespannt ist, die Koppelrichtung dar. Diese stellt auch die Aktuierungsrichtung dar, in der das Stößelpaar durch den Antrieb bewegt wird, um einen Freiheitsgrad des Instrumentenschaftes zu aktuieren. Sind in einem anderen Beispiel ein Ab- und ein damit gekoppeltes Eintriebsglied als zweiseitig drehfest gekoppelte Wellen ausgebildet, stellt die Längsachsenrichtung des Wellenpaares, um die das Stößelpaar durch den Antrieb gedreht wird, um einen Freiheitsgrad des Instrumentenschaftes zu aktuieren, die Aktuierungsrichtung dar. Diese stellt auch die Koppelrichtung dar, in der die Ab- gegen die Eintriebswelle vorgespannt ist.

In einer Ausführung der vorliegenden Offenbarung weist ein Instrumentenschaft eine Aufnahme zur, insbesondere formschlüssigen, lösbaren Befestigung einer Antriebseinheit auf.

Die Antriebseinheit kann in einer Ausführung durch einen Bajonettverschluss formschlüssig lösbar in der Aufnahme befestigbar bzw. befestigt sein bzw. werden. Hierzu kann eine von der Antriebseinheit und der Aufnahme eine oder mehrere Vorsprünge aufweisen, die infolge einer Verdrehung der Antriebseinheit in der Aufnahme in entsprechende Aussparungen der anderen von der Antriebseinheit und der Aufnahme eingreifen. Gleichermaßen kann eine von der Antriebseinheit und der Aufnahme eine oder mehrere Vorsprünge aufweisen, die infolge einer Verschiebung der Antriebseinheit innerhalb der Aufnahme, vorzugsweise durch Aufbringen einer Vorspannkraft, in entsprechende Aussparungen der anderen von der Antriebseinheit und der Aufnahme eingreifen und/oder in diesen verschoben werden. In einer Ausführung erstreckt sich eine Aussparung quer, insbesondere senkrecht, zu einer Einführrichtung der Antriebseinheit in die Aufnahme, so dass ein Vorsprung nach Einführen der Antriebseinheit in die Aufnahme quer zur Einführrichtung in der Aussparung verschoben werden kann und in dieser verschobenen Stellung die Antriebseinheit formschlüssig gegen ein Herausziehen aus der Aufnahme sichert. Vorzugsweise erfolgt diese Verschiebung durch Aufprägen der Vorspannkraft, so dass die Verschiebung nach Wegfall der Vorspannkraft reversibel ist, um die Antriebseinheit wieder aus der Aufnahme herauszuziehen.

Die Aufnahme kann in einer Ausführung eine ein- oder mehrteilige, insbesondere formschlüssige, Führung zum Einführen der Antriebseinheit in einer Einführrichtung aufweisen. Die Führung kann insbesondere eine oder mehrere Führungsnuten und/oder -rippen aufweisen, die dazu ausgebildet sind, mit entsprechenden Vorsprüngen bzw. Vertiefungen an der Antriebseinheit formschlüssig zusammenzuwirken. Auf diese Weise kann das Verbinden bzw. Lösen von Antriebseinheit und Instrumentenschaft verbessert werden.

Zusätzlich oder alternativ kann die Aufnahme in einer Ausführung eine Einführöffnung zum Einführen der Antriebseinheit in einer Einführrichtung aufweisen. Die Einführöffnung kann in einer Weiterbildung, insbesondere durch eine verschwenk- und/oder -schiebbare Klappe, verschließbar sein, um die Antriebseinheit entgegen der Einführrichtung zu sichern, insbesondere festzulegen.

Zusätzlich oder alternativ kann der Instrumentenschaft eine Antriebseinheit-Verriegelungsanordnung zum, insbesondere form- und/oder reibschlüssigen, Verriegeln der Antriebseinheit in der Aufnahme aufweisen, insbesondere eines beweglichen, vorzugsweise vorgespannten, Riegels, der in der Antriebseinheit einrastet, wenn diese in der Aufnahme platziert ist.

Zusätzlich oder alternativ kann die Aufnahme relativ zu einer Längsachse des Instrumentenschafts beweglich, insbesondere verschwenkbar, sein. Dies ermöglicht es in einer Ausführung, die Antriebseinheit zunächst, wenigstens teilweise, in die in eine Aufnahmestellung bewegte, insbesondere verschwenkte Aufnahme einzuführen, und dann die Aufnahme in eine Verriegelungsstellung zu bewegen, insbesondere zu verschwenken, wobei die Antriebseinheit vorzugsweise in der Verriegelungsstellung der Aufnahme formschlüssig fixiert ist. Auf diese Weise kann insbesondere der Zugang zur Aufnahme verbessert und zugleich eine Fixierungsfunktionalität der Antriebseinheit in der Aufnahme integriert werden.

Die Einführrichtung kann in einer Ausführung, wenigstens im Wesentlichen, senkrecht zur Längsachse des Instrumentenschaftes sein. Die Einführöffnung kann dann insbesondere auf der instrumentenschaftabgewandten Seite angeordnet sein, insbesondere, um einen Antriebseinheitenwechsel bei teilweise in einen Patienten eingeführtem Instrumentenschaft zu erleichtern. Gleichermaßen kann die Einführöffnung in einer Ausführung auf der instrumentenschaftzugewandten Seite angeordnet sein, insbesondere, um eine Interferenz zwischen mehreren kooperierenden chirurgischen Instrumenten zu vermeiden.

In einer anderen Ausführung kann die Einführrichtung, wenigstens im Wesentlichen, parallel zur Längsachse des Instrumentenschaftes sein. Die Einführöffnung kann dann wiederum insbesondere auf der instrumentenschaftabgewandten Seite angeordnet sein, insbesondere, um einen Antriebseinheitenwechsel bei teilweise in einen Patienten eingeführtem Instrumentenschaft zu erleichtern.

Nach einem Aspekt der vorliegenden Offenbarung stehen ein oder mehrere bewegliche Eintriebsglieder einer Eintriebsgliedanordnung zum Aktuieren eines Freiheitsgrades eines Instrumentenschaftes, wenigstens im Wesentlichen, senkrecht zu einer Längsachse des Instrumentenschaftes zu einer Aufnahme des Instrumentenschaftes für eine Antriebseinheit hin vor. In einer Ausführung ist eine Schnittstelle bzw. Kontaktebene der Eintriebsgliedanordnung, wenigstens im Wesentlichen, parallel zur Längsachse.

Indem die Eintriebsglieder nicht parallel zur Längsachse des Instrumentenschaftes vorstehen, wie dies in der eingangs genannten WO 2011/143022 A1 der Fall ist, sondern nach diesem Aspekt wenigstens im Wesentlichen, senkrecht, interferieren in einer Ausführung vorteilhaft Deformationen des Instrumentenschaftes nicht oder nur weniger mit der Kopplung der Ab- und Eintriebsanordnung. Auf diese Weise kann vorzugsweise insbesondere eine Longitudinalschwingung im Instrumentenschaft, wenigstens teilweise, entkoppelt werden.

Insbesondere, um ein Einführen einer Antriebseinheit in eine Aufnahme eines Instrumentenschaftes zu verbessern, kann in einer Ausführung der vorliegenden Erfindung eine Eintriebsgliedanordnung des Instrumentenschaftes und/oder eine Abtriebsgliedanordnung der Antriebseinheit in einer Vertiefung, insbesondere in Koppelrichtung, angeordnet sein. Zusätzlich oder alternativ kann die Antriebseinheit ein, insbesondere konvergentes und/oder bewegliches, Verdrängungsmittel zum Verdrängen der Eintriebsgliedanordnung des Instrumentenschaftes bei einem Einführen der Antriebseinheit in die Aufnahme des Instrumentenschaftes aufweisen. Das bewegliche Verdrängungsmittel kann insbesondere eine oder mehrere drehbare Rollen aufweisen, welche beim Einführen überdurchschnittlich vorstehende Eintriebsglieder einer Eintriebsgliedanordnung zurückdrängen und so die Eintriebsgliedanordnung nivellieren. Zusätzlich oder alternativ kann das Verdrängungsmittel eine in Einführrichtung konvergierende, insbesondere abgeschrägte oder konvexe, Fläche zum Zurückdrängen überdurchschnittlich vorstehender Eintriebsglieder aufweisen. Nach Überlaufen der Rolle(n) und/oder konvexen Fläche, stehen die Eintriebsglieder vorzugsweise, wenigstens im Wesentlichen, gleichmäßig zu der Aufnahme des Instrumentenschaftes hin vor. An eine in Einführrichtung konvergierende, insbesondere abgeschrägte oder konvexe, Fläche kann sich in einer Weiterbildung eine in Einführrichtung divergierende, insbesondere gegensinnig abgeschrägte oder konvexe, Fläche anschließen, um auch bei einem Ausführen der Antriebseinheit aus der Aufnahme vorstehende Eintriebsglieder zurückzudrängen.

Ein chirurgisches Instrument nach einem Aspekt der vorliegenden Offenbarung weist ein Antriebsmodul mit einem oder mehreren drehbaren Abtriebsgliedern auf. Ein Abtriebsglied ist in einer Ausführung eine Abtriebswelle eines Aktuators des Antriebsmoduls, insbesondere eines Elektromotors oder eines damit gekoppelten Getriebes. In einer Ausführung ist ein Abtriebsglied unbegrenzt drehbar, in einer anderen Ausführung um höchstens 360°, vorzugsweise um höchstens 215°.

Das chirurgische Instrument weist weiter einen Instrumentenschaft auf, der mit dem Antriebsmodul lösbar verbindbar, insbesondere verbunden ist. Der Instrumentenschaft weist einen oder mehrere, insbesondere intrakorporale, Freiheitsgrade auf.

In einer Ausführung weist der Instrumentenschaft ein starres, gelenkiges oder biegsames Rohr auf, an dessen distalem Ende ein Endeffektor, insbesondere ein Skalpell, eine Zange, Schere, Klemme, Nadel, Pipette oder dergleichen angeordnet sein kann. Der Endeffektor kann eine Öffnung zum Ein- und/oder Austreten von elektromagnetischer Strahlung, insbesondere eine Optik einer Kamera oder eines Lasers, und/oder von gasförmigen und/oder flüssigen Fluid, insbesondere eine Saug- bzw. Spüldüse, aufweisen.

Der Endeffektor kann einen oder mehrere funktionale Freiheitsgrade, beispielsweise das Öffnen und Schließen einer Zange oder Öffnung, aufweisen. Zusätzlich oder alternativ kann der Endeffektor einen oder mehrere kinematische Freiheitsgrade, beispielsweise das Drehen und/oder Verschieben einer Zange oder Öffnung, aufweisen. Ein intrakorporaler Freiheitsgrad des Instrumentenschaftes kann insbesondere ein funktionaler oder kinematischer Freiheitsgrad eines Endeffektors oder ein Gelenk- bzw. elastischer Freiheitsgrad des gelenkigen oder biegsamen Rohrs sein. In einer Ausführung weist das Rohr einen oder mehrere Drehfreiheitsgrade um seine Längsachse auf. Diese können durch intra- und/oder extrakorporale Drehgelenke implementiert sein. Zur kompakteren Darstellung werden auch solche Drehfreiheitsgrade um die Rohrlängsachse als intrakorporale Freiheitsgrade des Instrumentenschaftes bezeichnet, da sie eine Drehbarkeit eines intrakorporalen Schaftendes, insbesondere Endeffektors darstellen. Um einen oder mehrere Freiheitsgrade des Instrumentenschaftes durch das damit verbundene Antriebsmodul zu aktuieren, weist der Instrumentenschaft ein oder mehrere verschiebbar geführte Eintriebsglieder auf, die mit einem Abtriebsglied des Antriebsmoduls gekoppelt werden bzw. sind, wenn Antriebsmodul und Instrumentenschaft miteinander verbunden sind. Ein Eintriebsglied aktuiert in einer Ausführung einen oder mehrere Freiheitsgrade des Instrumentenschaftes. Gleichermaßen können auch mehrere Eintriebsglieder denselben Freiheitsgrad aktuieren. In einer Ausführung ist ein Eintriebsglied durch ein oder mehrere, insbesondere gegensinnige, Zug- und/oder Druckmittel, beispielsweise Seilzüge oder Druckstangen, mit dem Rohr oder dem Endeffektor des Instrumentenschaftes verbunden, wobei das Zug- und/oder Druckmittel vorzugsweise, wenigstens im Wesentlichen, parallel zu einer Verschiebeachse des Eintriebsgliedes ist. In einer Ausführung ist ein Eintriebsglied formschlüssig und/oder zwischen zwei Endanschlägen, verschiebbar geführt.

Nach einem Aspekt der vorliegenden Offenbarung wird somit eine Drehbewegung wenigstens eines Abtriebsgliedes in einer Schnittstelle zwischen dem Antriebsmodul und dem Instrumentenschaft in eine translatorische, insbesondere lineare, Bewegung eines mit dem Abtriebsglied gekoppelten Eintriebsgliedes umgesetzt.

Hierzu sind Ab- und Eintriebsglied nach einem Aspekt in der Schnittstelle kreuzschubkurbelartig koppelbar bzw. gekoppelt. Nach einem Aspekt der vorliegenden Offenbarung weist die Schnittstelle eine, insbesondere gerade bzw. lineare, Nut und ein Führungselement auf, das in der Nut verschiebbar geführt ist, wenn Ab- und Eintriebsglied miteinander gekoppelt sind.

In einer Ausführung ist die Nut an, insbesondere in, dem Eintriebsglied angeordnet. Die Nut kann in einer Weiterbildung quer, insbesondere wenigstens im Wesentlichen senkrecht zu einer Verschiebeachse des verschiebbar geführten Eintriebsgliedes sein bzw. mit dieser einen Winkel einschließen, dessen Betrag vorzugsweise zwischen 45° und 90° liegt. Das Führungselement ist, vorzugsweise exzentrisch, an dem drehbaren Abtriebsglied angeordnet. Die Drehachse des drehbaren Abtriebsgliedes ist in einer Weiterbildung quer, insbesondere wenigstens im Wesentlichen senkrecht zu einer Verschiebeachse des verschiebbar geführten Eintriebsgliedes und/oder der Nut. Insbesondere kann in einer Ausführung die Drehachse mit der Verschiebeachse und/oder mit der Nut jeweils einen Winkel einschließen, dessen Betrag vorzugsweise zwischen 45° und 90° liegt.

Gleichermaßen kann umgekehrt die Nut an dem Abtriebsglied angeordnet sein, das Führungselement entsprechend an dem Eintriebsglied.

Das Eintriebsglied ist in einer Ausführung verschiebbar an dem Instrumentenschaft geführt. Zusätzlich oder alternativ kann es an dem mit dem Instrumentenschaft verbundenen Antriebsmodul verschiebbar geführt sein. Insbesondere kann das Eintriebsglied mit einem größeren Spiel, insbesondere lose, verschiebbar an dem Instrumentenschaft geführt sein und mit einem kleineren Spiel, insbesondere wenigstens im Wesentlichen spielfrei, an dem Antriebsmodul verschiebbar geführt sein, wenn dieses mit dem Instrumentenschaft verbunden ist. Hierdurch kann die aufwändigere, präzisere Führung auf das Antriebsmodul verlagert und so der Instrumentenschaft einfacher und/oder kostengünstiger, insbesondere leichter sterilisierbar und/oder als Einmalartikel ausgebildet werden bzw. sein. Sobald Instrumentenschaft und Antriebsmodul verbunden sind, übernimmt dieses die - präzisere - Führung des Eintriebsgliedes. In einer Ausführung ist das Eintriebsglied unverlierbar, insbesondere formschlüssig, an dem Instrumentenschaft gesichert.

In einer Ausführung weist das Führungselement einen oder mehrere drehbar gelagerte Wälzkörper zum Kontaktieren der Nut auf. Hierdurch kann in einer Ausführung vorteilhaft die Reibung zwischen Führungselement und Nut reduziert werden. In einer Weiterbildung weist das Führungselement einen Zapfen auf, auf dem wenigstens ein Wälzkörper in Form eines Laufrings gleitgelagert ist, der ebenfalls einen Wälzkörper im Sinne der vorliegenden Offenbarung darstellen kann. Zur kompakteren Darstellung werden also auch ein oder mehrere konzentrische Ringe, deren Inner(st)er auf dem Zapfen angeordnet ist und deren Äußer(st)er die Nut kontaktiert, und von denen wenigstens einer radial innen und/oder außen gleitgelagert ist, zur kompakteren Darstellung verallgemeinernd als Wälzkörper bezeichnet, auch wenn sie keine Roll- bzw. Abwälzbewegung ausführen. In einer anderen Ausführung sind zwischen dem Zapfen und dem Laufring mehrere in Umfangsrichtung verteilte Wälzkörper, insbesondere eines Kugel-, Nadel- oder Zylinderrollenlagers, angeordnet. In einer anderen Ausführung sind auf dem Zapfen ein oder mehrere Wälzkörper, insbesondere eines außenringlosen Kugel-, Nadel- oder Zylinderrollenlagers, angeordnet, die die Nut kontaktieren, wenn Ab- und Eintriebselement gekoppelt sind.

Zum Koppeln von Ab- und Eintriebselement kann ein Spiel zwischen der Nut und dem Führungselement in der Verschiebeachse vorteilhaft sein. Auf der anderen Seite ist zur präzisen Aktuierung des Instrumentenschaftes durch das Antriebsmodul eine möglichst spielfreie Kopplung in dieser Achse vorteilhaft. Daher ist in einer Ausführung der vorliegenden Offenbarung ein Toleranzelement vorgesehen, welches das Abtriebsglied und das Eintriebsglied in der Verschiebeachse des Eintriebsgliedes verspannt, wenn Ab- und Eintriebsglied miteinander gekoppelt sind. In einer Weiterbildung verspannt das Toleranzelement das am Abtriebsglied angeordnete Führungsglied gegen das Eintriebsglied oder das am Eintriebsglied angeordnete Toleranzelement das Führungsglied gegen das Abtriebsglied. Dieses Toleranzelement kann in einer Ausführung eine präzise Übertragung von Bewegungen zwischen Ab- und Eintriebsglied bewirken und andererseits beim An- bzw. Abkoppeln gegen seine Vorspannung ausweichen und dieses so verbessern. In einer Ausführung weist das Toleranzelement eine Toleranzelementnut auf, die vorzugsweise, wenigstens im Wesentlichen parallel zu der Nut der Schnittstelle ist und von dem Führungselement durchgriffen wird, wenn Ab- und Eintriebselement gekoppelt sind.

In einer Weiterbildung ist das Toleranzelement an dem Eintriebsglied und/oder dem Führungselement verschiebbar geführt und gegen dieses elastisch vorgespannt. Gleichermaßen kann es integral mit dem Eintriebsglied oder Führungselement ausgebildet sein, insbesondere durch einen Hohlraum, in den ein integraler Schenkel einfedern kann, der ein- oder beidseitig gelagert ist.

Das Toleranzelement ist in einer Ausführung parallel zu einer Verschiebeachse des verschiebbar geführten Eintriebsglieds verschiebbar geführt und vorgespannt. Zusätzlich oder alternativ kann das Toleranzelement axial auf dem Führungselement geführt und vorgespannt sein. In einer Ausführung weisen die Nut und das Führungselement, insbesondere ein Wälzkörper des Führungselementes, und/oder das Toleranzelement, insbesondere gegensinnige, komplementäre Schrägen auf. Insbesondere durch axiales Zustellen solcher Schrägen kann in einer Ausführung ebenfalls das Toleranzelement (auch) in Verschiebeachsen vorspannen und so die Führung des Führungselements in der Nut verbessern. Eine oder mehrere der Schrägen können in einer Weiterbildung konvex, insbesondere bogenförmig ausgebildet sein, vorzugsweise in der Art eines Axial-Pendelrollenlagers mit asymmetrischen Tonnenrollen.

Um Ab- und Eintriebsglied miteinander beim oder nach dem Verbinden von Antriebsmodul und Instrumentenschaft zu koppeln bzw. vor oder beim Lösen von Antriebsmodul und Instrumentenschaft voneinander zu entkoppeln, ist das Führungselement in einer Ausführung axial verschiebbar gelagert. Hierdurch kann es axial in die Nut ein- bzw. aus dieser ausgeführt werden.

In einer Weiterbildung ist das axial verschiebbar gelagerte Führungselement axial vorgespannt. Auf diese Weise kann es in einer Ausführung selbsttätig in die Nut einfahren und/oder in dieser elastisch gesichert sein.

In einer Ausführung ist eine Kulisse zum axialen Verschieben des Führungselements vorgesehen. Auf diese Weise kann durch Drehen des Abtriebsgliedes zunächst über die Kulisse das Führungselement verschoben und so in und/oder außer Eingriff mit der Nut gebracht werden. Die Kulisse kann insbesondere eine oder mehrere Schrägen in Drehrichtung aufweisen, auf denen ein Vorsprung, vorzugsweise ein Bund, des Führungselements durch Drehen des Abtriebsglieds aufläuft und das Führungselement so axial verschiebt. In einer Weiterbildung weist die Kulisse zwei in Drehrichtung voneinander beabstandete gegensinnige Schrägen auf, an denen der Vorsprung in in Drehrichtung voneinander beabstandeten Drehstellungen aufläuft und das Führungselement auf diese Weise in den verschiedenen Drehstellungen gegensinnig axial verschiebt.

Der Drehbereich zum axialen Verschieben des Führungselements schließt in einer Ausführung an einen Drehbereich des Abtriebsgliedes zum Aktuieren des damit gekoppelten Eintriebsgliedes an. Auf diese Weise kann durch (Weiter)Drehen des Abtriebsgliedes das Eintriebsglied an- oder abgekoppelt und anschließend bzw. zuvor das Eintriebsglied aktuiert werden.

Das Führungselement kann an dem Ab- oder Eintriebsglied axial verschiebbar gelagert und vorgespannt sein. Gleichermaßen kann das Führungselement zusammen mit dem Ab- oder Eintriebsglied axial verschiebbar gelagert und vorgespannt sein. Insbesondere hierzu kann das Abtriebsglied an dem Antriebsmodul und/oder das Eintriebsglied an dem Instrumentenschaft, vorzugsweise parallel zur Drehachse des Abtriebsgliedes, verschiebbar gelagert und vorgespannt sein.

Um Ab- und Eintriebsglied miteinander beim oder nach dem Verbinden von Antriebsmodul und Instrumentenschaft zu koppeln bzw. vor oder beim Lösen von Antriebsmodul und Instrumentenschaft voneinander zu entkoppeln, weist in einer Ausführung eine Führungswand der Nut eine Öffnung zum Einführen des Führungselementes durch Drehen des Abtriebsgliedes auf. Hierdurch kann das Führungselement in die Nut ein- bzw. aus dieser herausgedreht werden. Die Öffnung kann insbesondere durch einen verkürzten Schenkel eines offenen bzw. U-förmigen oder im Übrigen geschlossenen bzw. O-förmigen Schenkelpaares ausgebildet sein, das seinerseits die Nut definieren kann.

Es kann, insbesondere zur Erfassung einer Koordinate eines Freiheitsgrades des Instrumentenschaftes auf Basis der Drehstellung des damit gekoppelten Abtriebsgliedes, vorteilhaft sein, wenn An- und Eintriebsglied bzw. Führungselement und Nut, wenigstens im Wesentlichen, eineindeutig miteinander koppeln, so dass jeder Position des Eintriebsgliedes in seiner Verschiebeachse genau eine Drehstellung des Abtriebsgliedes entspricht.

Insbesondere daher ist in einer Ausführung die Nut asymmetrisch zur Drehachse des Abtriebsgliedes und/oder einer Verschiebeachse des Eintriebsglieds ausgebildet. In einer Weiterbildung erstreckt sie sich, wenigstens im Wesentlichen, nur bis zu dieser Drehachse.

In einer Ausführung ist das Eintriebsglied mit genau einem Zug- und/oder Druckmittel verbunden, das, wenigstens im Wesentlichen, parallel zu einer Verschiebeachse des Eintriebsgliedes ist. Hierdurch kann eine Bewegung des Eintriebsglieds vorteilhaft präzise und einfach in eine Aktuierung eines Freiheitsgrades des Instrumentenschaftes umgesetzt werden.

Ein chirurgisches Instrument nach der vorliegenden Offenbarung kann insbesondere als minimalinvasives und/oder robotergeführtes Instrument verwendet werden. Hierzu weist in einer Ausführung das Instrument, insbesondere der Instrumentenschaft und/oder das Antriebsmodul, eine Schnittstelle zur Anbindung an einen Roboter auf. Nach einem Aspekt der vorliegenden Offenbarung wird entsprechend ein Roboter mit einem damit, vorzugsweise lösbar, über eine Schnittstelle verbundenen Instrument unter Schutz gestellt, wie es hier offenbart ist. Gleichermaßen wird ein Antriebsmodul bzw. ein Instrumentenschaft für ein solches chirurgisches Instrument unter Schutz gestellt, das bzw. der eine oder mehrere Nuten oder Führungselemente der hier offenbarten Schnittstelle des chirurgischen Instruments aufweist, um mit entsprechenden Führungselementen bzw. Nuten eines Instrumentenschaftes bzw. Antriebsmoduls zu koppeln.

Nach einem Aspekt der vorliegenden Offenbarung werden bei oder nach einem Verbinden des Antriebsmoduls und des Instrumentenschaftes eines vorstehend erläuterten chirurgischen Instruments das bzw. die Führungselemente in die entsprechende(n) Nut(en) rotiert oder axial eingeschoben, um Ab- und Eintriebsglied(er) zu koppeln. Zum Entkoppeln werden beim oder vor dem Lösen der Verbindung des Antriebsmoduls und des Instrumentenschaftes das bzw. die Führungselemente aus den entsprechende(n) Nut(en) herausrotiert oder axial herausgezogen.

Die vorliegende Offenbarung betrifft insbesondere folgende Ausgestaltungen: Eine erste Ausgestaltung betrifft eine chirurgische Instrumentenanordnung, mit einer modularen motorischen Antriebseinheit, die eine Abtriebsanordnung mit wenigstens einem Abtriebselement aufweist und einem Instrumentenschaft, der lösbar mit der Antriebseinheit verbindbar ist und eine Antriebsanordnung mit wenigstens einem Eintriebselement aufweist, wobei die Abtriebsanordnung und die Antriebsanordnung mittels einer mechanischen Schnittstelle miteinander koppelbar sind, die wenigstens eine einseitige Bindung, einen Zapfen und eine Aussparung, wobei der Zapfen in der Aussparung radial, insbesondere elastisch und/oder durch wenigstens einen separaten Körper, aufweitbar ist, und/oder zwischen dem Zapfen und der Aussparung ein in radialer Richtung wellenförmiger Spalt ausgebildet ist, in dem eine radial verschiebbare, axial feste Zwischenelementanordnung angeordnet ist und/oder einen Kipphebel aufweist.

Eine zweite Ausgestaltung betrifft eine Instrumentenanordnung nach der vorhergehenden Ausgestaltung, wobei wenigstens ein Ab- und/oder Eintriebselement translatorisch oder rotatorisch verstellbar geführt ist.

Eine dritte Ausgestaltung betrifft eine Instrumentenanordnung nach einer der vorhergehenden Ausgestaltungen, wobei wenigstens ein Ab- und/oder Eintriebselement derart mit einem Koppelmittel gekoppelt ist, dass eine von einer translatorischen oder rotatorischen Bewegung von dem einen von dem Element und dem Koppelmittel in die andere von einer translatorischen oder rotatorischen Bewegung von dem anderen von dem Element und dem Koppelmittel umgesetzt wird.

Eine vierte Ausgestaltung betrifft eine Instrumentenanordnung nach der vorhergehenden Ausgestaltung, wobei das Koppelmittel ein Drehschublager und/oder eine Verzahnung aufweist.

Eine fünfte Ausgestaltung betrifft eine Instrumentenanordnung nach einer der vorhergehenden Ausgestaltungen, wobei der Instrumentenschaft einen Flansch aufweist, und wobei die mechanische Schnittstelle auf einer endeffektorzugewandten, endeffektorabgewandten oder seitlichen Fläche dieses Flansches angeordnet ist.

Eine sechste Ausgestaltung betrifft eine Instrumentenanordnung nach einer der vorhergehenden Ausgestaltungen, wobei die Abtriebsanordnung und/oder die Antriebsanordnung wenigstens ein Paar gegensinniger Ab- bzw. Eintriebselemente aufweist.

Eine siebte Ausgestaltung betrifft eine Instrumentenanordnung nach einer der vorhergehenden Ausgestaltungen, mit einem Ausgleichmittel zum Toleranzausgleich.

Eine achte Ausgestaltung betrifft eine Instrumentenanordnung nach einer der vorhergehenden Ausgestaltungen, wobei wenigstens ein Ab- und/oder Eintriebselement entgegen seiner Verstellrichtung vorgespannt ist.

Eine neunte Ausgestaltung betrifft eine Instrumentenanordnung nach einer der vorhergehenden Ausgestaltungen, wobei wenigstens eine von einander zugewandten Stirnseiten eines Ab- und eines damit koppelbaren Eintriebselements eben oder ballig ausgebildet sind und/oder einen Vorsprung zum Eingreifen in eine Aussparung in der anderen Stirnseite aufweisen.

Eine zehnte Ausgestaltung betrifft eine Instrumentenanordnung nach einer der vorhergehenden Ausgestaltungen, mit einem Klemmmittel zum radialen Aufweiten eines in eine Aussparung eingeführten Zapfens der mechanischen Schnittstelle.

Eine elfte Ausgestaltung betrifft eine chirurgische Instrumentenanordnung, insbesondere nach einer der vorhergehenden Ausgestaltungen, mit einer modularen motorischen Antriebseinheit, die eine Abtriebsanordnung mit wenigstens einem Abtriebselement aufweist, einem Instrumentenschaft, der lösbar mit der Antriebseinheit verbindbar ist und eine Antriebsanordnung mit wenigstens einem Eintriebselement aufweist, wobei die Abtriebsanordnung und die Antriebsanordnung mittels einer mechanischen Schnittstelle miteinander koppelbar sind, und mit einer Sterilbarriere, die dazu vorgesehen ist, die Antriebseinheit zu umhüllen und zwischen der Antriebseinheit und dem Instrumentenschaft angeordnet zu werden, und die im Bereich der mechanischen Schnittstelle eine, insbesondere vorgespannte, Lose in einer Verstellrichtung der Ab- und Antriebsanordnung, eine berührungslose translatorisch verschiebbare Dichtung, ein Ausgleichmittel zum Toleranzausgleich und/oder einen Elementfortsatz aufweist, der lösbar mit einem von einer Ab- und einem Eintriebselementbasis verbindbar ist, welches die Sterilbarriere zerstörend durchgreift.

Eine zwölfte Ausgestaltung betrifft eine chirurgische Instrumentenanordnung, insbesondere nach einer der vorhergehenden Ausgestaltungen, mit einer modularen motorischen Antriebseinheit, die eine Abtriebsanordnung mit wenigstens einem Abtriebselement aufweist, einem Instrumentenschaft, der lösbar mit der Antriebseinheit verbindbar ist und eine Antriebsanordnung mit wenigstens einem Eintriebselement aufweist, wobei die Abtriebsanordnung und die Antriebsanordnung mittels einer mechanischen Schnittstelle miteinander koppelbar sind, einer Sterilbarriere, die dazu vorgesehen ist, die Antriebseinheit zu umhüllen, und mit einem Befestigungselement zur lösbaren Verbindung mit der Antriebseinheit, welches dazu vorgesehen ist, auf einer der Antriebseinheit abgewandten Oberfläche der Sterilbarriere angeordnet zu werden.

Eine dreizehnte Ausgestaltung betrifft ein Manipulatorchirurgiesystem mit wenigstens einem Manipulator und wenigstens einer manipulatorgeführten Instrumentenanordnung nach einer der vorhergehenden Ausgestaltungen, deren Antriebseinheit und Instrumentenschaft miteinander verbunden sind.

Eine vierzehnte Ausgestaltung betrifft ein Verfahren zum Bestücken eines Manipulators eines Manipulatorchirurgiesystems nach der vorhergehenden Ausgestaltung, wobei eine modulare motorische Antriebseinheit und ein Instrumentenschaft der Instrumentenanordnung lösbar miteinander verbunden und die Abtriebsanordnung und die Antriebsanordnung mittels der mechanischen Schnittstelle miteinander gekoppelt werden.

Eine fünfzehnte Ausgestaltung betrifft eine Antriebsstranganordnung mit wenigstens einem Antriebsstrang zur Aktuierung eines Freiheitsgrades eines Endeffektors eines, insbesondere robotergeführten, chirurgischen Instruments relativ zu einem Instrumentenschaft durch einen Antrieb, mit einer an dem Antriebsstrang angeordneten Messanordnung zum Erfassen einer Last in dem Antriebsstrang.

Eine sechszehnte Ausgestaltung betrifft eine Antriebsstranganordnung nach der vorhergehenden Ausgestaltung, wobei die Antriebsstranganordnung wenigstens zwei, insbesondere gegensinnige, Antriebsstränge zur Aktuierung desselben Freiheitsgrades des Endeffektors aufweist, wobei die Messanordnung wenigstens ein Messmittel aufweist, welches an einem dieser Antriebsstränge zum Erfassen einer Last in diesem Antriebsstrang angeordnet ist.

Eine siebzehnte Ausgestaltung betrifft eine Antriebsstranganordnung nach der vorhergehenden Ausgestaltung, wobei die Messanordnung ein erstes Messmittel, welches an einem ersten Antriebsstrang zum Erfassen einer Last in diesem Antriebsstrang angeordnet ist, und ein zweites Messmittel aufweist, welches an einem zweiten, insbesondere gegensinnigen, Antriebsstrang zum Erfassen einer Last in diesem Antriebsstrang angeordnet ist, wobei derselbe Freiheitsgrades des Endeffektors durch den ersten und zweiten Antriebsstrang aktuierbar ist, und wobei das erste und zweite Messmittel signaltechnisch miteinander verknüpft sind.

Eine achtzehnte Ausgestaltung betrifft eine Antriebsstranganordnung nach der vorhergehenden Ausgestaltung, wobei das erste und zweite Messmittel kompensatorisch, insbesondere in zwei Zweigen einer Wheatstoneschen Brückenschaltung miteinander verknüpft sind.

Eine neunzehnte Ausgestaltung betrifft eine Antriebsstranganordnung nach der vorhergehenden Ausgestaltung, wobei die Messanordnung ein drittes Messmittel, welches, insbesondere dem ersten Messmittel gegenüberliegend, an dem ersten Antriebsstrang zum Erfassen einer Last in diesem Antriebsstrang angeordnet ist, und ein viertes Messmittel aufweist, welches, insbesondere dem zweiten Messmittel gegenüberliegend, an dem zweiten Antriebsstrang zum Erfassen einer Last in diesem Antriebsstrang angeordnet ist, wobei die Messmittel kompensatorisch, insbesondere das erste Messmittel in einem ersten Zweig, das zweite Messmittel in einem zweiten Zweig, das dritte Messmittel in einem dritten Zweig und das vierte Messmittel in einem vierten Zweig der Wheatstoneschen Vollbrückenschaltung, miteinander verknüpft sind.

Eine zwanzigste Ausgestaltung betrifft eine Antriebsstranganordnung nach einer der vorhergehenden Ausgestaltungen, wobei ein Messmittel der Messanordnung einen, insbesondere elektrischen, magnetoelastischen, optischen und/oder akustischen, Dehnungsmesser zum Erfassen einer mechanischen Last aufweist, insbesondere ist.

Eine einundzwanzigste Ausgestaltung betrifft eine Antriebsstranganordnung nach einer der vorhergehenden Ausgestaltungen, wobei ein Messmittel der Messanordnung, wenigstens im Wesentlichen, zum Erfassen einer axialen Zug- und/oder Drucklast in einem Antriebsstrang an diesem Antriebsstrang angeordnet ist.

Eine zweiundzwanzigste Ausgestaltung betrifft eine Antriebsstranganordnung nach einer der vorhergehenden Ausgestaltungen, wobei ein Messmittel der Messanordnung in einer Aussparung und/oder in einem Bereich einer Wandstärkenreduzierung eines Antriebsstrangs angeordnet ist.

Eine dreiundzwanzigste Ausgestaltung betrifft ein Antriebsmodul eines chirurgischen Instruments zur lösbaren Verbindung mit einem Instrumentenschaft, der einen Endeffektor aufweist, mit einem Antrieb und einer antriebsmodulseitigen Antriebsstranganordnung nach einer der vorhergehenden Ausgestaltungen zur Aktuierung wenigstens eines Freiheitsgrades des Endeffektors, welche über eine Schnittstelle mit einer instrumentenschaftseitigen Antriebsstranganordnung zur Aktuierung des Endeffektors, insbesondere einseitig, translatorisch und/oder über eine Sterilbarriere, koppelbar ist.

Eine vierundzwanzigste Ausgestaltung betrifft einen Instrumentenschaft eines chirurgischen Instruments zur lösbaren Verbindung mit einem Antriebsmodul, der einen Endeffektor und eine instrumentenschaftseitige Antriebsstranganordnung nach einer der vorhergehenden Ausgestaltungen zur Aktuierung wenigstens eines Freiheitsgrades des Endeffektors aufweist, welche über eine Schnittstelle mit einer antriebsmodulseitigen Antriebsstranganordnung zur Aktuierung des Endeffektors durch einen Antrieb des Antriebsmoduls, insbesondere einseitig, translatorisch und/oder über eine Sterilbarriere, koppelbar ist.

Eine fünfundzwanzigste Ausgestaltung betrifft ein chirurgisches Instrument mit einem Instrumentenschaft , der einen Endeffektor aufweist, und mit einem Antriebsmodul, das einen Antrieb aufweist, wobei der Instrumentenschaft eine instrumentenschaftseitige Antriebsstranganordnung und/oder das Antriebsmodul eine antriebsmodulseitige Antriebsstranganordnung nach einer der vorhergehenden Ausgestaltungen zur Aktuierung wenigstens eines Freiheitsgrades des Endeffektors aufweist.

Eine sechsundzwanzigste Ausgestaltung betrifft eine Manipulatoranordnung mit wenigstens einem Manipulator, insbesondere Roboter, und einem durch den Manipulator geführten chirurgischen Instrument nach der vorhergehenden Ausgestaltung.

Eine siebenundzwanzigste Ausgestaltung betrifft ein Verfahren zum Steuern eines Antriebs und/oder manuellen Teleoperationsmittels eines chirurgischen Instruments nach einer der vorhergehenden Ausgestaltungen, wobei der Antrieb und/oder das Teleoperationsmittel auf Basis wenigstens einer durch die Messanordnung erfassten Last gesteuert wird.

Eine achtundzwanzigste Ausgestaltung betrifft ein Steuermittel zum Steuern eines chirurgischen Instruments nach einer der vorhergehenden Ausgestaltungen, das dazu eingerichtet ist, wenigstens eine durch die Messanordnung erfasste Last weiterzuverarbeiten, insbesondere den Antrieb und/oder ein manuelles Teleoperationsmittel auf Basis wenigstens einer durch die Messanordnung erfassten Last zu steuern.

Eine neunundzwanzigste Ausgestaltung betrifft ein chirurgisches Instrument mit einem Instrumentenschaft mit einer Eintriebsgliedanordnung mit wenigstens einem beweglichen Eintriebsglied zum Aktuieren eines Freiheitsgrades des Instrumentenschaftes, insbesondere nach einer der nachfolgenden Ausgestaltungen, und einer lösbar mit dem Instrumentenschaft verbindbaren Antriebseinheit nach einer der nachfolgenden Ausgestaltungen.

Eine dreißigste Ausgestaltung betrifft ein chirurgisches Instrument nach der vorhergehenden Ausgestaltung, wobei die Koppelrichtung, in der das Antriebsmodul in dem Gehäuse der Antriebseinheit beweglich gelagert und vorgespannt ist, mit einer Längsachse des Instrumentenschaftes einen Winkel einschließt, dessen Betrag größer als 0°, insbesondere größer als 45°, insbesondere wenigstens im Wesentlichen 90° ist.

Eine einunddreißigste Ausgestaltung betrifft ein chirurgisches Instrument nach der vorhergehenden Ausgestaltung, mit einer Antriebseinheit-Verriegelungsanordnung zum Verriegeln der Antriebseinheit in einer Aufnahme des Instrumentenschaftes.

Eine zweiunddreißigste Ausgestaltung betrifft ein chirurgisches Instrument nach einer der vorhergehenden Ausgestaltungen, mit einer Schnittstelle zum Befestigen an einem Roboter.

Eine dreiunddreißigste Ausgestaltung betrifft einen Instrumentenschaft mit einer Eintriebsgliedanordnung mit wenigstens einem beweglichen Eintriebsglied zum Aktuieren eines Freiheitsgrades des Instrumentenschaftes, welches, wenigstens im Wesentlichen, senkrecht zu einer Längsachse des Instrumentenschaftes zu einer Aufnahme des Instrumentenschaftes für eine Antriebseinheit hin vorsteht.

Eine vierunddreißigste Ausgestaltung betrifft einen Instrumentenschaft nach der vorhergehenden Ausgestaltung, wobei die Aufnahme eine Führung und/oder eine, insbesondere verschließbare, Einführöffnung zum Einführen einer Antriebseinheit in einer Einführrichtung aufweist, welche insbesondere, wenigstens im Wesentlichen, senkrecht oder parallel zur Längsachse des Instrumentenschaftes ist.

Eine fünfunddreißigste Ausgestaltung betrifft einen Instrumentenschaft nach einer der vorhergehenden Ausgestaltungen, wobei die Aufnahme relativ zu einer Längsachse des Instrumentenschafts beweglich, insbesondere verschwenkbar, ist.

Eine sechsunddreißigste Ausgestaltung betrifft einen Instrumentenschaft nach einer der vorhergehenden Ausgestaltungen, wobei die Eintriebsgliedanordnung in einer Vertiefung angeordnet ist.

Eine siebenunddreißigste Ausgestaltung betrifft eine Antriebseinheit mit einem Gehäuse und wenigstens einem Antriebsmodul mit einem Antrieb und einer Abtriebsgliedanordnung mit wenigstens einem beweglichen Abtriebsglied zum Aktuieren eines Freiheitsgrades eines Instrumentenschaftes, welches in dem Gehäuse in einer Koppelrichtung beweglich gelagert und vorgespannt ist.

Eine achtunddreißigste Ausgestaltung betrifft eine Antriebseinheit nach der vorhergehenden Ausgestaltung, mit einer Magnetanordnung zum Vorspannen des Antriebsmoduls in der Koppelrichtung.

Eine neununddreißigste Ausgestaltung betrifft eine Antriebseinheit nach einer der vorhergehenden Ausgestaltungen, mit einer, insbesondere mechanischen und/oder magnetischen, Rückzuganordnung zum Zurückziehen des Antriebsmoduls gegen die Vorspannung.

Eine vierzigste Ausgestaltung betrifft eine Antriebseinheit nach der vorhergehenden Ausgestaltung, mit einer Antriebsmodul-Verriegelungsanordnung zum Verriegeln des zurückgezogenen Antriebsmoduls.

Eine einundvierzigste Ausgestaltung betrifft eine Antriebseinheit nach einer der vorhergehenden Ausgestaltungen, wobei die Rückzuganordnung durch einen Antrieb des Antriebsmoduls aktuierbar ist.

Eine zweiundvierzigste Ausgestaltung betrifft eine Antriebseinheit nach einer der vorhergehenden Ausgestaltungen, mit einem, insbesondere konkaven und/oder beweglichen, Verdrängungsmittel zum Verdrängen einer Eintriebsgliedanordnung eines Instrumentenschafts bei einem Einführen der Antriebseinheit in eine Aufnahme des Instrumentenschaftes.

Eine dreiundvierzigste Ausgestaltung betrifft eine Antriebseinheit nach einer der vorhergehenden Ausgestaltungen, wobei die Abtriebsgliedanordnung in einer Vertiefung angeordnet ist.

Eine vierundvierzigste Ausgestaltung betrifft ein chirurgisches Instrument mit: einem Antriebsmodul mit wenigstens einem, insbesondere unbegrenzt, drehbaren Abtriebsglied, einem mit dem Antriebsmodul lösbar verbindbaren Instrumentenschaft mit wenigstens einem, insbesondere zwischen Endanschlägen, verschiebbar geführten Eintriebsglied und einer Schnittstelle zwischen dem Antriebsmodul und dem Instrumentenschaft, die ein Führungselement und eine Nut aufweist, wobei die Nut an einem von dem Ab- und Eintriebsglied und das Führungselement an dem anderen von dem Ab- und Eintriebsglied angeordnet und in der Nut verschiebbar geführt ist, wenn Ab- und Eintriebsglied miteinander gekoppelt sind.

Eine fünfundvierzigste Ausgestaltung betrifft ein chirurgisches Instrument nach der vorhergehenden Ausgestaltung, wobei das Eintriebsglied an dem Instrumentenschaft und/oder dem damit verbundenen Antriebsmodul verschiebbar geführt ist.

Eine sechsundvierzigste Ausgestaltung betrifft ein chirurgisches Instrument nach einer der vorhergehenden Ausgestaltungen, wobei das Führungselement wenigstens einen drehbar gelagerten Wälzkörper zum Kontaktieren der Nut aufweist.

Eine siebenundvierzigste Ausgestaltung betrifft ein chirurgisches Instrument nach einer der vorhergehenden Ausgestaltungen, mit einem Toleranzelement, welches das Abtriebsglied und das Eintriebsglied in einer Verschiebeachse des Eintriebsgliedes verspannt, wenn Ab- und Eintriebsglied miteinander gekoppelt sind.

Eine achtundvierzigste Ausgestaltung betrifft ein chirurgisches Instrument nach einer der vorhergehenden Ausgestaltungen, wobei das Führungselement, insbesondere vorgespannt, axial verschiebbar gelagert ist.

Eine neunundvierzigste Ausgestaltung betrifft ein chirurgisches Instrument nach der vorhergehenden Ausgestaltung, mit einer Kulisse zum axialen Verschieben des Führungselements.

Eine fünfzigste Ausgestaltung betrifft ein chirurgisches Instrument nach einer der vorhergehenden Ausgestaltungen, wobei eine Führungswand der Nut eine Öffnung zum Einführen des Führungselementes durch Drehen des Abtriebsgliedes aufweist.

Eine einundfünfzigste Ausgestaltung betrifft ein chirurgisches Instrument nach einer der vorhergehenden Ausgestaltungen, wobei die Nut asymmetrisch zu einer Drehachse des Abtriebsgliedes und/oder einer Verschiebeachse des Eintriebsglieds, insbesondere im Wesentlichen nur bis zu dieser Drehachse ausgebildet ist.

Eine zweiundfünfzigste Ausgestaltung betrifft ein chirurgisches Instrument nach einer der vorhergehenden Ausgestaltungen, wobei das Führungselement exzentrisch an dem Abtriebsglied, insbesondere einer Abtriebswelle eines Drehmotors des Antriebsmoduls, angeordnet ist.

Eine dreiundfünfzigste Ausgestaltung betrifft ein chirurgisches Instrument nach einer der vorhergehenden Ausgestaltungen, wobei die Nut quer, insbesondere senkrecht, zu einer Verschiebeachse des Eintriebsgliedes an diesem angeordnet ist.

Eine vierundfünfzigste Ausgestaltung betrifft ein chirurgisches Instrument nach einer der vorhergehenden Ausgestaltungen, wobei das Eintriebsglied mit einem Zug- und/oder Druckmittel verbunden ist, das, wenigstens im Wesentlichen, parallel zu einer Verschiebeachse des Eintriebsgliedes ist.

Eine fünfundfünfzigste Ausgestaltung betrifft ein chirurgisches Instrument nach einer der vorhergehenden Ausgestaltungen, mit einer Schnittstelle zur Anbindung an einen Roboter.

Eine sechsundfünfzigste Ausgestaltung betrifft ein Antriebsmodul für ein chirurgisches Instrument nach einer der vorhergehenden Ausgestaltungen, mit wenigstens einem, insbesondere unbegrenzt, drehbaren Abtriebsglied und dem Führungselement oder der Nut der Schnittstelle des chirurgischen Instruments.

Eine siebenundfünfzigste Ausgestaltung betrifft einen Instrumentenschaft für ein chirurgisches Instrument nach einer der vorhergehenden Ausgestaltungen, mit wenigstens einem, insbesondere zwischen Endanschlägen, verschiebbar geführten Eintriebsglied und der Nut oder dem Führungselement der Schnittstelle des chirurgischen Instruments.

Eine achtundfünfzigste Ausgestaltung betrifft ein Verfahren zum lösbaren Verbinden des Antriebsmoduls und des Instrumentenschaftes eines chirurgischen Instruments nach einer der vorhergehenden Ausgestaltungen, wobei das Führungselement axial in die Nut eingeschoben oder in die Nut rotiert wird.

Weitere Vorteile und Merkmale ergeben sich aus den Unteransprüchen und den Ausführungsbeispielen. Hierzu zeigt, teilweise schematisiert:

Fig. 1: eine mechanische Schnittstelle einer Instrumentenanordnung nach einer Ausführung der vorliegenden Offenbarung;

Fig. 2 bis 6: mechanische Schnittstellen von Instrumentenanordnungen nach weiteren Ausführungen der vorliegenden Offenbarung;

Fig. 7: verschiedene Ausführungen der einander zugewandten Stirnseiten der Ab- und Eintriebselemente der mechanischen Schnittstellen der Fig. 1 bis 6;

Fig. 8 bis 10: Ausgleichsmittel zum Toleranzausgleich;

Fig. 11 bis 15: verschiedene Ankopplungen eines instrumentenschaftseitigen Antriebsstranges an eine erfindungsgemäße mechanische Schnittstelle;

Fig. 16: mechanische Schnittstellen von Instrumentenanordnungen nach weiteren Ausführungen der vorliegenden Offenbarung;

Fig. 17 bis 19: verschiedene Ausführungen von Zapfen und Aussparung der Schnittstelle der Fig. 16;

Fig. 20: eine Instrumentenanordnung nach einer weiteren Ausführung der vorliegenden Offenbarung;

Fig. 21: eine Instrumentenanordnung nach einer weiteren Ausführung der vorliegenden Offenbarung;

Fig. 22: einen Zapfen und ein Klemmmittel der Instrumentenanordnung der Fig. 21;

Fig. 23: Schritte des weggesteuerten Ankoppelvorgangs der Instrumentenanordnung der Fig. 21;

Fig. 24: mechanische Schnittstellen von Instrumentenanordnungen nach weiteren Ausführungen der vorliegenden Offenbarung;

Fig. 25: Schritte des weggesteuerten Ankoppelvorgangs der Instrumentenanordnung der Fig. 24;

Fig. 26: verschiedene Anordnungen bzw. Fügerichtungen eines Instrumentenschafts an eine Antriebseinheit einer Instrumentenanordnung nach weiteren Ausführungen der vorliegenden Offenbarung;

Fig. 27 bis 29: mechanische Schnittstellen von Instrumentenanordnungen nach weiteren Ausführungen der vorliegenden Offenbarung;

Fig. 30: mechanische Schnittstellen von Instrumentenanordnungen nach weiteren Ausführungen der vorliegenden Offenbarung mit einer Sterilbarriere, die eine Lose in Verstellrichtung aufweist;

Fig. 31: mechanische Schnittstellen von Instrumentenanordnungen nach weiteren Ausführungen der vorliegenden Offenbarung mit einer Sterilbarriere, die eine berührungslose translatorisch verschiebbare Dichtung aufweist;

Fig. 32: eine mechanische Schnittstelle einer Instrumentenanordnung nach einer weiteren Ausführung der vorliegenden Offenbarung mit einer Sterilbarriere, die einen Elementfortsatz aufweist, der lösbar mit einer Ab- oder Eintriebselementbasis verbunden ist;

Fig. 33: eine Instrumentenanordnung nach einer weiteren Ausführung der vorliegenden Erfindung mit einem Befestigungselement in Form eines sterilen Adapters 4;

EP 2 942 027 B1

Fig. 34:     einen Teil eines chirurgischen Instruments nach einer Ausführung der vorliegenden Offenbarung;

Fig. 35:     eine signaltechnische Verknüpfung von Messmitteln einer Messanordnung des chirurgischen Instruments der Fig. 34;

Fig. 36:     ein Steuermittel bzw. Verfahren nach einer Ausführung der vorliegenden Offenbarung;

Fig. 37:     einen Teil eines robotergeführten chirurgischen Instruments nach einer Ausführung der vorliegenden Offenbarung in einem Teilschnitt;

Fig. 38:     ein Antriebsmodul und eine damit gekoppelte Eintriebsgliedanordnung des chirurgischen Instruments der Fig. 37;

Fig. 39:     ein Antriebsmodul und eine damit gekoppelte Eintriebsgliedanordnung nach einer weiteren Ausführung der vorliegenden Offenbarung in Fig. 38 entsprechender Darstellung;

Fig. 40A:    ein Antriebsmodul mit einer Rückzuganordnung nach einer weiteren Ausführung der vorliegenden Offenbarung in Fig. 38 entsprechender Darstellung in einem mit einer Eintriebsgliedanordnung gekoppelten Zustand;

Fig. 40B:    das zurückgezogene und verriegelte Antriebsmodul der Fig. 40A;

Fig. 41:     ein Antriebsmodul nach einer weiteren Ausführung der vorliegenden Offenbarung in Fig. 38 entsprechender Darstellung;

Fig. 42(a):  eine Antriebseinheit und einen Instrumentenschaft eines chirurgischen Instruments nach einer weiteren Ausführung der vorliegenden Erfindung in einem Teilschnitt;

Fig. 42(b):  eine Antriebseinheit und einen Instrumentenschaft eines chirurgischen Instruments nach einer weiteren Ausführung der vorliegenden Erfindung in Fig. 42(a) entsprechender Darstellung;

Fig. 43(a):  eine Antriebseinheit und einen Instrumentenschaft eines chirurgischen Instruments nach einer weiteren Ausführung der vorliegenden Erfindung in Fig. 42(a) entsprechender Darstellung;

Fig. 43(b):  eine Antriebseinheit und einen Instrumentenschaft eines chirurgischen Instruments nach einer weiteren Ausführung der vorliegenden Erfindung in Fig. 42(a) entsprechender Darstellung;

Fig. 44(a):  eine Antriebseinheit und einen Instrumentenschaft eines chirurgischen Instruments nach einer weiteren Ausführung der vorliegenden Erfindung in Fig. 42(a) entsprechender Darstellung;

Fig. 44(b):  eine Antriebseinheit und einen Instrumentenschaft eines chirurgischen Instruments nach einer weiteren Ausführung der vorliegenden Erfindung in Fig. 42(a) entsprechender Darstellung;

Fig. 45(a):  eine Antriebseinheit und einen Instrumentenschaft eines chirurgischen Instruments nach einer weiteren Ausführung der vorliegenden Erfindung in Fig. 42(a) entsprechender Darstellung;

Fig. 45(b):  eine Antriebseinheit und einen Instrumentenschaft eines chirurgischen Instruments nach einer weiteren Ausführung der vorliegenden Erfindung in Fig. 42(a) entsprechender Darstellung;

Fig. 46(a):  eine Antriebseinheit und einen Instrumentenschaft eines chirurgischen Instruments nach einer weiteren Ausführung der vorliegenden Erfindung in Fig. 42(a) entsprechender Darstellung;

Fig. 46(b):  eine Antriebseinheit und einen Instrumentenschaft eines chirurgischen Instruments nach einer weiteren Ausführung der vorliegenden Erfindung in Fig. 42(a) entsprechender Darstellung;

Fig. 47:     ein chirurgisches Instrument nach einer Ausführung der vorliegenden Offenbarung;

Fig.48A, 48B:  eine Schnittstelle des chirurgischen Instruments der Fig. 47 in perspektivischen Ansichten;

23

Fig. 49(a), (b):        Schritte beim Koppeln eines Führungselements mit einer Nut der Schnittstelle der Fig. 48;

Fig. 49(c)-(f):        Schritte beim Aktuieren eines Eintriebsglieds durch ein Abtriebsglied des chirurgischen Instruments der Fig. 47;

Fig. 50:        eine Schnittstelle eines chirurgischen Instruments nach einer weiteren Ausführung der vorliegenden Offenbarung in einem Teilschnitt;

Fig. 51A, 51B:        eine Schnittstelle eines chirurgischen Instruments nach einer weiteren Ausführung der vorliegenden Offenbarung in perspektivischer Ansicht (Fig. 51A) und einem Teilschnitt (Fig. 51B);

Fig. 52:        eine Schnittstelle eines chirurgischen Instruments nach einer weiteren Ausführung der vorliegenden Offenbarung in Fig. 51B entsprechender Weise;

Fig. 53A, 53B:        eine Schnittstelle eines chirurgischen Instruments nach einer weiteren Ausführung der vorliegenden Offenbarung in verschiedenen Stellungen;

Fig. 54:        eine Schnittstelle eines chirurgischen Instruments nach einer weiteren Ausführung der vorliegenden Offenbarung; und

Fig. 55A - 55D:        eine Schnittstelle eines chirurgischen Instruments nach einer weiteren Ausführung der vorliegenden Offenbarung in einer Draufsicht in Richtung einer Verschiebeachse (Fig. 55A, 55B) bzw. in perspektivischer Ansicht (Fig. 55C, 55D), wobei ein Ab- und Eintriebsglied nicht miteinander gekoppelt (Fig. 55A, 55C, 55D) bzw. miteinander gekoppelt (Fig. 55B) sind.

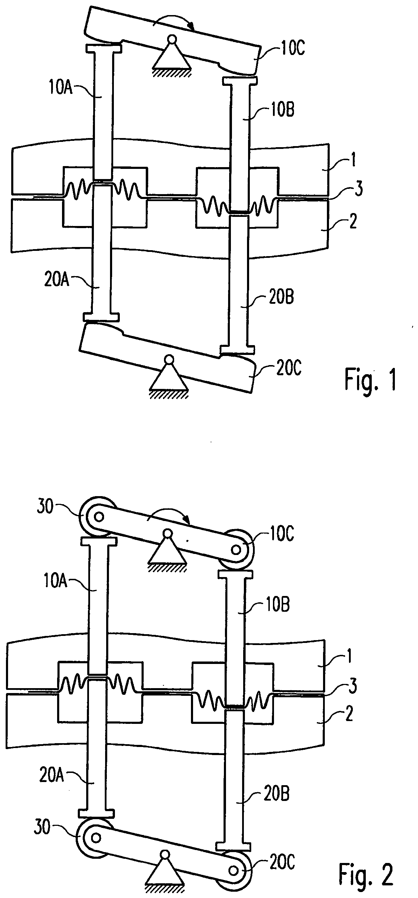

Fig. 1 zeigt eine mechanische Schnittstelle einer Instrumentenanordnung nach einer Ausführung der vorliegenden Offenbarung mit zwei gegensinnigen Abtriebselementen 10A, 10B einer Abtriebsanordnung einer modularen motorischen Antriebseinheit 1. Diese sind mit zwei Eintriebselementen 20A bzw. 20B einer Antriebsanordnung eines Instrumentenschaftes 2 gekoppelt. Eine Sterilbarriere 3 umhüllt die Antriebseinheit 1 und ist zwischen dieser und dem Instrumentenschaft 2 angeordnet.

Ab- und Eintriebselemente 10A, 10B bzw. 20A, 20B sind in der Antriebseinheit 1 bzw. dem Instrumentenschaft 2 translatorisch verstellbar geführt.

Die Abtriebselemente 10A, 10B sind mit einem als Wippe 10C ausgebildeten Koppelmittel derart gekoppelt, dass eine rotatorische Bewegung von dem Koppelmittel 10C, die in Fig. 1 durch einen Drehpfeil angedeutet ist, in eine translatorische Bewegung der Elemente 10A, 10B umgesetzt wird. Das Koppelmittel 10C kann beispielsweise mit einer Abtriebswelle eines Elektromotors der Antriebseinheit 1 verbunden oder über ein Getriebe gekoppelt sein (nicht dargestellt).

In ähnlicher Weise sind die Eintriebselemente 20A, 20B mit einem als Wippe 20C ausgebildeten weiteren Koppelmittel derart gekoppelt, dass eine translatorische Bewegung der Elemente 20A, 20B in eine rotatorische Bewegung von dem Koppelmittel 20C umgesetzt wird. An dem Koppelmittel 20C können beispielsweise axial voneinander beabstandet Zugseile oder Schubstangen des Instrumentenschaftes 2 befestigt sein, durch die ein Freiheitsgrad eines Endeffektors aktuiert, beispielsweise eine Schere geöffnet oder ein Skalpell verdreht wird (nicht dargestellt). Gleichermaßen kann die rotatorische Bewegung des Koppelmittels 20C beispielsweise über Zahnräder übertragen oder - etwa über ein Schneckengetriebe - wieder in eine translatorische Bewegung umgesetzt werden.

Sowohl Ab- und zugeordnetes Eintriebselement 10A, 20A bzw. 10B, 20B untereinander als auch die Abtriebselemente 10A, 10B und das Koppelmittel 10C sowie die Eintriebselemente 20A, 20B und das weitere Koppelmittel 20C sind jeweils durch eine einseitige Bindung miteinander gekoppelt. Man erkennt, dass das Doppelmittel 10C auf die Abtriebselemente 10A, 10B, diese auf die Eintriebselemente 20A, 20B und diese wiederum auf das weitere Koppelmittel 20C nur Druckkräfte übertragen können.

Die Ab- und Eintriebselemente sind in der Ausführung als Stößel ausgebildet, die entlang ihrer Längsachsen verstellt werden, beispielsweise durch einen Linearaktor oder eine Gelenkkinematik. Zwischen den Stößeln befindet sich die Sterilbarriere 3. Da mit einem Stößelpaar nur Druckkräfte übertragen werden können, wird durch das zweite Stößelpaar eine geschlossene kinematische Schleife gebildet. Das zweite Stößelpaar wird gegensinnig zum ersten bewegt, so dass Antriebskräfte in beiden Richtungen übertragen werden können. Allgemein ist daher in einer Ausführung der vorliegenden Offenbarung in der mechanischen Schnittstelle eine ParallelogrammKinematik vorgesehen.

Das Ankoppeln des Instrumentenschaftes an die Antriebseinheit gestaltet sich einfach und kann alternativ entlang oder quer zur Bewegungs- bzw. Verstellrichtung der Stößel 10A - 20B erfolgen. Die Stößel 10A, 10B der Antriebseinheit 1 sind von der Sterilbarriere 3 bedeckt. Der Instrumentenschaft 2 wird so an die Antriebseinheit 1 gefügt, dass sich die Stößel 10A, 20A bzw. 10B, 20B, anfänglich in einem gewissen Abstand, gegenüberliegen. Anschließend wird die Abtriebsseite auf die Antriebsseite gedrückt. Die Winkelstellung der Kipphebel bzw. Wippe 10C, 20C ist hierbei beliebig, da sich die Stellungen beider Seiten während des Koppelvorgangs angleichen.

Fig. 2 zeigt eine mechanische Schnittstelle einer Instrumentenanordnung nach einer weiteren Ausführung der vorliegenden Erfindung. Mit der vorstehend erläuterten Ausführung übereinstimmende Merkmale sind durch identische Bezugszeichen bezeichnet, so dass nachfolgend nur auf die Unterschiede eingegangen und im Übrigen auf die gesamte Beschreibung Bezug genommen wird.

In der Ausführung der Fig. 1 herrscht Gleitkontakt zwischen den Koppelmitteln 10C, 20C und Stößeln 10A, 10B bzw. 20A, 20B, wobei die Reibkräfte unter anderem von der Hebelstellung und den Kontaktflächen, insbesondere deren Geometrie und Oberfläche, abhängen. Daher ist in einer Ausführung der vorliegenden Offenbarung, wie sie exemplarisch in Fig. 2 dargestellt ist, in wenigstens einem einseitigen Kontakt eines Koppelmittels (in Fig. 2 beispielhaft: 10C, 20C) und Ab- bzw. Eintriebselements (in Fig. 2 beispielhaft: 10A, 10B bzw. 20A, 20B) eine Rolle 30 angeordnet, wodurch die Reibung verringert werden kann.

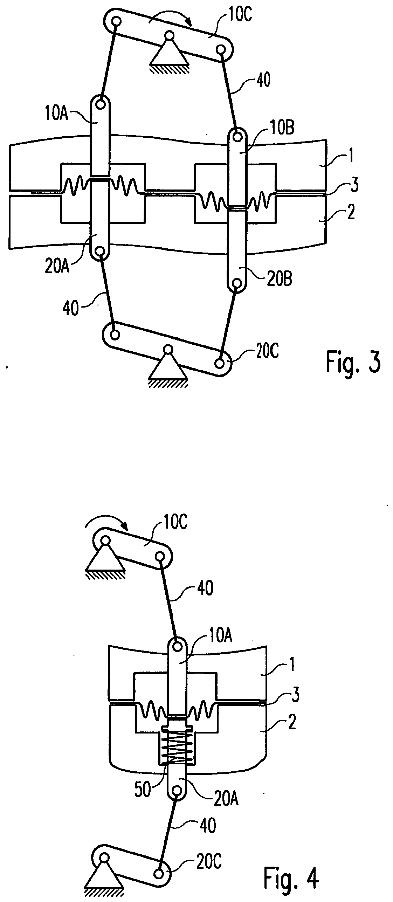

Fig. 3 zeigt eine mechanische Schnittstelle einer Instrumentenanordnung nach einer weiteren Ausführung der vorliegenden Erfindung. Mit den anderen Ausführungen übereinstimmende Merkmale sind durch identische Bezugszeichen bezeichnet, so dass nachfolgend nur auf die Unterschiede eingegangen und im Übrigen auf die gesamte Beschreibung Bezug genommen wird.

In den Ausführung der Fig. 1, 2 sind die Abtriebselemente 10A, 10B und das Koppelmittel 10C sowie die Eintriebselemente 20A, 20B und das weitere Koppelmittel 20C jeweils durch eine einseitige Bindung mit Gleit- (Fig. 1) bzw. Rollkontakt (Fig. 2) miteinander gekoppelt. In einer Ausführung, die exemplarisch in Fig. 3 gezeigt ist, ist hingegen wenigstens ein Abtriebselement (in Fig. 3 beispielhaft: 10A, 10B) und ein Koppelmittel (in Fig. 3 beispielhaft: 10C) und/oder wenigstens ein Eintriebselement (in Fig. 3 beispielhaft: 20A, 20B) und ein (weiteres) Koppelmittel (in Fig. 3 beispielhaft: 20C) durch wenigstens eine Koppelstange (in Fig. 3 beispielhaft: 40) miteinander gekoppelt, die gelenkig mit dem Koppelmittel bzw. Element verbunden ist.

Fig. 4 zeigt eine mechanische Schnittstelle einer Instrumentenanordnung nach einer weiteren Ausführung der vorliegenden Offenbarung. Mit den anderen Ausführungen übereinstimmende Merkmale sind durch identische Bezugszeichen bezeichnet, so dass nachfolgend nur auf die Unterschiede eingegangen und im Übrigen auf die gesamte Beschreibung Bezug genommen wird.

Bei dieser Ausführung ist zur Aktuierung eines Freiheitsgrades nur ein Stößelpaar 10A, 20A zur Kraftübertragung vorgesehen. Anstelle eines weiteren Paares aus Ab- und Eintriebselement ist das Eintriebselement 20A entgegen seiner Verstellrichtung durch eine Feder 50 vorgespannt. Diese stellt bei Wegnahme einer in Verstellrichtung aktuierenden Kraft bzw. bei aktuierter Bewegung des Abtriebselements entgegen dieser Verstellrichtung das Stößelpaar 10A, 20A entgegen der Verstellrichtung zurück.

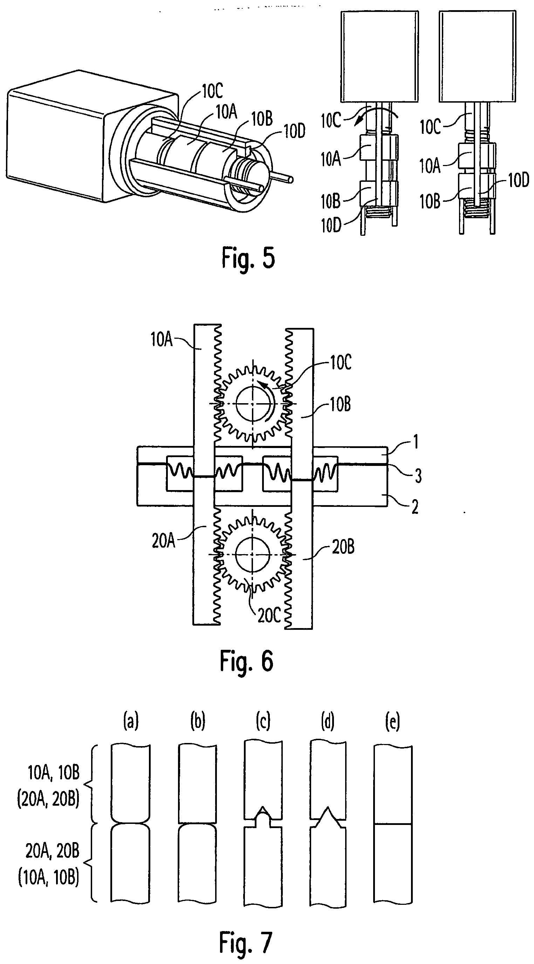

Fig. 5 zeigt eine mechanische Schnittstelle einer Instrumentenanordnung nach einer weiteren Ausführung der vorliegenden Offenbarung. Mit den anderen Ausführungen übereinstimmende Merkmale sind durch identische Bezugszeichen bezeichnet, so dass nachfolgend nur auf die Unterschiede eingegangen und im Übrigen auf die gesamte Beschreibung Bezug genommen wird.

In einer Ausführung, die exemplarisch in Fig. 5 gezeigt ist, ist wenigstens eine Kopplung zwischen einem Abtriebselement (in Fig. 5 beispielhaft: 10A, 10B) und einem Koppelmittel (in Fig. 5 beispielhaft: 10C) als Spindelantrieb mit gegenläufig bewegten Schiebehülsen ausgebildet. Das vorzugsweise als Gewindespindel ausgebildete Koppelmittel (in Fig. 5 beispielhaft: 10C) weist in einer Ausführung je einen Abschnitt mit Rechts- und Linksgewinde, auf denen jeweils ein als Spindelmutter ausgebildetes Abtriebselement (in Fig. 5 beispielhaft: 10A bzw. 10B) sitzt. Durch eine Drehung der Gewindespindel 10C werden die Spindelmuttern 10A, 10B gegenläufig bewegt. Die Muttern können, beispielsweise mit einer antriebseinheitsfesten Führungsschiene 10D, gegen Verdrehen gesichert sein.

Zur Verdeutlichung ist in Fig. 5 links ein perspektivischer Teilschnitt der Schnittstelle, in der Mitte bzw. rechts eine Seitansicht bei unterschiedlichen Stellungen der Abtriebselemente 10A, 10B gezeigt.

Fig. 6 zeigt eine mechanische Schnittstelle einer Instrumentenanordnung nach einer weiteren Ausführung der vorliegenden Offenbarung. Mit den anderen Ausführungen übereinstimmende Merkmale sind durch identische Bezugszeichen bezeichnet, so dass nachfolgend nur auf die Unterschiede eingegangen und im Übrigen auf die gesamte Beschreibung Bezug genommen wird.

In einer Ausführung, die exemplarisch in Fig. 6 gezeigt ist, sind wenigstens ein Abtriebselement (in Fig. 6 beispielhaft: 10A, 10B) und ein Koppelmittel (in Fig. 6 beispielhaft: 10C) und/oder wenigstens ein Eintriebselement (in Fig. 6 beispielhaft: 20A, 20B) und ein (weiteres) Koppelmittel (in Fig. 6 beispielhaft: 20C) durch ein Zahnstangetriebe gekoppelt. Hierzu sind in einer Weiterbildung Koppelmittel (in Fig. 6 beispielhaft: 10C, 20C) als Ritzel ausgebildet, mit denen die als Zahnstangen ausgebildeten Abtriebselemente (in Fig. 6 beispielhaft: 10A, 10B) bzw. Eintriebselemente (in Fig. 6 beispielhaft: 20A, 20B) jeweils gegensinnig kämmen und so eine rotatorische in eine translatorische Bewegung umsetzen. Da sie auf gegenüberliegenden Seiten des Ritzels angeordnet sind bewegen sie sich gegensinnig. Wenn in einer vorteilhaften Weiterbildung die Ein- und/oder Abtriebselemente entgegen ihrer Verstellrichtung bzw. aufeinander zu vorgespannt sind, kann dadurch vorteilhaft auch Umkehrspiel in den Zahneingriffen 10A-10C, 10B-10C, 20A-20C bzw. 20B-20C reduziert bzw. eliminiert werden.

Fig. 7 zeigt verschiedene Ausführungen der einander zugewandten Stirnseiten der Ab- und Eintriebselemente 10A, 10B bzw. 20A, 20B der Ausführungen der Fig. 1 bis 6, die eben oder ballig ausgebildet sind und/oder einen Vorsprung zum Eingreifen in eine Aussparung in der anderen Stirnseite aufweisen: Dabei zeigt Fig. 7 (a) zwei ebene Stirnseiten bzw. Kontaktflächen, die einen (einseitig gebundenen) Flächenkontakt bilden, Fig. 7 (b) eine ballige und eine ebene Stirnseite, die einen Punktkontakt bilden, Fig. 7 (c) einen kugelförmigen Vorsprung, der in eine kegelige Bohrung bzw. Aussparung eingreift und einen Ringkontakt bildet, Fig. 7 (d) einen kegelförmigen Vorsprung, der in eine kegelige Bohrung bzw. Aussparung eingreift und einen Flächenkontakt bildet, sowie Fig. 7 (e) zwei ballige Stirnseiten bzw. Kontaktflächen, die einen Punktkontakt bilden.

Um eine möglichst hohe Übertragungsgenauigkeit und Steifigkeit zu gewährleisten, sollten Abweichungen in der Position und Orientierung der Kontaktflächen vermieden werden. Mögliche Ursachen für derartige Abweichungen sind Fertigungs- und Montagetoleranzen, sowie Abweichungen in der Positionierung des Instruments zur Antriebseinheit durch den Anwender. Deshalb weist in einer Ausführung der vorliegenden Offenbarung wenigstens eine einseitige Bindung einen Punktkontakt zwischen Ab- und Eintriebselement auf.

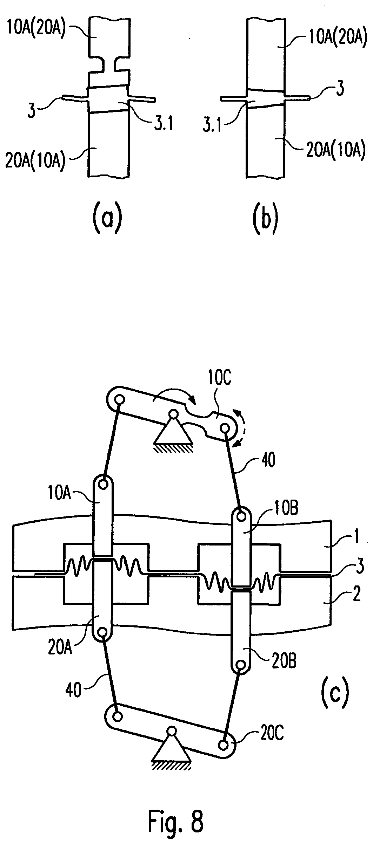

Fig. 8 bis 10 zeigen Ausgleichmittel zum Toleranzausgleich. Dabei zeigt Fig. 8 einen Ausgleich von Positions- und Orientierungsabweichungen der Stößel-Kontaktflächen durch gezielt eingebrachte Nachgiebigkeiten. In einer Ausführung, die exemplarisch in Fig. 8 (a) angedeutet ist, ist eine Nachgiebigkeit durch eine biegeweiche Gestaltung eines Ab- und/oder Eintriebselements (in Fig. 8 (a) beispielsweise 10A oder 20A) ausgebildet. Zusätzlich oder alternativ kann eine Nachgiebigkeit durch eine elastische Deformation der Sterilbarriere ausgebildet sein, wie dies exemplarisch in Fig. 8 (b) angedeutet ist. Die Sterilbarriere ist vorzugsweise ganz oder teilweise aus einem Elastomer hergestellt. Die biegeweiche Gestaltung eines Ab- und/oder Eintriebselements, wie in Fig. 8 (a) gezeigt, kann insbesondere in Hinblick auf das Übertragungsverhalten vorteilhaft sein. Allgemein kann eine Nachgiebigkeit in einer Ausführung der vorliegenden Offenbarung eine progressive Federkennlinie aufweisen, um so kleinere Toleranzen zu kompensieren und zugleich bei größeren Stellbewegungen eine relativ steife Übertragung zu gewährleisten.

Zusätzlich oder alternativ kann eine Nachgiebigkeit in einem Koppelmittel vorgesehen sein, wie dies exemplarisch in Fig. 8 (c) gezeigt ist. Aufgrund der geschlossenen kinematischen Kette handelt es sich prinzipiell um ein statisch überbestimmtes System. Um Fertigungs- und Montagetoleranzen in der kinematischen Kette auszugleichen und Spielfreiheit herzustellen, werden Längenunterschiede der Stößelpaare durch eine biegeweiche Gestaltung eines Koppelmittels kompensiert.

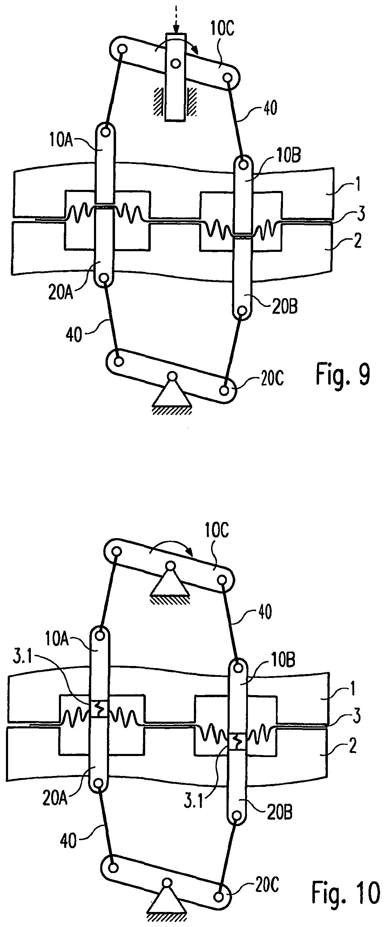

In einer Ausführung, die exemplarisch in Fig. 9 angedeutet ist, weist ein Ausgleichmittel zum Toleranzausgleich ein in Verstellrichtung (vertikal in Fig. 9) verschiebbares Lager bzw. eine in Verstellrichtung verschiebbare Lagerachse eines Koppelmittels (in Fig. 9 beispielhaft: 10C) auf. Dieses ist hierzu in einer Ausführung in einem Schlitten, der innerhalb der Antriebseinheit verschieblich angeordnet ist, drehbar gelagert. Dieses Schublager ermöglicht eine Verschiebung in Richtung der Stößelbewegung. In dieser Richtung wird, beispielsweise durch eine Feder oder durch statische Verstellung, eine Kraft aufgebracht, die die Stößelpaare in der Schnittstelle gegeneinander vorspannt (in Fig. 9 durch den strichlierten Kraftpfeil angedeutet).

Fig. 10 zeigt einen Ausgleich von Längentoleranzen zwischen den Stößelpaaren durch Nachgiebigkeiten in der Sterilbarriere, wie sie vorstehend bereits mit Bezug auf Fig. 8 (b) erläutert wurden. In einer Ausführung der vorliegenden Erfindung, die exemplarisch in Fig. 10 angedeutet ist, ist in die Sterilbarriere ein nachgiebiges Ausgleichselement 3.1 integriert. Durch Kompression dieses Elements wird eine Vorspannung in der kinematischen Schleife aufgebaut und gleichzeitig durch eine unterschiedliche Stauchung Längenunterschiede ausgeglichen. Insbesondere, damit nicht zu große Nachgiebigkeiten eingeführt werden, die sich ungünstig auf das Regelungsverhalten auswirken können, weist das Ausgleichselement 3.1 in einer Weiterbildung ein progressives Federverhalten auf. Dies kann insbesondere durch eine entsprechende Werkstoffwahl und/oder geometrische Gestaltung der Sterilbarriere erreicht werden.

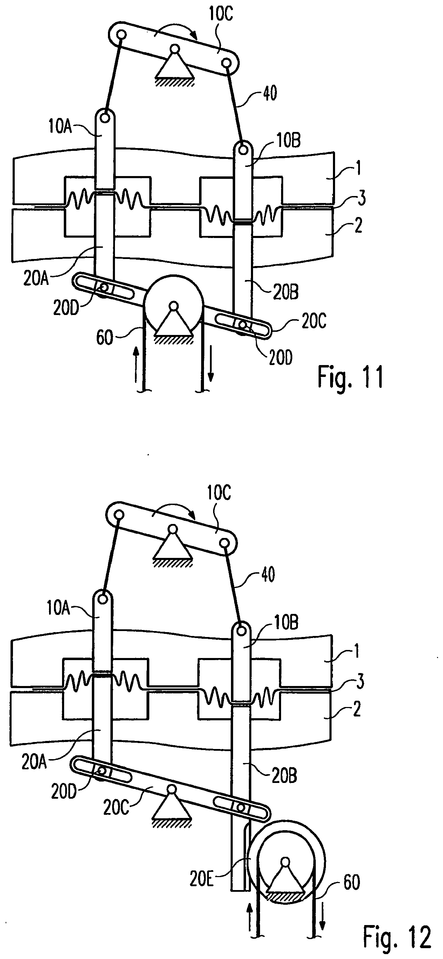

Fig. 11 bis 15 zeigen insbesondere verschiedene vorteilhafte Ankopplungen eines instrumentenschaftseitigen Antriebsstranges an eine erfindungsgemäße mechanische Schnittstelle, wie sie beispielsweise vorstehend mit Bezug auf Fig. 1 bis 10, aber auch nachfolgend mit Bezug auf die weiteren Figuren, beschrieben ist. Dabei zeigt Fig. 11 eine Ankopplung eines Seilzuges 60 an die Eintriebselemente. Um einen Freiheitsgrad des Instrumentenschafts, insbesondere eines Endeffektors (nicht dargestellt), beid- bzw. gegensinnig zu aktuieren, ist im Instrumentenschaft mit der drehbar gelagerten Wippe 20C eine kinematische Schleife zwischen den beiden Stößeln 20A, 20B gebildet. In der gezeigten Ausführung sind die Stößel jeweils mit einem Dreh-Schublager 20D an die Wippe gekoppelt. Mit der Wippe ist eine Seilrolle fest verbunden, die von dem Seilzug 60 umschlungen wird. In einer Weiterbildung ist auch eine form- und/oder stoffschlüssige Verbindung zwischen Rolle und Seilzug möglich. Durch eine geeignete Wahl des Seilrollendurchmessers kann optional eine Anpassung des Schnittstellen-Hubs an den erforderlichen Seilhub vorgenommen werden. Neben dem dargestellten zylindrischen Querschnitt der Seilrolle sind auch andere, insbesondere ellipsenartige, Querschnitte möglich.

Fig. 12 zeigt eine Ankopplung eines instrumentenseitigen Seilzuges 60 an die mechanische Schnittstelle nach einer weiteren Ausführung. Dabei ist die Seilrolle, die ein Element eines Koppelmittels im Sinne der vorliegenden Offenbarung bildet, zusätzlich mit einer Verzahnung 20E versehen, die mit einem verzahnten Abschnitt eines instrumentenseitigen Stößels (in Fig. 12 beispielhaft: 20B) kämmt. Die zusätzliche Übersetzung dieser Zahnradstufe erlaubt vorteilhaft eine noch bessere Anpassung des Stößelhubs an den Seilhub.

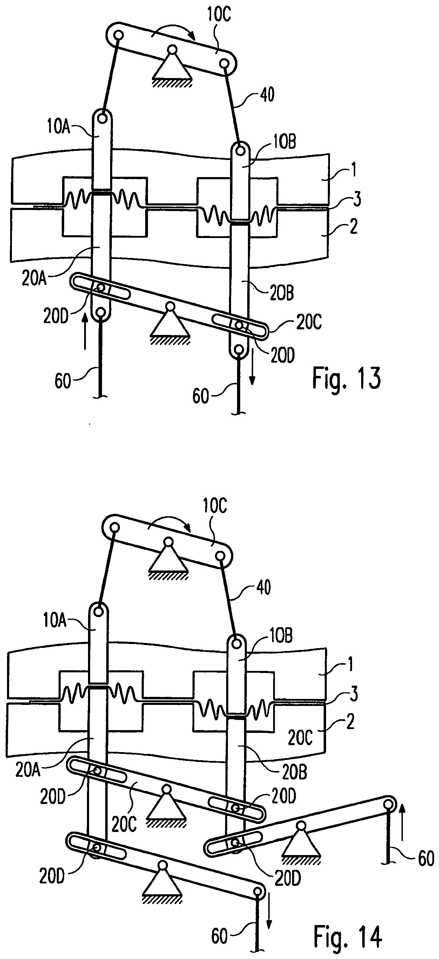

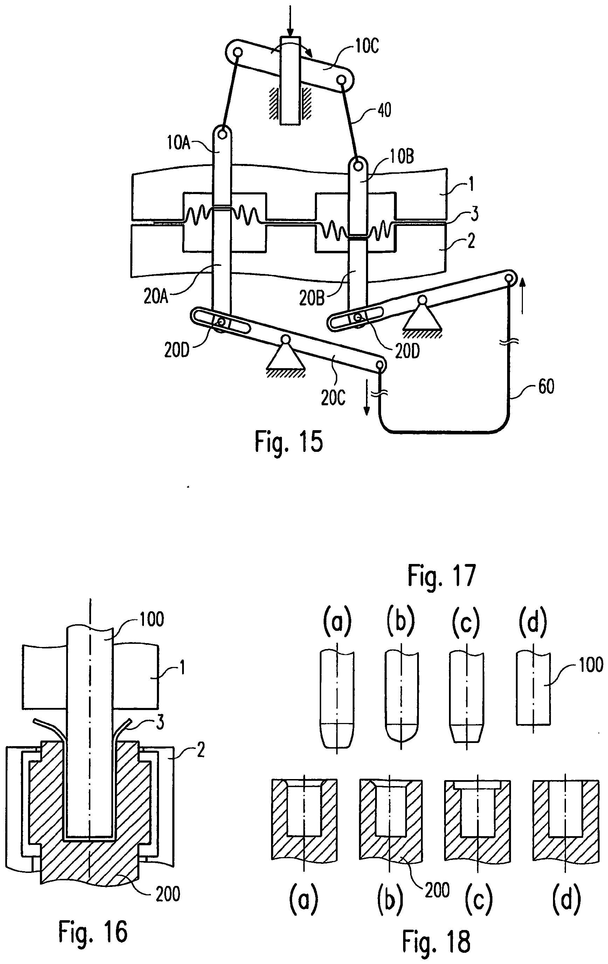

In den beiden Ausführungen der Fig. 11, 12 ist der Seilzug 60 geschlossen. In einer alternativen Ausführung der vorliegenden Offenbarung, die exemplarisch in Fig. 13, 14 und 15 angedeutet ist, kann ein Freiheitsgrad auch durch einen offenen Seilzug (in Fig. 13 beispielhaft: 60) oder durch Schubstangen (nicht dargestellt) aktuiert werden, dessen Enden mit Eintriebselementen (in Fig. 13 beispielhaft: 20A, 20B) oder einem damit gekoppelten Koppelmittel gekoppelt sein können. Dabei sind bei einer Ausführung der vorliegenden Offenbarung, wie sie exemplarisch in Fig. 14, 15 angedeutet ist, die Enden des Seilzugs 60 über zusätzliche Seilwippen an die mechanische Schnittstelle bzw. deren Eintriebselemente 20A, 20B gekoppelt. Über das Verhältnis der Hebelarme jeder Wippe kann vorteilhafterweise der Seilhub angepasst werden. Um eine Änderung der erforderlichen Seillänge zu vermeiden, können die Hebelverhältnisse der beiden Seilwippen gleich sein. In Fig. 14, 15 sind die beiden Lagerstellen der Seilwippen zur besseren Übersichtlichkeit versetzt zueinander dargestellt. In einer Ausführung können diese Lagerungen der Seilwippen koaxial zusammenfallen.

Bei der Ausführung der Fig. 14 wird die geschlossene kinematische Schleife zwischen Ab- und Eintriebselementen durch eine weitere instrumentenseitige Wippe gebildet, die jeweils über ein Dreh-Schublager 20D mit den instrumentenseitigen Stößeln 20A, 20B gekoppelt ist. Bei der Ausführung der Fig. 15 ist auf diese zusätzliche Wippe verzichtet und stattdessen die Vorspannung der Schnittstelle über den Seilzug aufbaut, durch den ohnehin eine geschlossene kinematische Schleife vorliegt. Insbesondere auf diese Weise kann allgemein nach einer Ausführung der vorliegenden Erfindung eine Vorspannung der mechanischen Schnittstelle auch zur Vorspannung eines instrumentenschaftseitigen Seilzugs verwendet werden, wodurch sich die Komplexität des instrumentenseitigen Antriebsstrangs reduziert. An dieser Stelle sei ausdrücklich darauf hingewiesen, dass in den gezeigten Ausführungen die Zuordnung von Ab- und Eintriebselementen rein beispielhaft ist und insbesondere auch Anordnungen bzw. Merkmale eines Abtriebselements einer Ausführung auch mit Anordnungen bzw. Merkmale eines Eintriebselements einer anderen Ausführung kombiniert sein können. So kann beispielsweise in der Ausführung der Fig. 14 anstelle der eintriebsseitigen Drehschublager 20D analog zu der abtriebsseitigen Anordnung auch eine Anordnung bzw. Kopplung mit Koppelstangen (vgl. abtriebsseitige Koppelstange 40 in Fig. 14) denkbar.

Fig. 16 zeigt eine mechanische Schnittstelle einer Instrumentenanordnung nach einer weiteren Ausführung der vorliegenden Offenbarung. Mit den anderen Ausführungen übereinstimmende Merkmale sind durch identische Bezugszeichen bezeichnet, so dass nachfolgend nur auf die Unterschiede eingegangen und im Übrigen auf die gesamte Beschreibung Bezug genommen wird. Bei dieser Ausführung weist die Schnittstelle ein Abtriebselement in Form eines Zapfens 100 und ein Eintriebselement mit einer Aussparung 200 auf, wobei der Zapfen in der Aussparung durch ein Klemmmittel radial elastisch aufweitbar ist. Diese Ausführung eignet sich zur Übertragung von Zug- und Druckkräften. Nachfolgend wird exemplarisch eine translatorische Aktuierung bzw. Verstellung der mechanischen Schnittstelle erläutert, die mechanische Schnittstelle kann jedoch auch zur Übertragung von rotatorischen oder überlagerten translatorischen und rotatorischen Bewegungen verwendet werden.

Der Antriebszapfen 100 ist translatorisch verstellbar in der Antriebseinheit 1 geführt und aktuiert und in eine instrumentenschaftseitigen Aussparung in Form einer Kupplungsbuchse 200 eingeführt. Die dünnwandige Sterilbarriere 3 ist zwischen Antriebseinheit und Instrumentenschaft angeordnet.

Die Verbindung von Antriebszapfen 100 und Kupplungsbuchse 200 kann kraft- oder formschlüssig und ab- oder unabhängig vom Instrumentenantrieb erfolgen. Vorteilhafterweise können Komponenten mit höherer Komplexität und engeren Toleranzen in der Antriebseinheit angeordnet werden, so dass diese Schnittstellen insbesondere auch für preiswerte Einweginstrumentenschäfte vorteilhaft sind. Positionierung und Befestigung des Instrumentenschafts relativ zur Antriebseinheit erfolgen in einer Weiterbildung durch eine separate Funktionseinheit, wie nachfolgend beschrieben. Die Lagerung der Kupplungselemente ist deshalb vorzugsweise so gewählt, dass hohe Anforderungen an die Form- und Lagetoleranzen vermieden werden und die Verbindung von Ab- und Eintriebselement, wenigstens im Wesentlichen, zwangsfrei erfolgt. Der Antriebszapfen ist daher in einer Weiterbildung in der Antriebseinheit mit einem fünfwertigen Schublager geführt, d.h. es sind nur Verschiebungen entlang der Längsachse möglich. Die Lagerung der Kupplungsbuchse im Instrumentenschaft hat in radialer Richtung Spiel, d.h. die Kupplungsbuchse ist in radialer Richtung nicht eindeutig geführt. Sofern der Instrumentenschaft nicht an der Antriebseinheit angekoppelt ist, gewährleistet die Radiallagerung, dass die Kupplungshülse mit hinreichender Genauigkeit vorpositioniert ist und während der Handhabung und Reinigung nicht gelöst werden kann. Sobald der Instrumentenschaft an die Antriebseinheit gekoppelt ist, hat diese Lagerung keine Funktion. Dann übernimmt das Schublager des Antriebszapfens auch die Lagerung des instrumentenschaftseitigen Eintriebselements. Auf diese Weise wird vorteilhaft eine zwangsfreie Verbindung erreicht, ohne dass sich die beiden Lagerungen verspannen. Die Lagerung der Kupplungsbuchse im Instrumentenschaft verfügt in einer Weiterbildung über zwei Anschläge in axialer bzw. Verstellrichtung. Somit kann der benötigte Arbeitshub für jeden Instrumentenschaft individuell festgelegt und die Antriebseinheit für verschiedene Instrumentenschäfte verwendet werden.

Durch die geometrische Gestaltung der Kupplungselemente erfolgt die radiale Ausrichtung der Kupplungsbuchse 200 zum Antriebszapfen 100 automatisch. Somit ist nur eine Fügebewegung in Richtung der Antriebsbolzen erforderlich. Dadurch wird vorteilhafterweise der Instrumentenschaftwechsel während eines chirurgischen Eingriffs erleichtert und ist in kürzerer Zeit durchführbar.

In Fig. 17, 18 sind verschiedene vorteilhafte Ausführungen von Antriebszapfen und Kupplungsbuchse dargestellt, insbesondere eine flache (Fig. 17 (d)), konische (Fig. 17(c)), sphärisch (Fig. 17 (b)) und eine elliptische (Fig. 17 (a)) Stirnseite des Antriebszapfens, die jeweils mit verschiedenen Einführgeometrie der instrumentenschaftseitigen Kupplungsbuchse kombiniert sein können, insbesondere einer zylindrischen (Fig. 18 (d)) Sacklochbohrung, insbesondere mit einem oder mehreren Absätzen (Fig. 18 (c)), einer Fase (Fig. 18 (b)) oder Verrundung (Fig. 18(a)).

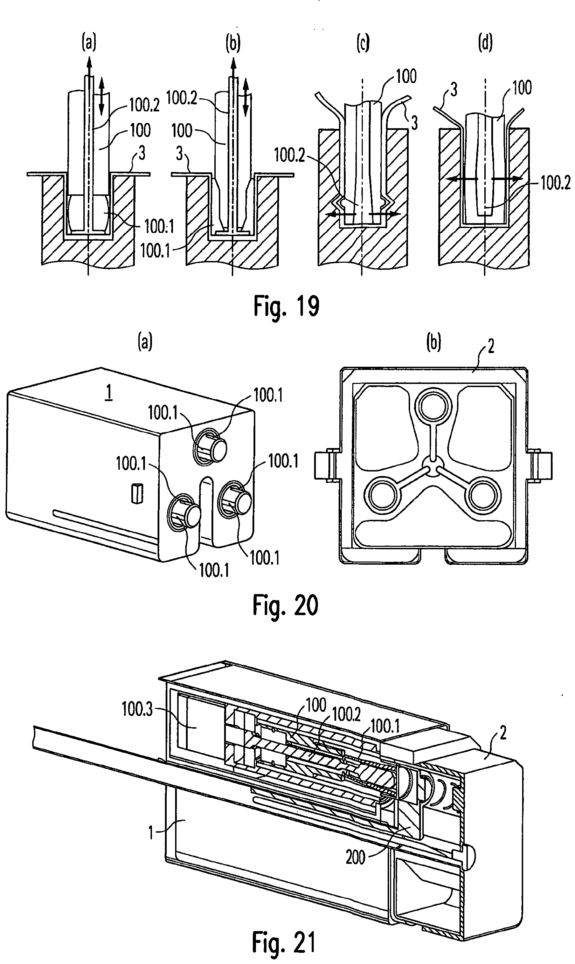

Fig. 19 zeigt verschiedene Koppelungen von Zapfen 100 und Aussparung 200: bei einer Ausführung, die exemplarisch in Fig. 19 (a), (b) und (d) angedeutet ist, sind Zapfen und Aussparung reibschlüssig durch elastisches Aufweiten eines, insbesondere einteilig (Fig. 19 (d)) oder mehrteilig (Fig. 19 (a), (b)) ausgebildeten, Antriebszapfens gekoppelt, der hierzu einen elastischen Körper (in Fig. 19 (a), (b) beispielhaft: 100.1) aufweisen kann, dessen Durchmesser durch elastische Verformung mittels eines Klemmmittels (in Fig. 19 (a), (b), (d): 100.2) vergrößert wird. Bei einer Ausführung, die exemplarisch in Fig. 19 (c) angedeutet ist, sind Zapfen und Aussparung zusätzlich oder ausschließlich formschlüssig durch elastisches Aufweiten eines ein- oder mehrteilig ausgebildeten Antriebszapfens gekoppelt. In einer Ausführung, die in Fig. 19 (c) exemplarisch in Kombination mit dem Formschluss dargestellt ist, weist ein Klemmmittel (in Fig. 19 (c) beispielhaft: 100.2) eine konische Außenform auf und ist im Zapfen 100 axial verstellbar, um diesen von innen radial aufzuweiten. In den Ausführungen der Fig. 19 (a), (b) weist das Klemmmittel 100.2 hingegen einen Flansch auf, um den elastischen Zapfen durch axiale Kompression radial aufzuweiten. In der Ausführung der Fig. 19 (d) ist das Klemmmittel 100.2 hydraulisch oder pneumatisch ausgebildet, der Zapfen 100 wird durch Druckbeaufschlagung von innen radial aufgeweitet.

Eine sterile Schutzhülle 3 ist zwischen Zapfen und Aussparung angeordnet und ermöglicht aufgrund ihrer Elastizität den vorstehend beschrieben Form- oder Reibschluss. Wie an anderer Stelle ausgeführt, wird auch bei dieser Ausführung eine Bewegung der (unsterilen) Antriebseinheit auf einen (sterilen) Instrumentenschaft nicht durch eine Aussparung in der Sterilbarriere hindurch, sondern über die geschlossene Sterilbarriere hinweg übertragen, was die sterile Handhabung erleichtert.

Die Klemmbewegung bzw. das Klemmmittel (in Fig. 19 beispielhaft: 100.2) kann von dem Instrumentenantrieb abhängig oder davon unabhängig betätigt werden.

Fig. 20 (a) zeigt exemplarisch eine Antriebseinheit 1 mit drei Abtriebselementen in Form von Zapfen, Fig. 20 (b) einen damit koppelbaren Instrumentenschaft 2 mit drei Eintriebselementen, die entsprechende Aussparungen aufweisen. In einer Ausführung kann ein Zapfen eines Ab- oder Eintriebselements nicht-elastisch radial aufweitbar sein und hierzu ein oder mehrere radial verschieblich geführte, vorzugsweise lamellenartige, separate Körper (in Fig. 20 (a) beispielsweise 100.1) aufweisen, wie dies exemplarisch in Fig. 20 (a) dargestellt ist.

Fig. 21 bis 23 zeigen Schnitte durch eine Antriebseinheit 1 und einen damit gekoppelten Instrumentenschaft 2 mit einer (Hub)Zapfen-Schnittstelle nach einer weiteren Ausführung der vorliegenden Erfindung. Mit den anderen Ausführungen übereinstimmende Merkmale sind durch identische Bezugszeichen bezeichnet, so dass nachfolgend nur auf die Unterschiede eingegangen und im Übrigen auf die gesamte Beschreibung Bezug genommen wird.

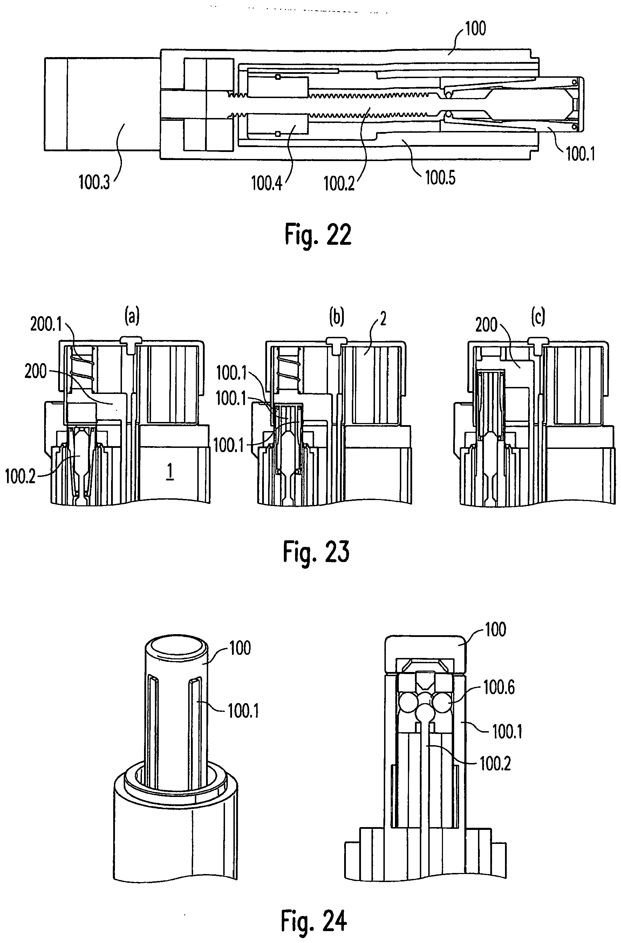

Schematisch angedeutet sind insbesondere ein Klemmmittelantrieb in Form eines Elektromotors 100.3 und eine Gewindespindel 100.2, beispielsweise ein Wälzschraubtrieb, eines Klemmmittels, ein Hubzapfen 100 und eine instrumentenschaftseitige Kupplungsbuchse mit einer Aussparung 200. Die Gewindespindel 100.2, beispielsweise eines Kugel- oder Rollengewindetriebs, wird von dem Elektromotor 100.3 weggesteuert angetrieben. Die Gewindespindel ist durch eine Spindellagerung in der Antriebseinheit 1 gelagert. Eine mit der Gewindespindel 100.2 kämmende Spindelmutter 100.4 ist drehfest mit dem Hubzapfen 100 verbunden. Der Hubzapfen ist seinerseits in einem Schublager 100.5 geführt, das nur eine Translation in Axialrichtung zulässt und sämtliche Radialkräfte und Momente aufnimmt. Zum Reib- oder Formschluss (vgl. insb. Fig. 19) weist der Zapfen 100 mehrere separate Körper in Form von lamellenartigen Spannhebeln 100.1 auf, die gleichmäßig am Umfang des Hubzapfens verteilt sind. Die Spannhebel 100.1 sind am distalen Ende des Hubzapfens 100 (rechts in Fig. 22) drehbar in diesem gelagert und hierdurch radial verschieblich geführt, so dass eine radiale Auslenkung der Spannhebel zu einer kraft- oder formschlüssigen Klemmung des Hubzapfens in der instrumentenseitigen Kupplungsbuchse führt. Die Auslenkung der Spannhebel erfolgt weggesteuert durch eine Steuerkontur, die in die Gewindespindel integriert sein kann, wie exemplarisch in Fig. 21 bis 23 angedeutet.

Fig. 23 zeigt Schritte des weggesteuerten Koppelvorgangs von Abtriebsanordnung und Antriebsanordnung mittels der mechanischen Schnittstelle miteinander, und zwar eine Situation vor dem Ankoppeln von Ab- und Eintriebselement 100, 200 (Fig. 23 (a)), ein Herstellen der Klemmung nach Einführen des Antriebszapfens in die Kupplungsbuchse (Fig. 23 (b)) und ein Aufrechterhalten der Klemmung über den gesamten Stellbereich durch mechanische Zwangsführung der Spannhebel.

Fig. 23 (a) zeigt die Situation vor dem Ankoppeln. Die Antriebseinheit 1 ist durch eine sterile Hülle 3 abgedeckt und der Instrumentenschaft an der Antriebseinheit 1 fixiert. Der Antriebszapfen 100 ist in eine untere Grenzlage gefahren. Eine Druckfeder 200.1 im Instrumentenschaft unterstützt den Kopplungsvorgang, indem sie sicherstellt, dass sich die Kupplungsbuchse 200 ebenfalls in einer unteren Grenzlage befindet. Fig. 23(b) zeigt die Situation unmittelbar nach dem Ankoppeln. Durch Ausfahren des Zapfens 100 aus der Antriebseinheit 1 wird dieser in die Kupplungsbuche des Instrumentenschafts eingeführt. Anschließend werden die Spannhebel 100.1 zwangsgeführt durch die auf die Gewindespindel 100.2 aufgebrachte Steuerkontur radial ausgefahren und dadurch die reib- oder formschlüssige Verbindung hergestellt. Wie in Fig. 23 (c) gezeigt, wird diese mechanische Verbindung durch eine mechanische Zwangsführung der Spannhebel 100.1 im gesamten Arbeitsbereich des Instrumentenschafts aufrechterhalten, im Ausführungsbeispiel bei translatorisch verstell- bzw. aktuiertem Zapfen 100 (vertikal in Fig. 23).

Fig. 24 zeigt mechanische Schnittstellen von Instrumentenanordnungen nach weiteren Ausführungen der vorliegenden Offenbarung. Mit den anderen Ausführungen übereinstimmende Merkmale sind durch identische Bezugszeichen bezeichnet, so dass nachfolgend nur auf die Unterschiede eingegangen und im Übrigen auf die gesamte Beschreibung Bezug genommen wird.

Bei der Ausführung der Fig. 24 ist der Hubzapfen- bzw. Klemmmittelantrieb kraftgesteuert, die Klemmkraft wird im Gegensatz zur Ausführung der Fig. 21 bis 24 nicht durch eine vom Stellantrieb des Abtriebselements bzw. Zapfens abhängige Zwangsführung aufgebracht. Die Kopplung zwischen Ab- und Eintriebselement wird durch elastisches Aufweiten des Antriebszapfens 100 hergestellt und kann kraft- oder formschlüssig erfolgen. Durch Verspannen eines Klemmmechanismus bzw. -mittels wird der Antriebszapfen radial aufgeweitet. In der Ausführung der Fig. 24 weist das Klemmmittel hierzu einen Rastkugelmechanismus auf, in einer nicht dargestellten Abwandlung kann es beispielsweise einen Spreizdorn, einen Kniehebel-Mechanismus oder eine Sternscheibe aufweisen. Um die Klemmkraft über den gesamten Stellbereich aufrecht zu erhalten, ist das Klemmmittel in einer Ausführung der vorliegenden Erfindung, wie sie exemplarisch in Fig. 24 angedeutet ist, allgemein so gestaltet, dass es über einen kinematischen Totpunkt verfügt. Hierunter wird vorliegend insbesondere verstanden, dass es einen kinematischen Bereich gibt, in dem das Klemmmittel stabil geöffnet bleibt bzw. Ab- und Eintriebselement nicht koppelt, und einen weiteren, von diesem durch einen Totpunkt getrennten kinematischen Bereich, in dem das Klemmmittel stabil geschlossen bleibt bzw. Ab- und Eintriebselement koppelt. In der Ausführung der Fig. 24 weist das Klemmmittel hierzu mehrere am Umfang des Antriebszapfens 100 verteilte Rastkugeln 100.6 und einen Betätigungsstift 100.2 mit einem Kugelkopf auf, dessen Durchmesser größer ist als der durch die nicht radial aufgeweiteten Rastkugeln definierte Innenring. Das Klemmmittel wird betätigt bzw. aktuiert, indem der Betätigungsstift 100.2 in den Antriebszapfen 100 vorgeschoben und somit die Rastkugeln 100.6 radial nach außen gedrückt werden. Dadurch wird ein separater elastischer Körper in Form einer Dehnhülse 100.1 im Durchmesser aufgeweitet, die ausgeklinkt oder geschlitzt sein kann, um die Betätigungskraft möglichst gering zu halten. Diese Hülse vermeidet vorteilhaft Punktkontakt zwischen den Rastkugeln und der Sterilbarriere, die den Zapfen 100 umhüllt (nicht dargestellt), und ermöglicht eine gleichmäßigere Anpresskraft über eine möglichst große Kontaktfläche. Dadurch kann die Kontaktsteifigkeit erhöht und die Flächenpressung der Sterilbarriere minimiert werden. Der Betätigungsstift 100.2 wird über den Totpunkt des Rastkugelmechanismus hinaus vorgeschoben, so dass die Rastkugeln hinter dem Kugelkopf des Betätigungsstiftes geringfügig radial nach innen zurückstellen, um die Klemmkraft stabil aufrecht zu erhalten.

Als Stellantrieb zur Aktuierung des Ab- bzw. Eintriebselements kann insbesondere ein Spindelantrieb dienen, wie er beispielsweise mit Bezug auf Fig. 22 erläutert wurde, wobei der Klemmmechanismus bzw. das Klemmmittel abhängig oder unabhängig vom Stellantrieb aktuiert werden kann. Im ersteren Fall wirkt die Vorschubbewegung der Antriebseinheit auf den Betätigungsstift 100.2, wie mit Bezug auf Fig. 23 erläutert.

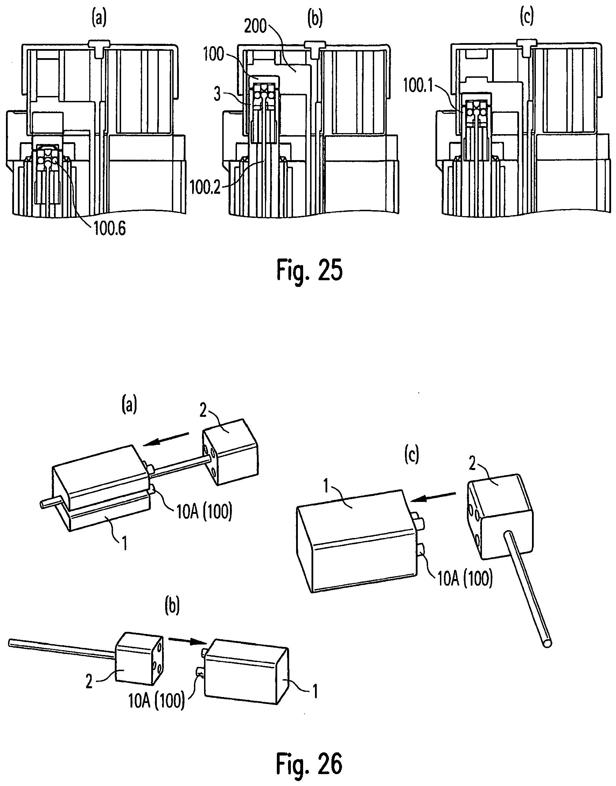

Fig. 25 zeigt Schritte des kraftgesteuerten Koppelvorgangs von Abtriebsanordnung und Antriebsanordnung mittels der mechanischen Schnittstelle der Fig. 24 miteinander, in Fig. 23 entsprechender Darstellung, auf die ergänzend Bezug genommen wird. Fig. 25 (a) zeigt die Situation vor dem Einkoppeln. Die Antriebseinheit 1 ist durch eine sterile Hülle abgedeckt und der Instrumentenschaft an der Antriebseinheit fixiert. Der Antriebszapfen 100 ist in eine untere Grenzlage gefahren. Fig. 25 (b) zeigt die Situation unmittelbar nach dem Ankoppeln: Um den Antriebszapfen zuverlässig in die Kupplungsbuche des Instrumentenschafts einzuführen, wird das Abtriebselement 100 gegen einen Endanschlag im Instrumentenschaft gefahren und der Kupplungsmechanismus ausgelöst bzw. das Klemmmittel betätigt. Die Rastkugeln 100.6 werden von dem Betätigungsstift 100.2 radial nach außen gedrückt und so die mechanische Verbindung von Ab- und Eintriebselement hergestellt. Wie in Fig. 25 (c) gezeigt, wird die mechanische Verbindung im gesamten Arbeitsbereich des Instruments aufrechterhalten, da der Totpunkt des Klemmmechanismus überwunden ist.

Bei einer Instrumentenanordnung nach der vorliegenden Offenbarung kann der Instrumentenschaft insbesondere einen Flansch aufweisen, wobei die mechanische Schnittstelle auf einer endeffektorzugewandten, endeffektorabgewandten oder seitlichen Fläche dieses Flansches angeordnet ist. Mit anderen Worten kann die Antriebseinheit 1 als "Hinterlader", "Vorderlader" oder "Seitenlader" ausgebildet sein.

Zur Erläuterung sind in Fig. 26 vorteilhafte Fügerichtungen eines Instrumentenschafts an eine Antriebseinheit einer Instrumentenanordnung nach verschiedenen Ausführungen der vorliegenden Offenbarung schematisch dargestellt. Nach einer Ausführung, die exemplarisch in Fig. 26 (a) angedeutet ist, wird der Instrumentenschaft entlang der Einführrichtung des Instruments in den Patienten an die Antriebseinheit gefügt, was daher als "Hinterlader" bezeichnet wird. In einer anderen Ausführung, die exemplarisch in Fig. 26 (b) angedeutet ist, wird der Instrumentenschaft entgegen der Einführrichtung des Instruments in den Patienten an die Antriebseinheit gefügt, was entsprechend als "Vorderlader" bezeichnet wird. In einer anderen Ausführung, die exemplarisch in Fig. 26 (c) angedeutet ist, wird der Instrumentenschaft quer zur Einführrichtung des Instruments in den Patienten an die Antriebseinheit gefügt, was als "Seitenlader" bezeichnet wird. Bei der in Fig. 26 gezeigten Instrumentenanordnung kann es sich insbesondere um eine der mit Bezug auf eine der anderen Figuren erläuterte Ausführung handeln, so dass auf deren Beschreibung Bezug genommen wird.

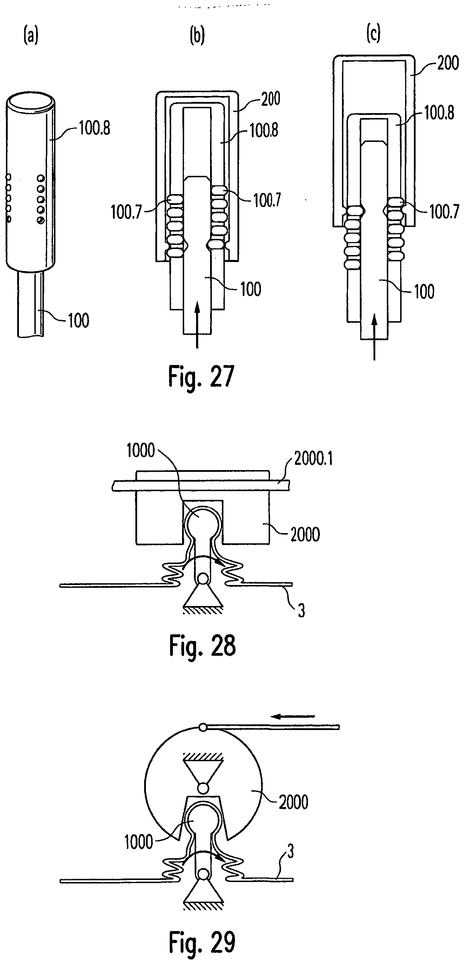

Fig. 27 zeigt eine mechanische Schnittstelle einer Instrumentenanordnung nach einer weiteren Ausführung der vorliegenden Offenbarung, und zwar in einer perspektivischen Ansicht (Fig. 27 (a)), und zwei Schnitten in unterschiedlichen Hubstellungen (Fig. 27(b), (c)). Mit den anderen Ausführungen übereinstimmende Merkmale sind durch identische Bezugszeichen bezeichnet, so dass nachfolgend nur auf die Unterschiede eingegangen und im Übrigen auf die gesamte Beschreibung Bezug genommen wird.

Bei dieser Ausführung ist zwischen dem Zapfen und der Aussparung ein in radialer Richtung wellenförmiger Spalt ausgebildet, in dem eine radial verschiebbare, axial feste Zwischenelementanordnung angeordnet ist, um eine translatorische Bewegung über eine Sterilbarriere hinweg zu übertragen.

Hierzu ist der Zapfen 100 mit umlaufender Einkerbung und eine instrumentenschaftseitige Kupplungsbuche 200 mit einem umlaufenden Ringprofil auf der Innenseite ausgebildet. Zapfen und Kupplungsbuchse sind so gestaltet, dass sich im gefügten Zustand zwischen diesen Bauteilen ein, vorzugsweise äquidistanter, wellenförmiger Spalt ausbildet. In diesen Spalt sind stabförmige Zwischenelemente 100.7 einer Zwischenelementanordnung eingebracht, die in einer Käfighülse 100.8 raumfest gelagert und nur in radialer Richtung verschieblich sind. Die dünne, folienartige Sterilbarriere (nicht dargestellt) ist zwischen Kupplungsbuche und Käfighülse angeordnet. Durch Verschieben des Zapfens 100 in axialer Richtung (vertikal in Fig. 27) wird der antriebsseitige Teil des wellenförmigen Spalts zwischen Zapfen und Kupplungsbuchse verschoben. Durch die kinematischen Zwänge in der Schnittstelle wird die Kupplungsbuchse zusammen mit dem Zapfen axial bzw. translatorisch verschoben, wie in der Figurenfolge Fig. 27 (b) → (c) angedeutet. In einer Weiterbildung können die Zwischenelemente der Zwischenelementanordnung hülsenförmig ausgebildet sein, an deren Stirnseiten Kugeln drehbar angeordnet sind, um den Reibungswiderstand zu reduzieren.

Fig. 28, 29 zeigen mechanische Schnittstellen von Instrumentenanordnungen nach weiteren Ausführungen der vorliegenden Offenbarung. Mit den anderen Ausführungen übereinstimmende Merkmale sind durch identische Bezugszeichen bezeichnet, so dass nachfolgend nur auf die Unterschiede eingegangen und im Übrigen auf die gesamte Beschreibung Bezug genommen wird.

Bei diesen Ausführungen weist die mechanische Schnittstelle einen Kipphebel auf, um insbesondere eine translatorische Antriebsbewegung über eine Sterilbarriere hinweg zu übertragen. Ein besonderer Vorzug dieses Konzepts ist eine einfache Gestaltung der Sterilbarriere: Sie muss lediglich für die Kippbewegungen des Hebels ausgelegt sein und kann in einer Weiterbildung daher als Kunststoff-Formteil, beispielsweise aus thermoplastischem Elastomer oder Silikon, insbesondere als tiefgezogene Folie, einfach hergestellt werden. Der Neigungswinkel des Hebels kann in einer Ausführung von einem Drehantrieb verstellt werden, insbesondere einem Elektromotor, gegebenenfalls mit zwischengeschaltetem Getriebe. Die Sterilbarriere kann die gesamte Antriebseinheit einhüllen und auch über den Hebel gestülpt sein. In einer nicht dargestellten Weiterbildung kann ein Hebel (in Fig. 28, 29 beispielhaft: 1000) allgemein auf seiner kontakt- bzw. sterilbarrierenabgewandten Seite (unten in Fig. 28, 29) über sein Drehlager hinaus verlängert und dort mit einem Antrieb bzw. einem instrumentenschaftseitigen Antriebsstrang, beispielsweise einem Zugseil oder einem Gestänge, gekoppelt sein.

Der Kipphebel (in Fig. 28, 29 beispielhaft: 1000) ist in einer Ausführung allgemein formschlüssig mit einem Kupplungsteil gekoppelt, insbesondere kann er, wie in den Ausführungen der Fig. 28, 29 angedeutet, in einer Nut eines Kupplungsteils (in Fig. 28, 29 beispielhaft: 2000) geführt sein. Der Kipphebel kann insbesondere mit einem Abtriebselement der Abtriebsanordnung der Antriebseinheit gekoppelt sein oder ein solches darstellen, das Kupplungsteil entsprechend mit einem Antriebselement der Antriebsanordnung des Instrumentenschafts gekoppelt sein oder ein solches darstellen. Gleichermaßen kann umgekehrt auch der Kipphebel mit einem Antriebselement der Antriebsanordnung des Instrumentenschafts gekoppelt sein oder ein solches darstellen, das Kupplungsteil entsprechend mit einem Abtriebselement der Abtriebsanordnung der Antriebseinheit gekoppelt sein oder ein solches darstellen.

Das Kupplungsteil 2000 kann in einer Ausführung, die exemplarisch in Fig. 28 angedeutet ist, durch ein Schublager 2000.1 translatorisch verstellbar geführt sein.

Somit wird die Drehbewegung des Kipphebels 1000 beispielsweise im Instrumentenschaft als translatorische Bewegung abgegriffen bzw. in der Antriebseinheit als translatorische Bewegung aufgeprägt. Die Kinematik dieser Schnittstelle ist nichtlinear und wird daher in einer Weiterbildung rechnerisch bzw. in der Antriebseinheitssteuerung kompensiert.

Indem ein Kipphebel in einer Weiterbildung kardanisch gelagert ist, können auch Bewegungen in zwei Freiheitsgraden übertragen werden. Hierzu ist beispielsweise die Abbildung der Fig. 28 als Schnittdarstellung in zwei zueinander senkrechten Ebenen zu denken. Durch eine optionale zusätzliche Verschiebbarkeit des Kipphebels entlang seiner Längsachse (vertikal in Fig. 28) kann eine Schnittstelle mit Kipphebel zur Aktuierung von drei Freiheitsgraden ausgebildet sein.

In einer anderen Ausführung, die exemplarisch in Fig. 29 angedeutet ist, kann das mit dem Kipphebel formschlüssig gekoppelte Kupplungsteil ebenfalls drehbar gelagert sein bzw. in einem Drehlager geführt sein. Auch diese Ausführung kann zur Aktuierung von zwei oder mehr Freiheitsgraden erweitert sein, wie dies vorstehend mit Bezug auf Fig. 28 erläutert wurde.

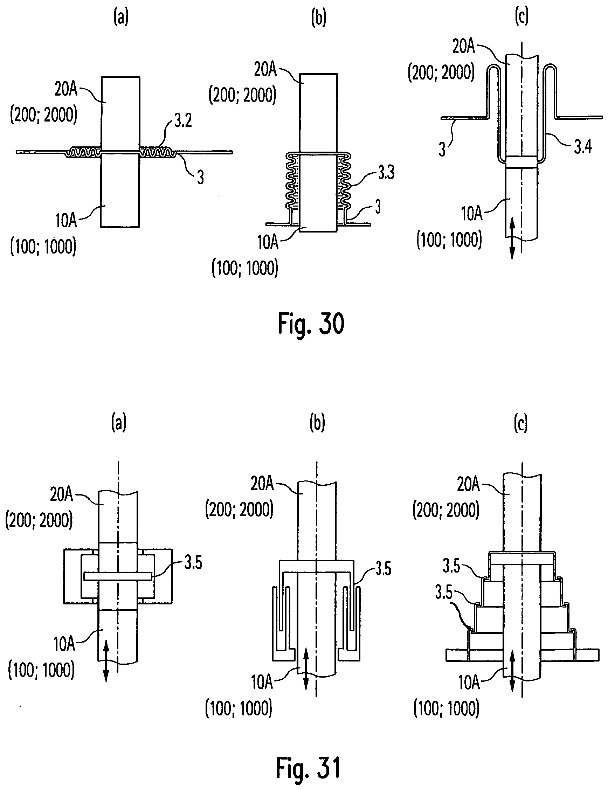

Die Figuren Fig. 30 bis 32 zeigen Instrumentenanordnungen nach weiteren Ausführungen der vorliegenden Offenbarung mit einer Sterilbarriere, die - wenigstens im OP-Betrieb - eine Antriebseinheit umhüllt und zwischen der Antriebseinheit und einem mit diesem mittels einer mechanischen Schnittstelle gekoppelten Instrumentenschaft angeordnet ist. Antriebseinheit, Instrumentenschaft und/oder mechanische Schnittstelle können insbesondere solcher der anderen Ausführungen und Figuren sein, so dass mit den anderen Ausführungen übereinstimmende Merkmale durch identische Bezugszeichen bezeichnet sind und nachfolgend nur auf die Unterschiede eingegangen und im Übrigen auf die gesamte Beschreibung Bezug genommen wird.

Die Sterilbarriere kann allgemein insbesondere einteilig und/oder als Folienschlauch ausgeführt sein. In einer Weiterbildung ist die Sterilbarriere luftdicht ausgebildet bzw. umhüllt die Antriebseinheit luftdicht. Wie insbesondere nachfolgend mit Bezug auf die Fig. 30 bis 32 beschrieben, erfolgt nach einer Ausführung der vorliegenden Offenbarung eine Übertragung einer Antriebs- bzw. Stellbewegung von einem Ab- auf ein Eintriebselement nicht durch eine Öffnung in der Sterilbarriere hindurch, sondern über die in diesem Bereich geschlossene Sterilbarriere hinweg.

In einer Ausführung, die in zwei Varianten in Fig. 30 dargestellt ist, weist die Sterilbarriere im Bereich der mechanischen Schnittstelle wenigstens eine, insbesondere im Bereich jedes Abtriebselements je eine, vorgespannte Lose in einer Verstellrichtung der Ab- und Antriebsanordnung auf. Die vorgespannte Lose ist in einer Weiterbildung als elastischer Faltenbalg, insbesondere als Elastomer-Faltenbalg ausgebildet, vorzugsweise als Wellmembran (in Fig. 30 (a) beispielhaft: 3.2) oder Wellbalg (in Fig. 30 (b) beispielhaft: 3.3), der direkt in die sterile Hülle integriert bzw. einteilig mit dieser ausgebildet, insbesondere ur- oder umgeformt, ist. In einer anderen Ausführung, die als Variante in Fig. 30 (c) dargestellt ist, weist die Sterilbarriere im Bereich der mechanischen Schnittstelle wenigstens eine, insbesondere im Bereich jedes Abtriebselements je eine, nicht vorgespannte Lose in einer Verstellrichtung der Ab- und Antriebsanordnung auf. Diese, wenigstens im Wesentlichen nicht vorgespannte, Lose ist in einer Weiterbildung als, vorzugsweise elastische, Stulpe, insbesondere als Thermoplast- oder Elastomer-Stulpe (in Fig. 30 (c) beispielhaft: 3.4) ausgebildet, die direkt in die sterile Hülle integriert bzw. einteilig mit dieser ausgebildet, insbesondere ur- oder umgeformt, ist.

Fig. 30 (a) zeigt eine Ausführung als ebene Wellmembran 3.2, Fig. 30 (b) als Wellbalg 3.3, dessen Querschnitt insbesondere zylindrisch oder kegelförmig sein kann. Beide Faltenbalge speichern Lose in Verstellrichtung (vertikal in Fig. 30), in die durch die Faltung bzw. vorgeformten Wellen eine rückstellende Vorspannung eingeprägt ist, die bei Aktuierung des Abtriebselements (in Fig. 30 beispielsweise 10A, 100 oder 1000) in Verstellrichtung den auftretenden Hub kompensiert.

In einer anderen Ausführung, die in drei Varianten in Fig. 31 dargestellt ist, weist die Sterilbarriere im Bereich der mechanischen Schnittstelle wenigstens eine, insbesondere im Bereich jedes Abtriebselements je eine, berührungslose translatorisch verschiebbare Dichtung auf (in Fig. 31 beispielhaft: 3.5). Diese kann in einer Weiterbildung, die exemplarisch in Fig. 31 (a) angedeutet ist, als axial verschiebliche Spaltdichtung ausgebildet sein. Gleichermaßen kann sie in einer Weiterbildung, die exemplarisch in Fig. 31 (b) angedeutet ist, als Labyrinthdichtung ausgebildet sein. Wie exemplarisch in Fig. 31 (c) angedeutet, kann eine translatorisch verschiebbare Dichtung vorzugsweise teleskopierbar sein, insbesondere als ein- oder mehrstufige Teleskophülse (in Fig. 31 (c) beispielhaft: dreistufig).

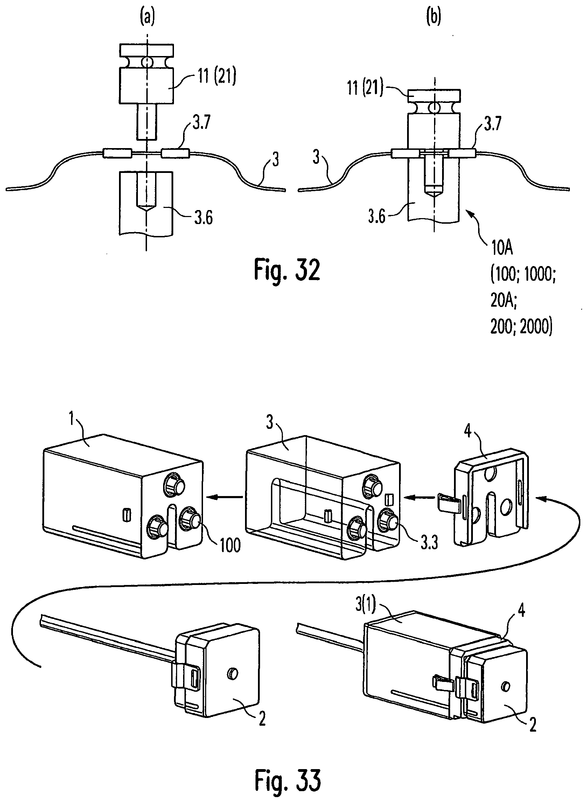

Fig. 32 zeigt eine weitere Ausführung der Sterilbarriere im Bereich der mechanischen Schnittstelle, insbesondere wenigstens eines, vorzugsweise jedes Ab- bzw. Eintriebselements, die sich durch eine sehr einfache Struktur und Fertigung auszeichnet. Die Sterilbarriere weist für wenigstens ein, vorzugsweise jedes Ab- oder Eintriebselement einen sterilen Elementfortsatz auf, der lösbar mit einer Elementbasis verbindbar ist, welche die Sterilbarriere zerstörend durchgreift. Wie in der Figurenfolge Fig. 32 (a) → 32 (b) angedeutet, durchgreift exemplarisch eine Abtriebselementbasis 11 zerstörend die Sterilbarriere 3 und wird mit ihrem Durchtrittsbereich lösbar mit einem sterilen Elementfortsatz 3.6 zu einem Abtriebselement verbunden, wie es beispielsweise als 10A, 10B, 100 oder 1000 in den anderen Ausführungen und Figuren erläutert ist. Gleichermaßen kann umgekehrt auch eine Eintriebselementbasis 21 zerstörend die Sterilbarriere 3 durchgreifen und mit ihrem Durchtrittsbereich lösbar mit einem sterilen Elementfortsatz 3.6 zu einem Eintriebselement verbunden werden, wie es beispielsweise als 20A, 20B, 200 oder 2000 in den anderen Ausführungen und Figuren erläutert ist.

In einer Ausführung, die exemplarisch in Fig. 32 angedeutet ist, weist die Sterilbarriere im Bereich der sie durchgreifenden Elementbasen je eine, vorzugsweise ringförmige, Aufdickung 3.7 auf, beispielsweise durch aufgeklebte Kunststoffscheiben, urgeformte lokale Wandstärkenvergrößerungen und/oder lokaler Werkstoffänderung. In der Mitte des verstärkten Bereichs kann die Sterilbarriere wieder als dünne Membran ausgeführt sein. Nach dem Einhüllen der Antriebseinheit wird, wie beschrieben, beispielsweise auf einen Zapfen der sterile Fortsatz 3.6 gefügt. Hierzu wird die dünne Membran der Sterilbarriere im Inneren des Verstärkungsrings durchstoßen. Die Fixierung des sterilen Fortsatzes kann insbesondere reib-, stoff- und/oder formschlüssig, schlüssig beispielsweise durch eine Schraub- oder Bajonettverbindung erfolgen oder auch als Kugelsperrbolzen ausgeführt sein.

Fig. 33 zeigt eine Instrumentenanordnung nach einer weiteren Ausführung der vorliegenden Offenbarung mit einer Sterilbarriere 3, die - wenigstens im OP-Betrieb - eine Antriebseinheit 1 umhüllt und zwischen der Antriebseinheit und einem mit diesem mittels einer mechanischen Schnittstelle gekoppelten Instrumentenschaft 2 angeordnet ist. Antriebseinheit 1, Instrumentenschaft 2 und/oder mechanische Schnittstelle 3 können insbesondere solcher der anderen Ausführungen und Figuren sein, so dass mit den anderen Ausführungen übereinstimmende Merkmale durch identische Bezugzeichen bezeichnet sind und nachfolgend nur auf die Unterschiede eingegangen und im Übrigen auf die gesamte Beschreibung Bezug genommen wird.

Die Instrumentenanordnung weist ein Befestigungselement in Form eines sterilen Adapters 4 zur lösbaren Verbindung des Instrumentenschafts 2 an der Antriebseinheit 1 auf, welcher auf einer der Antriebseinheit abgewandten Oberfläche der Sterilbarriere angeordnet wird bzw. ist.

Die Antriebseinheit 1, die in der in Fig. 33 dargestellten Ausführung beispielhaft mehrere Hubzapfen 100 aufweist, wird von der sterilen Hülle 3 umschlossen. In der sterilen Hülle sind die Abdeckungen für die Abtriebselemente integriert, in der in Fig. 33 dargestellten Ausführung beispielhaft als Elastomer-Faltenbälge wie vorstehend mit Bezug auf Fig. 30 erläutert. Nachdem die Antriebseinheit von der Sterilbarriere umschlossen wurde, wird der sterile Adapter 4 von außen an der steril verpackten Antriebseinheit fixiert. Der Adapter 4 interagiert somit nicht mit den Abtriebselementen 100, sondern stellt lediglich eine mechanische Schnittstelle zur Befestigung des Instrumentenschafts 2 an der umhüllten Antriebseinheit 1 zur Verfügung. Diese Trennung von mechanischer Kopplung von Ab- und Eintriebselementen einerseits (durch die mechanische Schnittstelle) und der mechanischen Befestigung von Antriebseinheit und Instrumentenschaft andererseits (durch das Befestigungselement bzw. den Adapter) erleichtert die sterile Handhabung der Instrumentenanordnung. In einer Ausführung, die exemplarisch in Fig. 33 angedeutet ist, ist der Adapter 4 form- und/oder reibschlüssig, beispielsweise durch Rast- bzw. Clipverbindungen, mit Instrumentenschaft und Antriebseinheit verbindbar bzw. verbunden, wobei die sterile Hülle 3 auch zwischen Rastvorsprüngen und -aussparungen von Antriebseinheit und Adapter dicht bzw. durchgangsfrei ist, um so die Sterilität zu wahren.

Die vorstehenden Instrumentenanordnungen sind in einer Weiterbildung robotergeführt bzw. zur Befestigung an einem Manipulator eines Manipulatorchirurgiesystems eingerichtet. Insbesondere kann hierzu die Antriebseinheit 1, der Instrumentenschaft 2 und/oder ein Befestigungselement bzw. ein Adapter 4 eine entsprechend eingerichtete Befestigungsschnittstelle aufweisen, beispielsweise entsprechende Aussparungen, Verriegelungen oder dergleichen.

Vorstehend wurden insbesondere Komponenten einer Instrumentenanordnung beschrieben, wobei jedoch auch Verfahren zum Bestücken eines Manipulators eines Manipulatorchirurgiesystems umfasst sind, bei denen eine modulare motorische Antriebseinheit und ein Instrumentenschaft lösbar miteinander verbunden und dabei die Abtriebsanordnung und die Antriebsanordnung mittels der mechanischen Schnittstelle miteinander gekoppelt werden, wie beispielsweise in den Figurenfolgen Fig. 23 (a) → Fig. 23 (b) → Fig. 23 (c), Fig. 25 (a) → Fig. 25 (b) → Fig. 25 (c) und Fig. 32 (a) → Fig. 32 (b) sowie den Montagepfeilen in Fig. 26 und Fig. 33.

Fig. 34 zeigt einen Teil eines robotergeführten minimalinvasiven chirurgischen Instruments nach einer Ausführung der vorliegenden Offenbarung mit einem Antriebsmodul 10 und einem damit in nicht näher gezeigter Weise lösbar verbundenen Instrumentenschaft 20 mit einem Endeffektor in Form einer beweglichen Klemme mit zwei Klingen 2.1, 2.2. Nachfolgend wird eine Ausführung der Erfindung insbesondere anhand der Klinge 2.1 näher erläutert, Aufbau und Funktion der Klinge 2.2 sind analog, so dass hierauf Bezug genommen wird.

Die Klinge 2.1 weist gegenüber dem Instrumentenschaft 20 einen Drehfreiheitsgrad $q_1$ auf. Um diesen Freiheitsgrad zu aktuieren bzw. die Klinge 2.1 der Klemme zu öffnen bzw. zu schließen, sind zwei Antriebsstränge 21, 22 einer instrumentenschaftseitigen Antriebsstranganordnung gegensinnig an der Klinge 2.1 angelenkt. Bei den Antriebssträngen 21, 22 kann es sich beispielsweise um Druckstangen bzw. Stößel handeln, die in dem Instrumentenschaft translatorisch beweglich gelagert sind.

Um die Druckstangen 21, 22 gegensinnig zu aktuieren, weist das Antriebsmodul zwei gegensinnige Antriebsstränge 11, 12 einer antriebsmodulseitigen Antriebsstranganordnung auf, die über eine Wippe durch einen Elektromotor 13 eines Antriebs des Antriebsmoduls gegensinnig aktuierbar sind. Bei den Antriebssträngen 11, 12 kann es sich ebenfalls um Druckstangen bzw. Stößel handeln, die in dem Antriebsmodul translatorisch beweglich gelagert sind.

In einer Schnittstelle zwischen Antriebsmodul und Instrumentenschaft ist optional eine flexible Sterilbarriere 4 angeordnet, über die die instrumentenschaftseitige und die antriebsmodulseitige Antriebsstranganordnung miteinander lösbar gekoppelt sind.

Die Antriebsstranganordnungen sind einseitig translatorisch gekoppelt: die Druckstangen bzw. Stößel 11 und 21 bzw. 12 und 22 sind translatorisch verschiebbar und können aufeinander über die Sterilbarriere hinweg nur Druckkräfte übertragen.

Um den Kraftschluss zwischen den Druckstangen bzw. Stößeln 11 und 21 sowie 12 und 22 sicherzustellen, die nur Druckkräfte über die Sterilbarriere 4 übertragen können, ist die antriebsmodulseitige Antriebsstranganordnung gegen die Schnittstelle vorgespannt, wie in Fig. 34 durch eine durch eine Feder 5 vorgespannte Lagerung des Elektromotors 13 mit angekoppelter Antriebsstranganordnung angedeutet.

An dem ersten antriebsmodulseitigen Antriebsstrang 11 sind zum Erfassen einer Last $F_1$ in diesem Antriebsstrang ein erstes Messmittel in Form eines Dehnmessstreifens 31 und diesem gegenüberliegend ein drittes Messmittel in Form eines Dehnmessstreifens 33 einer Messanordnung angeordnet.

An dem zweiten antriebsmodulseitigen Antriebsstrang 12 zum Aktuieren desselben Freiheitsgrades $q_1$ der Klinge 2.1 des Endeffektors sind zum Erfassen einer Last $F_2$ in diesem Antriebsstrang ein zweites Messmittel in Form eines Dehnmessstreifens 32 und diesem gegenüberliegend ein viertes Messmittel in Form eines Dehnmessstreifens 34 der Messanordnung angeordnet.

Wie in Fig. 35 gezeigt, sind das erste Messmittel 31 in einem ersten Zweig, das zweite Messmittel 32 in einem zweiten Zweig, das dritte Messmittel 33 in einem dritten Zweig und das vierte Messmittel 34 in einem vierten Zweig einer Wheatstoneschen Vollbrückenschaltung miteinander signaltechnisch verknüpft.

Dabei ist das zweite Messmittel 32 in Reihe mit dem ersten Messmittel 31 zwischen einer Speisespannung $U_E$ angeordnet, das dritte Messmittel 33 parallel zu dem zweiten Messmittel 32 zwischen der Speisespannung, und das vierte Messmittel 34 parallel zu dem ersten Messmittel 31 zwischen der Speisespannung.

Durch die Verschaltung des ersten und dritten bzw. zweiten und vierten Messmittels zu einem verknüpften Ausgangssignal in Form einer Brücken-Ausgangsspannung $U_A$ können insbesondere Biegemomente in den Antriebssträngen 11, 12 kompensiert werden, die mit keinen aktiven Kräften des Endeffektors korrespondieren. Durch die Verschaltung des ersten und zweiten bzw. dritten und vierten Messmittels im bzw. der Brücken-Ausgangsspannung $U_A$ kann insbesondere die gemeinsame Vorspannung der antriebsmodulseitigen Antriebsstranganordnung kompensiert werden, die auf die gegensinnigen Stößel 11, 12 wirkt und somit keine aktive, die Klinge 2.1 aktuierende Kraft ist. Bei im unbelasteten Zustand abgeglichener Brücke ergibt sich im Ausführungsbeispiel ein, wenigstens im Wesentlichen, linearer Zusammenhang zwischen der um die Vorspannung der Feder 5 bereinigten, d.h. aktiv die Klinge 2.1 aktuierenden Kraft und dem Doppelten der von dem Dehnmessstreifen 31 erfassten Dehnung, vorteilhafterweise also zusätzlich eine signaltechnische Verstärkung der erfassten Last.

Wie in Fig. 34 angedeutet, sind die Messmittel 31-34 der Messanordnung zum Erfassen axialer Drucklasten in Längsrichtung der Antriebsstränge 11, 12 ausgerichtet und in radialen Aussparungen der Antriebsstränge 11, 12 angeordnet.

Insbesondere, um den Elektromotor 13 und/oder ein manuelles Teleoperationsmittel, beispielsweise ein Spiegelinstrument (nicht dargestellt) zu steuern, werden durch die Messmittel 31-34 die aktiven bzw. generalisierten Lasten $F_1$, $F_2$ erfasst und der Antrieb und das Teleoperationsmittel auf Basis dieser erfassten Lasten gesteuert. Auf diese Weise kann dem Teleoperateur beispielsweise eine haptische Rückmeldung über die Klemmkräfte vermittelt werden, die der Endeffektor auf ein Lumen ausübt, bzw. über den Widerstand, den das Lumen der Klemme 2.1, 2.2 entgegensetzt.

Fig. 36 zeigt zur kompakteren Darstellung gleichermaßen einen Teil eines Steuermittels wie auch eines Verfahrens nach einer Ausführung der vorliegenden Offenbarung.

Ein Steuermittel 3, das beispielsweise in einer Steuerung des Roboters implementiert sein kann, der das minimalinvasive chirurgische Instrument der Fig. 34 führt, erhält von der Messanordnung 31-34 (vgl. auch Fig. 35) das verknüpfte Ausgangssignal $U_A$, das, wie vorstehend erläutert, insbesondere proportional zum Doppelten der Last $F_1$ im Antriebsstrang 11 ist. Auf Basis dieser durch die Messanordnung erfassten Last bestimmt das Steuermittel 3 ein Kommando S, welches es beispielsweise an eine Motorsteuerung des Elektromotors 13 oder ein Teleoperationsmittel in Form eines Spiegelinstruments (nicht dargestellt) ausgibt, so dass der Motor 13 eine gewünschte Soll-Kraft im Antriebsstrang 11 realisiert bzw. das Spiegelinstrument dem Teleoperateur eine virtuelle Belastung vermittelt, die den tatsächlich am Endeffektor wirkenden Kräften $F_{E1}$, $F_{E2}$ entspricht.

Ein Verfahren, das beispielsweise von dem vorstehend erläuterten Steuermittel 3 ausgeführt wird, steuert in entsprechender Weise den Antrieb 13 bzw. das Spiegelinstrument, indem es in einem Schritt S1 von der Messanordnung 31-34 das verknüpfte Ausgangssignal $U_A$ erhält und auf Basis dieser durch die Messanordnung erfassten Last das Kommando S bestimmt, welches beispielsweise die Motorsteuerung des Elektromotors 13 oder das Spiegelinstrument derart steuert, dass der Motor 13 die gewünschte Soll-Kraft im Antriebsstrang 11 realisiert bzw. das Spiegelinstrument dem Teleoperateur die virtuelle Belastung vermittelt, die den tatsächlich am Endeffektor wirkenden Kräften $F_{E1}$, $F_{E2}$ entspricht.

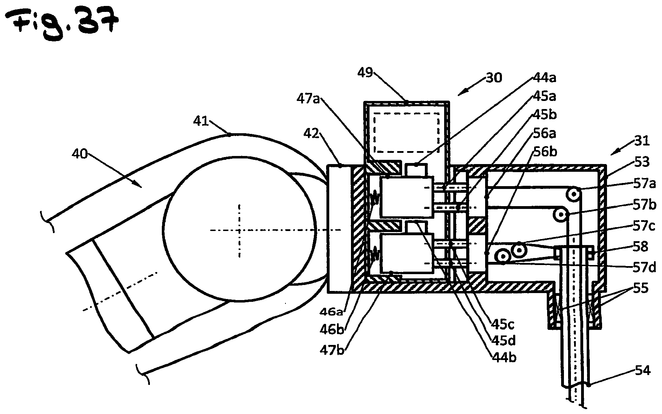

Fig. 37 zeigt einen Teil eines robotergeführten, minimalinvasiven chirurgischen Instruments nach einer Ausführung der vorliegenden Offenbarung in einem Teilschnitt. Das Instrument weist einen Instrumentenschaft 31 und eine damit lösbar verbundene Antriebseinheit 30 auf.

Der Instrumentenschaft weist eine Schnittstelle 42 zum Befestigen an einem Roboter 40 auf, der durch eine sterile Hülle 41 abgedeckt ist.

Der Instrumentenschaft weist mehrere Freiheitsgrade auf, von denen im Ausführungsbeispiel exemplarisch zwei angedeutet sind:

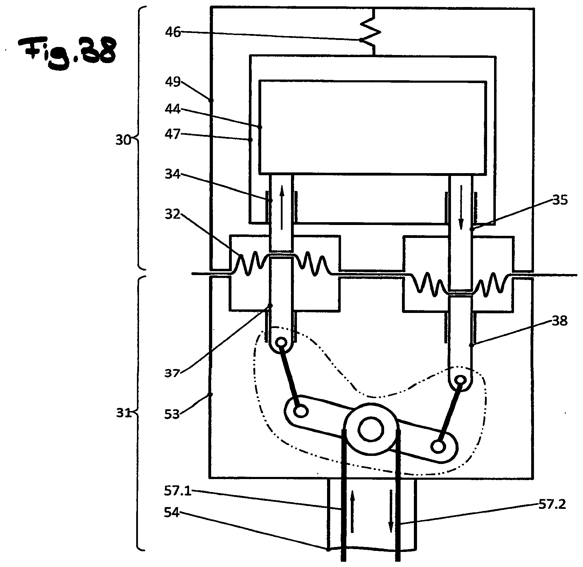

Der Instrumentenschaft weist ein Rohr 54 auf, das gegenüber einem Instrumentenschaftgehäuse 53 in einem Drehlager 55 drehbar gelagert ist. Zwei gegenläufige Zugtrumme 57c, 57d beaufschlagen gegensinnig ein Getrieberad 58 und sind jeweils mit nachfolgend näher erläuterten Eintriebsgliedern in Form von Eintriebsstößeln 37, 38 (vgl. Fig. 38) gekoppelt, die ihrerseits durch Abtriebsglieder in Form von Abtriebsstößeln 34, 35 (vgl. Fig. 38) aktuiert werden. Ab- und Eintriebsstößel 34/37 bzw. 35/38 bilden jeweils ein Stößelpaar, welches in Fig. 37 mit 45a - 45d bezeichnet ist. Durch gegensinnige Aktuierung der Stößelpaare 45c, 45d kann das Rohr 54 im Drehlager 55 in beide Richtungen verdreht und so dieser Freiheitsgrad des Instrumentenschaftes 31 aktuiert werden.

Am antriebseinheitfernen Ende des Rohres 54 ist ein Endeffektor angeordnet (nicht dargestellt), der wenigstens einen Freiheitsgrad relativ zum Rohr und/oder wenigstens einen funktionalen Freiheitsgrad, beispielsweise das Öffnen und Schließen einer Zange, aufweist. Zwei gegenläufige Zugtrumme 57a, 57b beaufschlagen gegensinnig den Endeffektor und sind jeweils mit nachfolgend näher erläuterten Eintriebsgliedern in Form von Eintriebsstößeln 37, 38 (vgl. Fig. 38) gekoppelt, die ihrerseits durch Abtriebsglieder in Form von Abtriebsstößeln 34, 35 (vgl. Fig. 38) aktuiert werden. Durch gegensinnige Aktuierung der Stößelpaare 45a, 45b kann ein Freiheitsgrad des Endeffektors aktuiert werden.

Die Eintriebsstößel 37, 38 sind im Ausführungsbeispiel translatorisch bzw. verschiebbar in einer Schnittstelle 56a bzw. 56b des Instrumentenschaftes 31 gelagert. In einer nicht dargestellten Abwandlung können gleichermaßen rotatorische bzw. drehbare Eintriebswellen drehfest mit Abtriebswellen gekoppelt sein, eine Ausführung der vorliegenden Offenbarung wird insofern nur exemplarisch mit zur Aktuierung verschiebbaren Ab- und Eintriebsgliedern erläutert, ohne hierauf beschränkt zu sein.

Die Antriebseinheit 30 weist ein Gehäuse 49 auf, in dem exemplarisch zwei Antriebsmodule 47a, 47b zum Aktuieren der vorstehend erläuterten Freiheitsgrade des Instrumentenschaftes 31 angeordnet sind. Die Antriebsmodule weisen jeweils einen Antrieb in Form eines Elektromotors 44a bzw. 44b und eine Abtriebsgliedanordnung mit zwei translatorisch beweglichen Abtriebsgliedern auf, die die Abtriebsstößel der Stößelpaare 45a, 45b bzw. 45c, 45d bilden.

Die Aktuierung der Eintriebsstößel durch Abtriebsstößel wird nachfolgend insbesondere mit Bezug auf Fig. 38 erläutert. Dabei können deren Stößelpaare 34/37 und 35/38 gleichermaßen die vorstehend genannten Stößelpaare 45a und 45b oder 45c und 45d darstellen.

Der Antrieb 44, der der Antrieb 44a oder 44b der Fig. 37 sein kann, aktuiert gegensinnig die zwei Abtriebsstößel 34 und 35, die verschiebbar in einem Gehäuse des Antriebsmoduls 47 gelagert sind, das das Antriebsmodul 47a oder 47b der Fig. 37 sein kann. Ab- und Eintriebsgliedanordnung 34, 35 und 37, 38 sind im Ausführungsbeispiel über eine optionale, flexible Sterilbarriere 32 einseitig gekoppelt. Die Eintriebsstößel 37, 38 sind über Koppelstangen mit einer Wippe gekoppelt, die ihrerseits die Zugtrumme 57.1, 57.2 gegensinnig aktuiert, die die Zugtrumme 57a, 57b oder 57c, 57d der Fig. 37 sein können. Koppelstangen und Wippe bilden ein Getriebe, welches in Fig. 38 strichpunktiert abgegrenzt ist.

Die Antriebsmodule sind, wie in Fig. 37, 38 angedeutet, in dem Gehäuse 49 der Antriebseinheit 30 jeweils in einer Koppelrichtung (horizontal in Fig. 37; vertikal in Fig. 38) gegen die Eintriebsgliedanordnung 37, 38 beweglich gelagert und vorgespannt. Die Koppelrichtungen der zwei Antriebsmodule 47a, 47b sind parallel (vgl. Fig. 37) zueinander und zur jeweiligen Aktuierungsrichtung, in der die Glieder zum Aktuieren der Freiheitsgrade des Instrumentenschaftes beweglich sind.

Die Antriebsmodule können eine Druckfeder aufweisen, die das Antriebsmodul in dem Gehäuse fesselt und in Koppelrichtung bzw. gegen die Eintriebsgliedanordnung vorspannt. Diese ist in Fig. 37 mit 46a bzw. 46b, in Fig. 38 gemeinsam mit 46 bezeichnet.

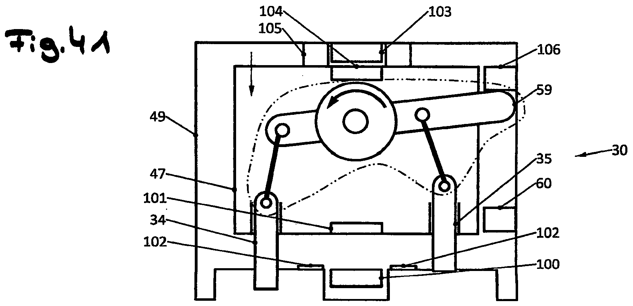

In einer in Fig. 41 gezeigten Abwandlung weist das Antriebsmodul stattdessen eine Magnetanordnung zum Vorspannen des Antriebsmoduls auf.

Die Magnetanordnung weist im Ausführungsbeispiel einen Elektromagneten 100 an dem Gehäuse 49 der Antriebseinheit auf einer instrumentenschaftzugewandten Seite (unten in Fig. 41) und diesem gegenüberliegend, einen Permanentmagneten 101 auf, der an dem Antriebsmodul 47 angeordnet ist. Zusätzlich ist ein Elektromagnet 103 an dem Gehäuse auf einer instrumentenschaftabgewandten Seite (oben in Fig. 41) angeordnet und diesem gegenüberliegend ein Permanentmagnet 104 an dem Antriebsmodul. Anstelle des Permanentmagneten 101 und/oder 104 kann auch ein weichmagnetischer Bereich vorgesehen sein, der durch die (bestromten) Elektromagnete 100 bzw. 103 angezogen werden kann.

Der bestromte Elektromagnet 100 zieht das Antriebsmodul 47 magnetisch in Koppelrichtung (nach unten in Fig. 41) und spannt so dessen Abtriebsgliedanordnung 34, 35 gegen die Eintriebsgliedanordnung (nicht dargestellt in Fig. 41) vor. Gleichermaßen kann der bestromte Elektromagnet 103 den gegenpoligen Permanentmagneten 104 abstoßen und so das Antriebsmodul 47 magnetisch in Koppelrichtung gegen die Eintriebsgliedanordnung vorspannen.

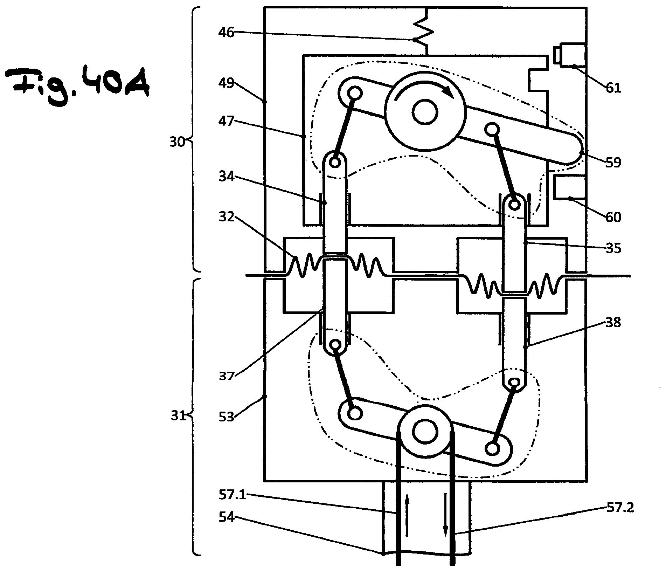

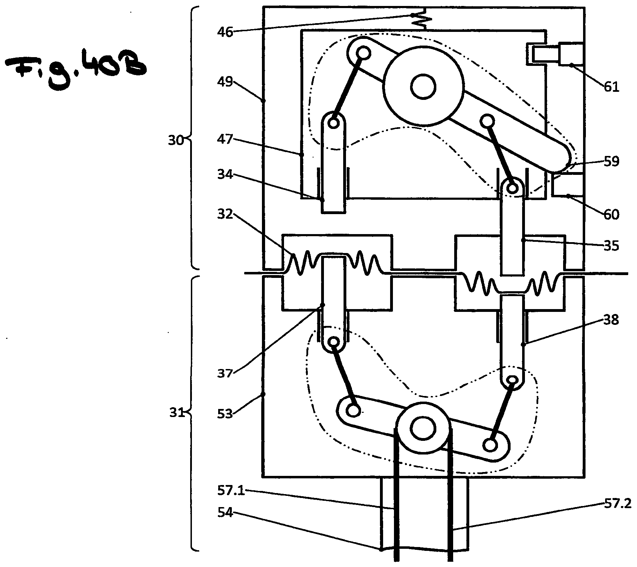

In einer nicht dargestellten Abwandlung kann einer der beiden Elektromagnete 100, 103 entfallen. Zusätzlich oder alternativ kann in einer Abwandlung anstelle des Elektromagneten 100 und/oder 103 auch ein Permanentmagnet vorgesehen sein. Die Vorspannwirkung eines Permanentmagneten 101 kann durch Bestromen des Elektromagneten 103 reduziert, insbesondere aufgehoben werden. Wenn in einer Abwandlung an Stelle des Elektromagneten 103 ein Permanentmagnet angeordnet ist, der zu dem Permanentmagneten 104 gegenpolig ist bzw. diesen magnetisch anzieht, oder der Permanentmagnet 104 durch einen weichmagnetischen Bereich des Antriebsmoduls ersetzt ist, kann hierdurch eine permanentmagnetische Antriebsmodul-Verriegelungsanordnung zum Verriegeln des zurückgezogenen Antriebsmoduls verwirklicht sein, auf die nachfolgend mit Bezug auf Fig. 40A, 40B noch näher eingegangen wird.

In der Ausführung der Fig. 41 weist die Magnetanordnung mehrere, vorzugsweise unmagnetische, Abstandselemente 102 auf, die einen direkten Kontakt zwischen dem Permanent- oder Elektromagneten 100 an dem Gehäuse der Antriebseinheit mit dem weich- oder hartmagnetischen Bereich, insbesondere (weiteren) Permanentmagneten 101 an dem Antriebsmodul verhindern. Gleichermaßen verhindern, vorzugsweise unmagnetische, Abstandselemente 105 einen direkten Kontakt zwischen dem Permanent- oder Elektromagneten 103 und dem weich- oder hartmagnetischen Bereich, insbesondere (weiteren) Permanentmagneten 104.

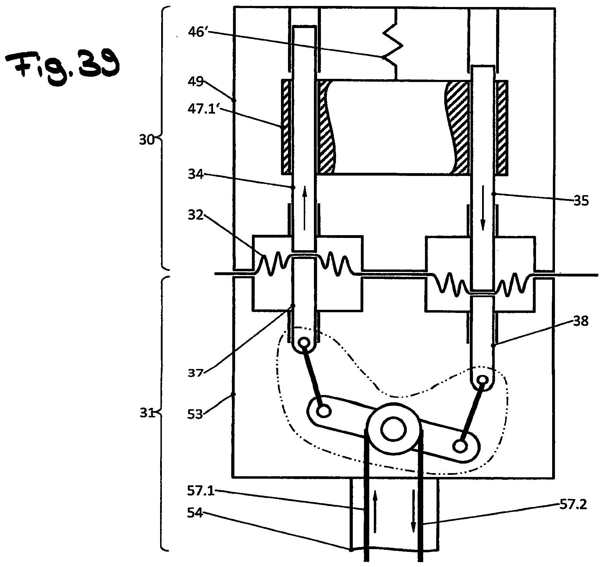

Fig. 39 zeigt ein Antriebsmodul und eine damit gekoppelte Eintriebsgliedanordnung nach einer weiteren Ausführung der vorliegenden Offenbarung in Fig. 38 entsprechender Darstellung. Mit den anderen Ausführungen übereinstimmende Merkmale sind durch identische Bezugszeichen bezeichnet, so dass auf deren Beschreibung Bezug genommen und nachfolgend nur auf die Unterschiede eingegangen wird.

Wie in der Ausführung der Fig. 38 exemplarisch gezeigt, kann ein Antriebsmodul 47 direkt im Gehäuse 49 der Antriebseinheit 30 beweglich gelagert sein, insbesondere formschlüssig, beispielsweise durch eine oder mehrere Nuten und/oder Rippen. Zusätzlich oder alternativ kann, wie in der Ausführung der Fig. 39 nur exemplarisch gezeigt, in einer Ausführung der vorliegenden Erfindung eine Abtriebsgliedanordnung in dem Gehäuse der Antriebseinheit beweglich gelagert sein, wobei der Antrieb, insbesondere ein Antriebsmodulgehäuse 47.1, in dem dieser abgestützt ist, beweglich an der Abtriebsgliedanordnung gelagert und, insbesondere elastisch und/oder permanent- und/oder elektromagnetisch, gegen das Gehäuse der Antriebseinheit gefesselt und dadurch in Koppelrichtung vorgespannt ist. In der Ausführung der Fig. 39 sind die Abtriebsstößel 34, 35 jeweils in Schublagern des Gehäuses 49 der Antriebseinheit beweglich gelagert. Ein Gehäuse 47.1 des Antriebsmoduls, in dem sich der die Abtriebsstößel 34, 35 gegensinnig beaufschlagende Antrieb abstützt, ist durch eine Magnetanordnung oder Druckfeder 46 gegen das Gehäuse 49 der Antriebseinheit gefesselt und dadurch in Koppelrichtung (vertikal nach unten in Fig. 39) vorgespannt.

Fig. 40A, 40B zeigen ein Antriebsmodul und eine damit gekoppelte Eintriebsgliedanordnung nach einer weiteren Ausführung der vorliegenden Offenbarung in Fig. 38 entsprechender Darstellung. Mit den anderen Ausführungen übereinstimmende Merkmale sind durch identische Bezugszeichen bezeichnet, so dass auf deren Beschreibung Bezug genommen und nachfolgend nur auf die Unterschiede eingegangen wird. Fig. 40A zeigt dabei das Antriebsmodul in einem mit der Eintriebsgliedanordnung gekoppelten Zustand, Fig. 40B das zurückgezogene und verriegelte Antriebsmodul.

Wie vorstehend mit Bezug auf Fig. 41 erläutert, kann durch wahlweises, insbesondere gesteuertes Bestromen einer Magnetanordnung mit wenigstens einem Elektromagneten 100 und/oder 103 das Antriebsmodul 47 gegen die Vorspannung zurückgezogen werden. Dies kann insbesondere ein An- und Abkoppeln der Antriebseinheit an den Instrumentenschaft erleichtern, da dabei nicht die (volle) Vorspannung, insbesondere manuell, überwunden werden muss. Somit stellt eine entsprechend bestromte Magnetanordnung, wie sie mit Bezug auf Fig. 41 erläutert wurde, eine magnetische Rückzuganordnung zum Zurückziehen des Antriebsmoduls gegen die Vorspannung dar.

Zusätzlich oder alternativ kann eine mechanische Rückzuganordnung zum Zurückziehen des Antriebsmoduls gegen die Vorspannung vorgesehen sein, wie sie nun exemplarisch mit Bezug auf Fig. 40A, 40B erläutert wird.

In der Ausführung der Fig. 40 weist der Antrieb ein Abtriebsmittel in Form einer Wippe 59 auf, mit der die Abtriebsstößel 34, 35 über Koppelstangen gegenläufig gekoppelt sind. Zum Aktuieren eines Freiheitsgrads des Instrumentenschaftes benötigt der Antrieb nur einen begrenzten Winkelbereich, der somit einen Aktuierbereich definiert. Von diesem ist ein Rückzugbereich durch einen mechanischen Anschlag 60 für die Wippe 59 abgegrenzt, die hierzu aus einem Gehäuse des Antriebsmoduls 47.1 hervorragt.

Solange der Antrieb die Wippe innerhalb des Aktuierbereichs bewegt, wie in Fig. 40A angedeutet, werden die Abtriebsstößel gegensinnig aktuiert. Mit Erreichen des Rückzugbereichs stützt sich, wie in Fig. 40B dargestellt, die Wippe 59 an dem mechanischen Anschlag 60 ab. Durch Weiterdrehen der Wippe in den Rückzugbereich hinein verschiebt der Antrieb über die Wippe 59 das Antriebsmodul gegen die Vorspannung des Federmittels 46 und zieht somit das Antriebsmodul motorisch gegen die Vorspannung zurück. In einer nicht dargestellten Abwandlung interagiert der Anschlag 60 nicht mit der Wippe 59, sondern einem oder beiden Stößeln 34, 35.

Wie in Fig. 41 dargestellt, kann die Rückzuganordnung 59, 60 auch mit einer magnetischen Vorspannung, insbesondere durch eine Magnetanordnung mit Permanentmagnete 101 und/oder 104, kombiniert sein.

Insbesondere, um den Antrieb zu entlasten, kann eine Antriebsmodul-Verriegelungsanordnung zum Verriegeln des zurückgezogenen Antriebsmoduls vorgesehen sein. Diese weist in der Ausführung der Fig. 40B einen federbelasteten und manuell oder automatisch lösbaren Riegel 61 auf, durch den das gegen die Vorspannung zurückgezogene Abtriebsmodul formschlüssig gesichert wird.

Die Antriebsmodul-Verriegelungsanordnung kann auch magnetisch ausgebildet sein. Wenn, wie mit Bezug auf Fig. 41 erläutert, ein Magnet, insbesondere ein Permanentmagnet 101, einen weichmagnetischen Bereich oder gegenpoligen Permanentmagneten 104 des Antriebsmoduls magnetisch anzieht, kann hierdurch das (stärker vorgespannte) Antriebsmodul magnetisch verriegelt werden. In einer Ausführung der vorliegenden Offenbarung ist die Rückzuganordnung auch zum Lösen der Verriegelung bzw. zum Verstellen des Antriebsmoduls in Koppelrichtung ausgebildet. Hierzu kann in einer Ausführung allgemein ein mechanischer Gegenanschlag vorgesehen sein, an dem sich das Abtriebsmittel abstützt, wenn es in einen von dem Aktuierungs- und Rückzugbereich verschiedenen Vorschubbereich verstellt wird. In der Ausführung der Fig. 41 ist ein entsprechender Gegenanschlag 106 am Gehäuse der Antriebseinheit angeordnet und definiert einen vom Aktuierbereich und dem durch den Anschlag 60 definierten Rückzugbereich verschiedenen Vorschubbereich. Mit Erreichen des Vorschubbereichs stützt sich, wie in Fig. 41 dargestellt, die Wippe 59 an dem mechanischen Gegenanschlag 106 ab. Durch Weiterdrehen der Wippe in den Vorschubbereich hinein verschiebt der Antrieb über die Wippe 59 das Antriebsmodul gegen die Verriegelung der Magnetanordnung 103, 104 in Koppelrichtung (vertikal nach unten in Fig. 41). Auch hier kann in einer Abwandlung der Anschlag 60 statt mit Wippe 59 mit einem oder beiden Stößeln 34, 35 interagieren.

Wie insbesondere in Fign. 37 und 42-46 erkennbar, schließt die Koppelrichtung (horizontal in den Figuren), in der das Antriebsmodul 47(a, b) in dem Gehäuse 49 der Antriebseinheit beweglich gelagert und vorgespannt ist, mit einer Längsachse des Instrumentenschaftes 31 (vertikal in den Figuren) einen Winkel ein, der im Wesentlichen 90° beträgt.

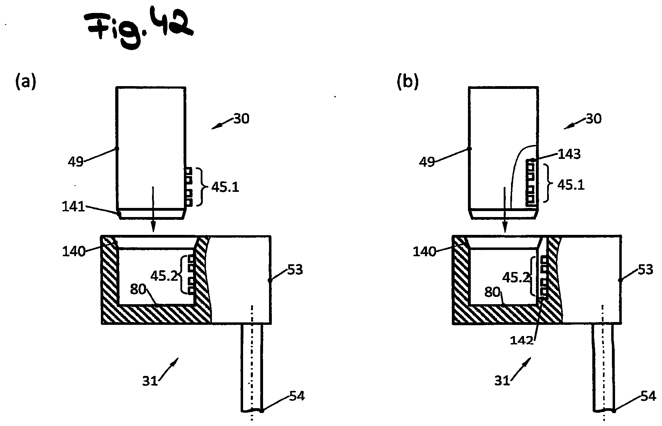

Mit Bezug auf Fig. 42 - 46 wird nachfolgend eine Aufnahme des Instrumentenschafts zur formschlüssigen, lösbaren Befestigung der Antriebseinheit nach verschiedenen Ausführungen der vorliegenden Erfindung erläutert. Mit den anderen Ausführungen übereinstimmende Merkmale sind durch identische Bezugszeichen bezeichnet, so dass auf deren Beschreibung Bezug genommen und nachfolgend nur auf die Unterschiede eingegangen wird. Die Figuren zeigen jeweils einen Teil des Instrumentenschafts mit seiner Aufnahme sowie die noch hiervon getrennte Antriebseinheit, wobei eine Einführrichtung der Antriebseinheit in die Aufnahme durch einen Bewegungspfeil angedeutet ist.

Die Aufnahme 80 der Ausführung der Fig. 42(a) weist eine angefaste Einführöffnung 140 zum Einführen der Antriebseinheit 30 in einer Einführrichtung auf, wobei die Einführrichtung parallel zur Längsachse des Instrumentenschaftes 31 (vertikal in Fig. 42(a)) ist. Die Einführöffnung 140 ist auf der instrumentenschaftabgewandten Seite (oben in Fig. 42(a)) angeordnet.

Die beweglichen Eintriebsglieder der Eintriebsgliedanordnung 45.2 des Instrumentenschaftes, beispielsweise die Eintriebsstößel 37, 38, wie sie vorstehend mit Bezug auf Fig. 38 - 41 erläutert wurden, stehen senkrecht zur Längsachse des Instrumentenschaftes 31 zu dessen Aufnahme 80 hin vor, wobei die Schnittstelle bzw. Kontaktebene der Eintriebsgliedanordnung 45.2 parallel zur Längsachse ist.

In der Ausführung der Fig. 42(b) ist die Eintriebsgliedanordnung 45.2 des Instrumentenschaftes 31 in einer Vertiefung 142 angeordnet. Zusätzlich oder alternativ ist die Abtriebsgliedanordnung 45.1 der Antriebseinheit 30, beispielsweise die Abtriebsstößel 34, 35, wie sie vorstehend mit Bezug auf Fig. 38 - 41 erläutert wurden, in einer Vertiefung 143 angeordnet, wenn sie durch die Rückzuganordnung gegen die Vorspannung zurückgezogen ist. Nach Einführen der Antriebseinheit 30 in die Aufnahme 80 und Entriegeln der Rückzuganordnung bzw. Aufbauen der Vorspannung kontaktiert die vorgespannte Abtriebsgliedanordnung 45.1, die dann aus der Vertiefung 143 vorsteht, die Eintriebsgliedanordnung 45.2 des Instrumentenschaftes 31.

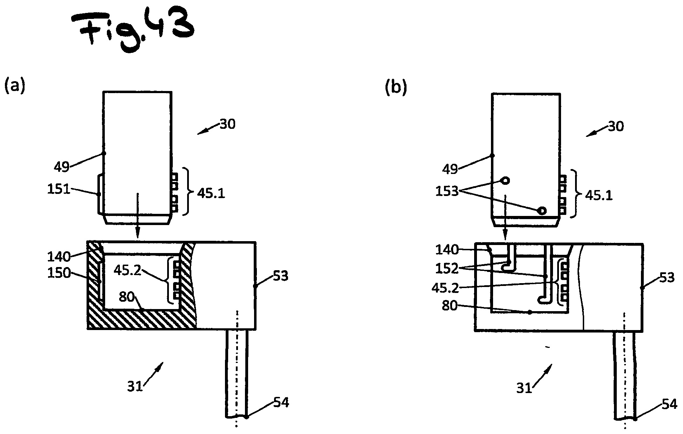

Die Ausführung der Fig. 43(a) entspricht im Wesentlichen derjenigen der Fig. 42(a). Zum formschlüssigen Befestigen der Antriebseinheit 30 in der Aufnahme 80 des Instrumentenschafts 31 ist ein Bajonettverschluss mit wenigstens einem Vorsprung 151 am Gehäuse 49 der Antriebseinheit vorgesehen, der infolge einer Verdrehung der Antriebseinheit in eine Aussparung 150 der Aufnahme 80 eingreift. Gleichermaßen kann der Vorsprung 151 in einer Abwandlung infolge einer Verschiebung (horizontal nach links in Fig. 43(a)) statt durch eine Verdrehung in die Aussparung 150 der Aufnahme 80 eingreifen, wobei diese Verschiebung vorzugsweise durch Aufprägen der Vorspannkraft erfolgt. Der Anwender schiebt somit die Antriebseinheit (vertikal von oben in Fig. 43(a)) in die Aufnahme ein. Anschließend wird, insbesondere manuell oder automatisch, ein Ankopplungsvorgang initiiert, bei dem die Schnittstellen mit der Vorspannkraft beaufschlagt werden. Dadurch wird der Vorsprung 151 der Antriebseinheit senkrecht zur Einführrichtung in die Aussparung 150 gedrückt und somit die Antriebseinheit formschlüssig verriegelt.

Die Ausführung der Fig. 43(b) entspricht im Wesentlichen derjenigen der Fig. 42(a), 43(a). Die Aufnahme 80 weist in dieser Ausführung eine mehrteilige formschlüssige Führung zum Einführen der Antriebseinheit 30 in der Einführrichtung auf. Die Führung weist mehrere Führungsnuten 152 auf, die mit entsprechenden Vorsprüngen 153 am Gehäuse 49 der Antriebseinheit 30 formschlüssig zusammenwirken, um dieses formschlüssig in der Aufnahme 80 des Instrumentenschafts 31 zu befestigen. Die Führungsnuten 152 sind im Wesentlichen L-förmig ausgebildet, so dass die Antriebseinheit wiederum infolge einer Verdrehung in der Aufnahme formschlüssig festgelegt werden kann. Wie bei dem Bajonettverschluss der Ausführung nach Fig. 43(a) ist die nach Einführen in der Aufnahme verdrehte und hierdurch formschlüssig festgelegte Antriebseinheit entgegen der Einführrichtung vorgespannt, beispielsweise durch entsprechendes Übermaß oder elastische Federmittel (nicht dargestellt), um so reibschlüssig einem Zurückdrehen und damit Lösen der Antriebseinheit entgegenzuwirken. Gleichermaßen können auch hier in einer Abwandlung wie in der vorstehend mit Bezug auf Fig. 43(a) erläuterten Abwandlung die Vorsprünge 153 infolge einer Verschiebung (horizontal nach links in Fig. 43(b)) in den kurzen Schenkel der Aussparungen 152 senkrecht zur Einführrichtung verschoben werden, wobei diese Verschiebung wiederum vorzugsweise durch Aufprägen der Vorspannkraft erfolgt. Der Anwender schiebt somit die Antriebseinheit (vertikal von oben in Fig. 43(b)) in die Aufnahme ein. Dabei gleiten die Vorsprünge 153 in den langen Schenkeln der L-förmigen Aussparungen 152 bis zu deren Knick. Anschließend wird, insbesondere manuell oder automatisch, ein Ankopplungsvorgang initiiert, bei dem die Schnittstellen mit der Vorspannkraft beaufschlagt werden. Dadurch werden die Vorsprünge 153 der Antriebseinheit senkrecht zur Einführrichtung in den Aussparungen 152 verschoben und somit die Antriebseinheit formschlüssig verriegelt.

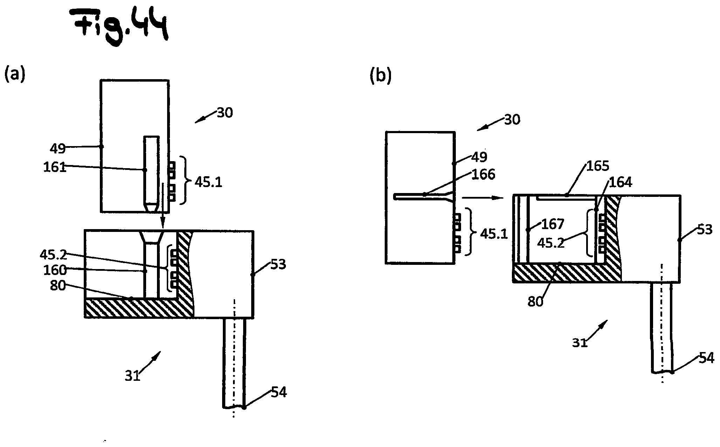

Die Ausführung der Fig. 44(a) entspricht im Wesentlichen derjenigen der Fig. 43(b), wobei hier eine Führungsrippe 161, die sich in Einführrichtung erstreckt, in eine hierzu komplementäre Führungsnut 160 der Aufnahme 80 eingeführt und in dieser beispielsweise reibschlüssig festgelegt wird bzw. ist. In einer Ausführung der vorliegenden Erfindung, wie sie exemplarisch in Fig. 44(a) dargestellt ist, weist die Aufnahme allgemein zusätzlich zu der Einführöffnung eine weitere Öffnung (links in Fig. 44(a)) auf, insbesondere, um eine signal- und/oder energietechnische Verbindung der Antriebseinheit zu verbessern (nicht dargestellt).

In der Ausführung der Fig. 44(b) ist die Einführrichtung senkrecht zur Längsachse des Instrumentenschaftes. Die Einführöffnung ist auf der instrumentenschaftabgewandten Seite (links in Fig. 44(b)) angeordnet.

In der Ausführung der Fig. 44(b) ist eine Antriebseinheit-Verriegelungsanordnung zum formschlüssigen Verriegeln der Antriebseinheit 30 in der Aufnahme 80 in Form eines beweglichen, vorgespannten Riegels 167 vorgesehen, der in der Antriebseinheit 30 einrastet, wenn diese in der Aufnahme 80 platziert ist. Obwohl nicht dargestellt, kann eine solche oder ähnliche Antriebseinheit-Verriegelungsanordnung auch in den anderen Ausführungen vorgesehen sein, insbesondere zusätzlich oder alternativ zu einer formschlüssigen Festlegung, insbesondere einem Bajonettverschluss, oder einer reibschlüssigen Festlegung.

Die Aufnahme 80 der Ausführung der Fig. 44(b) weist eine oder mehrere Führungsrippen 165 auf, die in entsprechende Führungsnuten 166 im Gehäuse 49 der Antriebseinheit 30 eingreifen. Wie mit Bezug auf Fig. 42(b) beschrieben, ist die Eintriebsgliedanordnung 45.2 in einer Vertiefung 164 angeordnet.

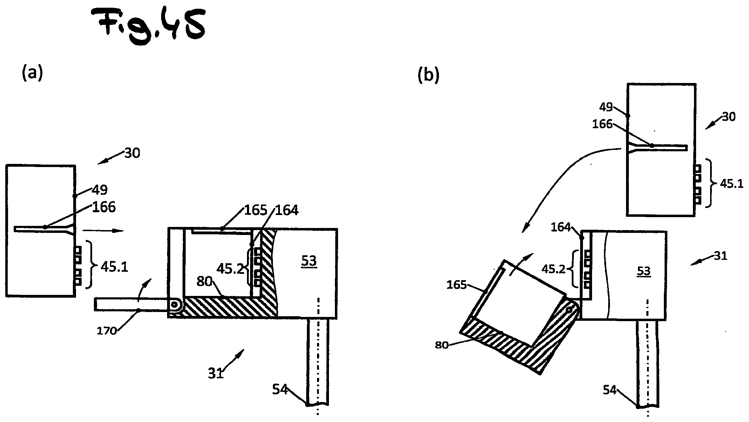

Die Ausführung der Fig. 45(a) entspricht im Wesentlichen derjenigen der Fig. 44(b), wobei die Einführöffnung durch eine verschwenkbare Klappe 170 verschließbar ist, um die Antriebseinheit 30 entgegen der Einführrichtung formschlüssig festzulegen.

In der Ausführung der Fig. 45(b) ist die Aufnahme 80 relativ zur Längsachse des Instrumentenschafts verschwenkbar. Dies ermöglicht es, die Antriebseinheit 30, wie in Fig. 45(b) durch Bewegungspfeile angedeutet, zunächst in die in eine Aufnahmestellung (vgl. Fig. 45(b)) verschwenkte Aufnahme einzuführen, und dann die Aufnahme in eine Verriegelungsstellung zu verschwenken, wobei die Antriebseinheit in dieser Verriegelungsstellung der Aufnahme dann formschlüssig fixiert ist.

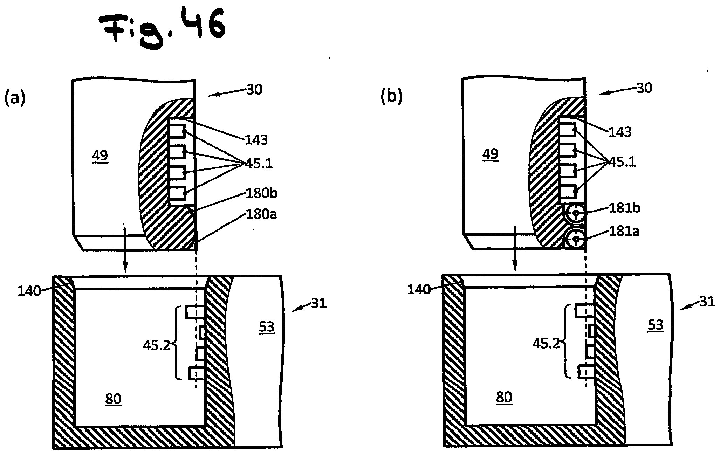

In der Ausführung der Fig. 46(a) weist die Antriebseinheit 30 ein konvergentes Verdrängungsmittel zum Verdrängen der Eintriebsgliedanordnung des Instrumentenschaftes bei einem Einführen der Antriebseinheit in die Aufnahme des Instrumentenschaftes auf. Das konvergente Verdrängungsmittel der Ausführung der Fig. 46(a) weist eine konvexe, insbesondere schräge oder elliptische Fläche auf, die in einem ersten Abschnitt 180a in Einführrichtung konvergiert und so überdurchschnittlich vorstehende Eintriebsglieder der Eintriebsgliedanordnung 45. 2 beim Einführen formschlüssig zurückdrängt. An die in Einführrichtung konvergierende Fläche 180a schließt sich eine in Einführrichtung divergierende, in der Ausführung der Fig. 46(a) ebenfalls konvexe Fläche 180b an, um auch bei einem Ausführen der Antriebseinheit 30 aus der Aufnahme 80 vorstehende Eintriebsglieder zurückzudrängen.

In der Ausführung der Fig. 46(b) weist die Antriebseinheit 30 hingegen ein bewegliches Verdrängungsmittel in Form mehrerer drehbarer Rollen 181a, 181b auf, welche beim Einführen überdurchschnittlich vorstehende Eintriebsglieder der Eintriebsgliedanordnung 45.2 zurückdrängen und so die Eintriebsgliedanordnung nivellieren. Nach Überlaufen der Rollen 181a, 181b bzw. der konvexen Fläche 180a stehen die Eintriebsglieder, wenigstens im Wesentlichen, gleichmäßig zu der Aufnahme des Instrumentenschaftes hin vor.

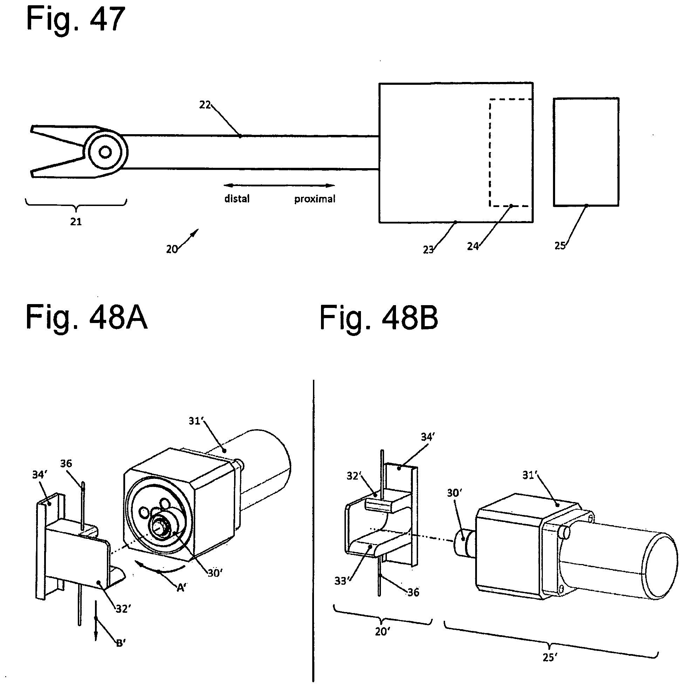

Fig. 47 zeigt schematisch ein chirurgisches Instrument nach einer Ausführung der vorliegenden Offenbarung mit einem Instrumentenschaft 20. Der Instrumentenschaft weist ein starres, gelenkiges oder biegsamens Rohr 22 auf, an dessen distalem Ende ein Endeffektor 21 angeordnet ist, der einen oder mehrere funktionale und/oder kinematische Freiheitsgrade aufweist. In einem proximalen Instrumentengehäuse 23 des Instrumentenschafts ist ein Antriebsmodul 25 lösbar an einer Schnittstelle 24 mit dem Instrumentenschaft verbunden. Das Rohr 22 kann an dem Instrumentengehäuse 23 fixiert oder drehbar gelagert sein, sodass das Rohr 22 einen Drehfreiheitsgrad um seine Längsachse aufweist.

Fig. 48A, 48B zeigen diese Schnittstelle in unterschiedlichen perspektivischen Ansichten. Zur besseren Übersicht sind nur einige Komponenten des Antriebsmoduls 25 und Instrumentenschaftes 20 dargestellt und daher durch einen Apostroph (') gekennzeichnet. Insbesondere ist nur ein Antriebsstrang zur Aktuierung eines Freiheitsgrades des Instrumentenschaftes gezeigt, weitere Antriebsstränge sind analog aufgebaut und beispielsweise parallel zu dem gezeigten Antriebsstrang angeordnet.

Jeder Antriebsstrang weist einen Aktuator in Form einer Elektromotor-Getriebe-Einheit 31' auf, deren Abtriebswelle ein unbegrenzt drehbares Abtriebsglied des Antriebsmoduls darstellt.

Mit diesem Abtriebsglied wird in nachfolgend beschriebener Weise ein Eintriebsglied 32' gekoppelt, das in einem Schublager 34' formschlüssig in einer Verschiebeachse B' verschiebbar in dem Instrumentenschaft geführt ist.

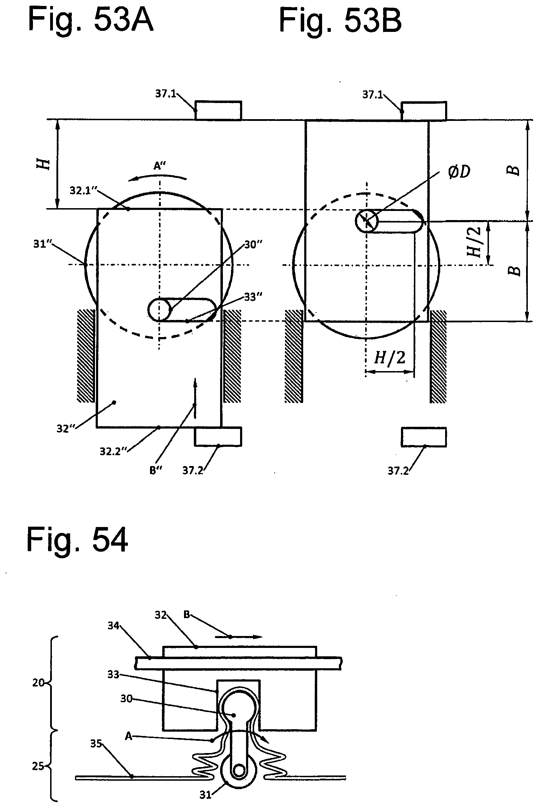

Das Eintriebsglied ist durch ein Zugmittel oder eine Druckstange 36 mit dem Endeffektor 21 verbunden, um diesen zu aktuieren (nicht dargestellt), wobei die Druckstange parallel zu der Verschiebeachse B' ist. Das Eintriebsglied ist zwischen zwei Endanschlägen 37.1, 37.2 (vgl. Fig. 53, in Fig. 48 nicht dargestellt) verschiebbar.

An dem Eintriebsglied ist eine lineare Nut 33' angeordnet, die senkrecht zu der Verschiebeachse B' ist. Ein Führungselement 30' ist exzentrisch an dem drehbaren Abtriebsglied angeordnet und in der Nut verschiebbar geführt, wenn Ab- und Eintriebsglied miteinander gekoppelt sind. Die Drehachse des drehbaren Abtriebsgliedes ist senkrecht zu der Verschiebeachse B' des verschiebbar geführten Eintriebsgliedes und der Nut.

Das Führungselement 30' weist einen Zapfen auf, auf dem ein Wälzkörper in Form eines Laufrings gleit- oder wälzgelagert ist. In einer Abwandlung kann stattdessen auch ein außenringloses Wälzlager auf dem Zapfen angeordnet sein.

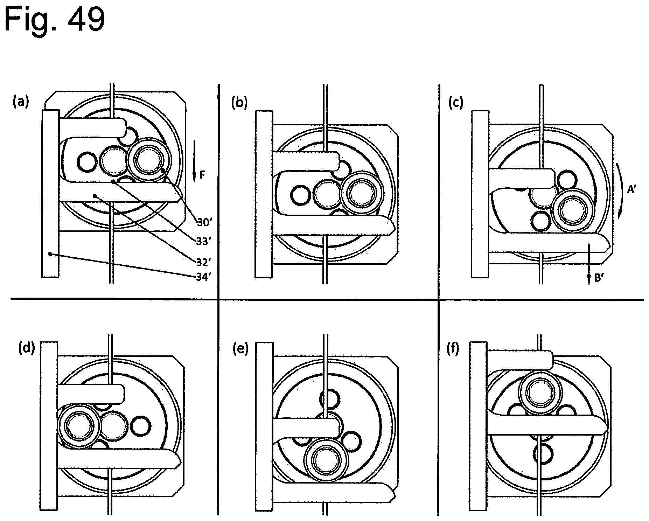

Fig. 49(a), (b) zeigen Schritte beim Koppeln des Führungselements mit der Nut, Fig. 49(c)-(f) Schritte beim Aktuieren des Eintriebsglieds durch das Abtriebsglied.

In Fig. 49(a) sind Antriebsmodul und Instrumentenschaft miteinander verbunden, wobei Abtriebsglied und Eintriebsglied 32' noch nicht miteinander gekoppelt sind. Durch Drehen des Abtriebsgliedes (vgl. Bewegungspfeil A' in Fig. 48A, 49(c)) rotiert das Führungselement 30' durch eine Öffnung in der in Fig. 49 oberen Führungswand der Nut 33' in die Nut (vgl. Bewegungspfeil F in Fig.49(a)) und koppelt so - zunächst einseitig - Abtriebsglied und Eintriebsglied (Fig. 49(b)). Bei Weiterdrehen des Abtriebsgliedes (vgl. Bewegungspfeil A' in Fig. 49(c)) verschiebt das nun in der Nut 33' geführte Führungselement 30' das Eintriebsglied 32' im Schublager 34' in seiner Verschiebeachse B'. In den Fig. 49(d) bis 49(f) wird deutlich, wie das drehende Abtriebsglied das Eintriebsglied beidseitig entlang seiner Verschiebeachse B' verschiebt und so den Endeffektor aktuieren kann: durch Drehen des Abtriebsglieds und des daran exzentrisch angeordneten Führungselements 30' in Richtung bzw. entgegen dem Bewegungspfeil A' in Fig. 49(c) kann das Eintriebsglied 32' in seiner Verschiebeachse B' in beide Richtungen (nach oben/unten in Fig. 49) verschoben und so über das Zugmittel bzw. die Druckstange 36 (vgl. Fig. 48) ein intrakorporaler Freiheitsgrad des Instruments aktuiert werden.

In der Ausführung der Fig. 48, 49 weist die (in den Figuren obere) Führungswand der Nut eine Öffnung zum Einführen des Führungselementes durch Drehen des Abtriebsgliedes auf, die durch einen verkürzten Schenkel eines offenen bzw. U-förmigen Schenkelpaares ausgebildet ist, das seinerseits die Nut definiert.

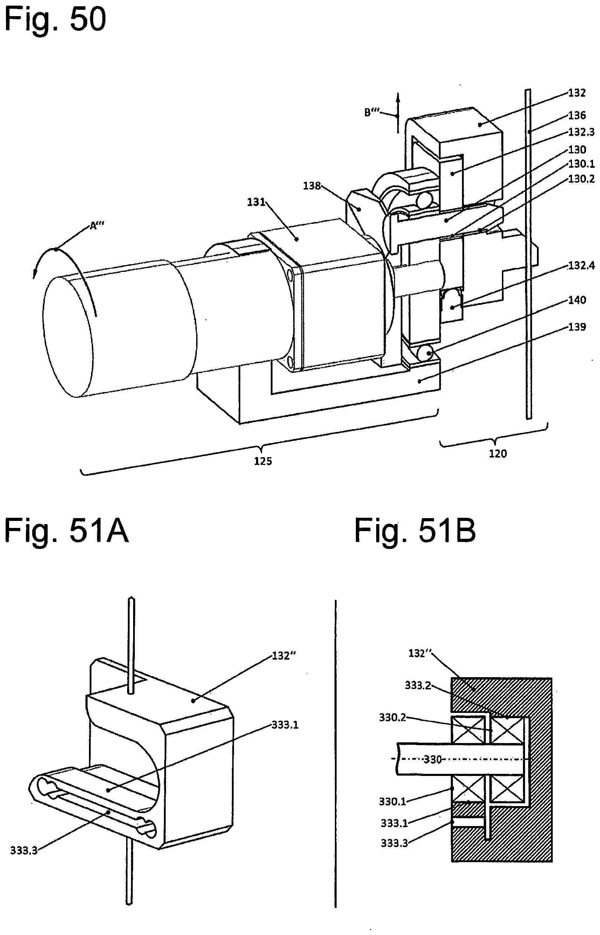

Fig. 50 zeigt in Fig. 48 entsprechender Weise eine Schnittstelle eines chirurgischen Instruments nach einer weiteren Ausführung der vorliegenden Offenbarung in einem Teilschnitt. Wie in Fig. 48 sind zur besseren Übersicht nur einige Komponenten eines Antriebsmoduls 125 und Instrumentenschaftes 120 dargestellt, insbesondere nur ein Antriebsstrang zur Aktuierung eines Freiheitsgrades des Instrumentenschaftes gezeigt, weitere Antriebsstränge können analog aufgebaut und beispielsweise parallel zu dem gezeigten Antriebsstrang angeordnet sein.

Jeder Antriebsstrang weist einen Aktuator, beispielsweise in Form einer Elektromotor-Getriebe-Einheit, 131 auf, deren Abtriebswelle ein unbegrenzt drehbares Abtriebsglied des Antriebsmoduls darstellt.

Mit diesem Abtriebsglied wird in nachfolgend beschriebener Weise ein Eintriebsglied 132 gekoppelt, das in einem Schublager (nicht dargestellt) formschlüssig in einer Verschiebeachse B''' verschiebbar in dem Instrumentenschaft geführt und durch ein Zugmittel oder eine Druckstange 136 mit dem Endeffektor verbunden ist, die parallel zu der Verschiebeachse B''' ist.

In dem Eintriebsglied ist eine lineare Nut (geschnitten in Fig. 50) angeordnet, die senkrecht zu der Verschiebeachse B''' und einer Achse eines Führungselements 130 ist, welches exzentrisch an dem drehbaren Abtriebsglied angeordnet und in der Nut verschiebbar geführt, wenn Ab- und Eintriebsglied miteinander gekoppelt sind. Die Drehachse des drehbaren Abtriebsgliedes ist senkrecht zu der Verschiebeachse B''' des verschiebbar geführten Eintriebsgliedes und der Nut. Das exzentrische Führungselement 130 stützt sich über ein Radiallager 140 an einem Rahmen 139 des Aktuators 131 ab.

In der Ausführung der Fig. 50 ist ein Toleranzelement 132.3 vorgesehen. Das Toleranzelement ist an dem Eintriebsglied 132 parallel zu dessen Verschiebeachse B''' verschiebbar geführt und gegen dieses durch ein Federmittel 132.4 elastisch vorgespannt. Hierdurch verspannt das Toleranzelement 132.3 das Abtriebsglied und das Eintriebsglied in der Verschiebeachse B''' des Eintriebsgliedes, wenn Ab- und Eintriebsglied miteinander gekoppelt sind.

Das Toleranzelement weist eine Toleranzelementnut auf, die parallel zu der Nut in dem Eintriebsglied 132 ist und von dem Führungselement 130.2 durchgriffen wird, wenn Ab- und Eintriebselement gekoppelt sind.

In der Ausführung der Fig. 50 weist das Führungselement einen drehbar gelagerten Wälzkörper in Form eines gleit- oder wälzgelagerten Laufrings 130.2 zum Kontaktieren der Nut in dem Eintriebselement auf. Axial daneben ist ein weiterer drehbar gelagerter Wälzkörper in Form eines gleit- oder wälzgelagerten Laufrings 130.1 zum Kontaktieren der Toleranzelementnut angeordnet. In einer Abwandlung können stattdessen auch außenringlose Wälzlager vorgesehen sein.

Das Führungselement 130 ist axial verschiebbar in dem Abtriebsglied gelagert. Hierdurch kann es axial in die Nut in dem Eintriebselement und die Toleranzelementnut ein- bzw. aus diesen ausgeführt werden. Es ist durch eine Axialfeder (nicht dargestellt) gegen die Nuten vorgespannt, so dass es selbsttätig in diese einfährt.

Eine Kulisse 138 zum axialen Verschieben des Führungselements ist drehfest mit dem Rahmen 139 verbunden. Sie weist eine Schräge in Drehrichtung auf, auf der ein Bund des Führungselements aufläuft. Auf diese Weise kann durch Drehen des Abtriebsgliedes in der in Fig. 50 durch einen Bewegungspfeil A''' angedeuteten Richtung über die Kulisse 138 das Führungselement 130.2 axial verschoben (nach links in Fig. 50) und so außer Eingriff mit den Nuten gebracht werden. In einer nicht dargestellten Abwandlung kann durch die Kulisse das Führungselement in voneinander beabstandeten Drehstellungen gegensinnig axial verschoben und so nicht nur außer Eingriff, sondern auch in Eingriff mit den Nuten gebracht werden. Hierfür kann die Kulisse entsprechend der in Fig. 50 dargestellten Schräge eine weitere, hierzu gegensinnige und in Drehrichtung beabstandete Schräge aufweisen, die den Bund des Führungselements bei Drehen des Abtriebsglieds entgegen der Richtung A''' axial in die Nut einschiebt. In diesem Fall kann eine Vorspannung durch eine Axialfeder reduziert werden oder entfallen.

Fig. 51A, 51B zeigen in perspektivischer Ansicht (Fig. 51A) und einem Teilschnitt (Fig. 51B) eine Schnittstelle eines chirurgischen Instruments nach einer weiteren Ausführung der vorliegenden Offenbarung. Diese entspricht im Wesentlichen der Ausführung der Fig. 50, so dass auf deren Beschreibung Bezug genommen und nachfolgend nur auf die Unterschiede eingegangen wird.

In der Ausführung der Fig. 51 ist das Toleranzelement integral mit dem Eintriebsglied 132" ausgebildet, und zwar durch einen Hohlraum 333.3, in den eine integraler Schenkel 333.1 einfedern kann, der beidseitig (links, rechts in Fig. 51A) gelagert ist.

Im Teilschnitt der Fig. 51B ist das Führungselement 330 erkennbar, das durch ein Wälzlager 330.2 in der Nut 333.2 des Eintriebsglieds 132" geführt ist. Zusätzlich stützt sich das Führungselement 330 über ein weiteres Wälzlager 330.1 an dem Schenkel 333.1 des integralen Toleranzelements ab, welches das Führungselement 330 und damit das Abtriebsglied, in dem dieses gelagert ist, und das Eintriebsglied 132" in einer Verschiebeachse des Eintriebsgliedes (vertikal in Fig. 51B) verspannt.

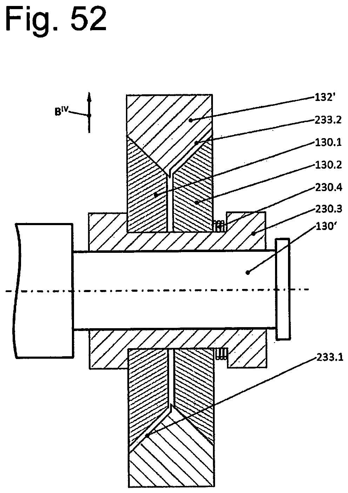

Fig. 52 zeigt in Fig. 51B entsprechender Weise eine Schnittstelle eines chirurgischen Instruments nach einer weiteren Ausführung der vorliegenden Offenbarung in einem Teilschnitt. Diese entspricht im Wesentlichen der Ausführung der Fig. 50, so dass auf deren Beschreibung Bezug genommen und nachfolgend nur auf die Unterschiede eingegangen wird.

In der Ausführung der Fig. 52 ist auf einem Zapfen 130' des Führungselements ein Innenring 230.3 eines außenringlosen Wälzlagers mit Wälzkörpern 130.1, 130.2 angeordnet. Dabei fungiert der in Fig. 52 rechte Wälzkörper 130.2 als Toleranzelement, das das Führungselement und damit das Abtriebsglied gegen das Eintriebsglied 132' in einer Verschiebeachse $B^{IV}$ des Eintriebsgliedes verspannt, wenn Ab- und Eintriebsglied miteinander gekoppelt sind.

Hierzu weist der in Fig. 52 linke Wälzkörper 130.1 des Führungselementes und das Toleranzelement 130.2 gegensinnige Schrägen auf, die zu gegensinnigen Schrägen 233.1, 233.2 des Eintriebselements 132' komplementär sind. Das Toleranzelement 130.2 ist auf dem Innenring 230.3 des Führungselements axial verschiebbar geführt und gegen diesen durch ein Federmittel 230.4 elastisch vorgespannt. Durch das federmittelbedingte axiale Zustellen der Schräge des Toleranzelementes 130.2 verspannt dieses das Abtriebsglied und das Eintriebsglied 132' in der Verschiebeachse $B^{IV}$.

Wie vorstehend ausgeführt, stellen auch der in Fig. 52 linke Laufring 130.1, der radial außen an dem Eintriebsglied 132' und/oder radial innen auf dem Innenring 230.3 gleitgelagert sein bzw. gleiten kann, sowie das in Fig. 52 rechte Toleranzelement 130.2, das radial außen an dem Eintriebsglied 132' und/oder radial innen auf dem Innenring 230.3 gleitgelagert sein bzw. gleiten kann, Wälzkörper im Sinne der vorliegenden Offenbarung dar, Wälzkörper 130.1, 130.2 und Innenring 230.3 zusammen somit ein außenringloses Wälzlager im Sinne der vorliegenden Erfindung. Zusätzlich oder alternativ zu einer Drehbarkeit bzw. Gleitlagerung der Wälzkörper 130.1, 130.2 gegenüber dem Eintriebsglied 132' und/oder dem Innenring 230.3 kann der Innenring 230.3 auf dem Zapfen 130' drehfest oder gleitgelagert sein bzw. gleiten.

Fig. 53A, 53B zeigen eine Schnittstelle eines chirurgischen Instruments nach einer weiteren Ausführung der vorliegenden Offenbarung in verschiedenen Stellungen. Diese entspricht im Wesentlichen den vorstehend erläuterten Ausführungen, so dass auf deren Beschreibung Bezug genommen und nachfolgend nur auf die Unterschiede eingegangen wird.

In der Ausführung der Fig. 53 ist die O-förmig geschlossene Nut 33" im Eintriebselement 32" asymmetrisch zur Drehachse des Abtriebsgliedes 31" (senkrecht auf der Bildebene der Fig. 53) und Verschiebeachse B" des Eintriebselements 32" ausgebildet und erstreckt sich im Wesentlichen nur bis zu dieser Drehachse.

Hierdurch werden Abtriebsglied 31" und Eintriebsglied 32" eindeutig miteinander gekoppelt. Denkt man sich die Nut 33" hingegen über die Drehachse hinaus, insbesondere symmetrisch zu dieser, verlängert (nach links in Fig. 53), wird deutlich, dass das Führungselement 30" dann jeweils in zwei zur Verschiebeachse B" symmetrischen Drehstellungen in die Nut 33" eingreifen könnte. Durch eine asymmetrische Ausbildung der Nut 33' kann dies verhindert werden, durch diese kann das Führungselement 30" nur in je genau einer Drehstellung in die Nut 33" eingreifen.

Die Figurenfolge Fig. 53A → Fig. 53B verdeutlicht nochmals das Funktionsprinzip der Schnittstelle nach einer Ausführung der Offenbarung. Dreht das Abtriebselement 31" in der in Fig. 53 durch den Bewegungspfeil A" angedeuteten Richtung, wird das hiermit gekoppelte Eintriebsglied 32" in seinem Schublager (schraffiert in Fig. 53) in seiner Verschiebeachse B" verschoben. Zur Begrenzung dieser Verschiebung, insbesondere bei abgekoppeltem Abtriebsglied, sind zwei Endanschläge 37.1, 37.2 vorgesehen, auf die Stirnseiten 32.1" bzw. 32.2" die Eintriebsglieder auflaufen.

Der (Voll)Hub H des Eintriebsglieds ergibt sich bei entkoppeltem Abtriebsglied durch den Abstand der Endanschläge 37.1, 37.2 abzüglich der Abstände B der Stirnseiten 32.1" bzw. 32.2" von einer Mittellinie der Nut 33". Daher ist in einer Ausführung der vorliegenden Erfindung, für die die Darstellung der Fig. 53 nur eine mögliche Ausführung zeigt, allgemein ein Abstand B einer Stirnseite des Eintriebsgliedes von einer Mittellinie der Nut in dem Eintriebsglied wenigstens gleich dem Vollhub zuzüglich der halben Nutweite, mit den Bezeichungen der Fig. 53:

$$B \geq H + D/2$$

mit

B:    Abstand einer Stirnseite des Eintriebsgliedes von einer Mittellinie der Nut;
H:    Gesamthub des Eintriebsgliedes; und
D:    Nutweite.

Fig. 54 zeigt eine Schnittstelle eines chirurgischen Instruments nach einer weiteren Ausführung der vorliegenden Offenbarung. Diese entspricht im Wesentlichen den vorstehend erläuterten Ausführungen, so dass auf deren Beschreibung Bezug genommen und nachfolgend nur auf die Unterschiede eingegangen wird.

In der Ausführung der Fig. 54 ist zwischen dem Führungselement 30 des Abtriebsglieds 31 des Antriebsmoduls 25, das in die Nut 33 des am Schublager 34 des Instrumentenschaftes 20 in Verschiebeachse B geführten Eintriebsglieds 32 eingreift, um eine Drehbewegung A des Führungselements 30 in einer translatorischen Verschiebung des Eintriebsglieds 32 umzusetzen, eine Sterilbarriere 35 angeordnet. Diese kann auch in den vorstehend erläuterten Ausführungen der Fig. 47 - 53 vorgesehen sein, ohne dort gezeigt zu sein.

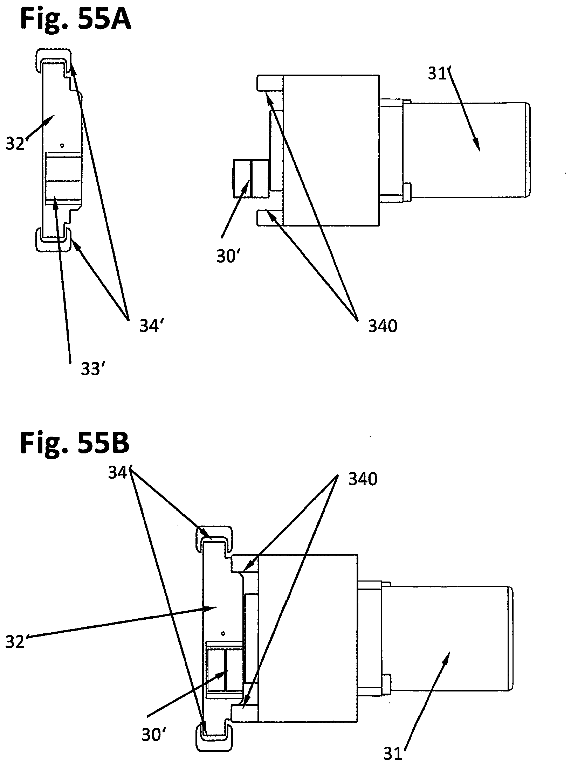

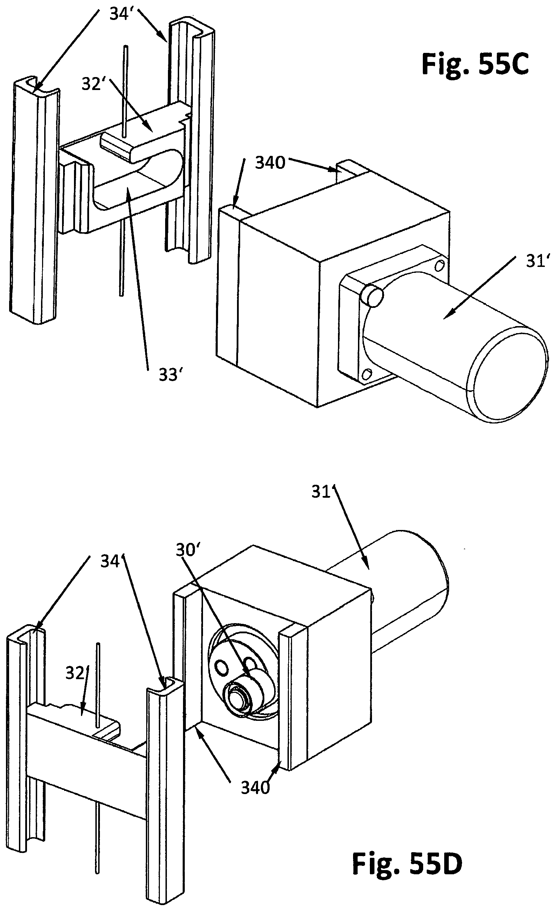

Fig. 55A - 55D zeigen eine Schnittstelle eines chirurgischen Instruments nach einer weiteren Ausführung der vorliegenden Offenbarung in einer Draufsicht in Richtung einer Verschiebeachse (Fig. 55A, 55B) bzw. in perspektivischer Ansicht (Fig. 55C, 55D), wobei das Ab- und Eintriebsglied nicht miteinander gekoppelt (Fig. 55A) bzw. miteinander gekoppelt (Fig. 55B) sind. Diese entspricht im Wesentlichen den vorstehend erläuterten Ausführungen, insbesondere gemäß Fig. 48, so dass auf die Beschreibung der vorhergehenden Ausführungen Bezug genommen und nachfolgend nur auf die Unterschiede eingegangen wird.

In der Ausführung der Fig. 55 ist das Eintriebsglied 32' in einem Schublager 34' mit einem größeren Spiel, insbesondere lose, verschiebbar an dem Instrumentenschaft geführt. Zusätzlich ist es in einem Schublager 340 mit einem kleineren Spiel, insbesondere wenigstens im Wesentlichen spielfrei, an dem Aktuator 31' des Antriebsmoduls verschiebbar geführt sein, wenn dieses mit dem Instrumentenschaft verbunden ist (vgl. Fig. 55B). Im verbundenen Zustand wird somit die ungenauere Führung am Instrumentenschaft funktionslos. Hierdurch kann die aufwändigere, präzisere Führung auf das Antriebsmodul verlagert und so der Instrumentenschaft einfacher und/oder kostengünstiger, insbesondere leichter sterilisierbar und/oder als Einmalartikel ausgebildet werden bzw. sein. Sobald Instrumentenschaft und Antriebsmodul verbunden sind, übernimmt dieses die - präzisere - Führung des Eintriebsgliedes.

## Bezugszeichen liste

In den Figuren 1 bis 33:

| | |
|---|---|
| 1 | Antriebseinheit |
| 2 | Instrumentenschaft |
| 3 | Sterilbarriere |
| 3.1 | Ausgleichselement |
| 3.2 | Wellmembran (vorgespannte Lose) |
| 3.3 | Wellbalg (vorgespannte Lose) |
| 3.4 | Elastomer-Stulpe (Lose) |
| 3.5 | translatorisch verschiebbare Dichtung |
| 3.6 | steriler Fortsatz |
| 3.7 | Aufdickung |
| 4 | Adapter (Befestigungselement) |
| 10A, 10B | Abtriebselement (Abtriebsanordnung) |
| 10C | Koppelmittel |
| 10D | Führungsschiene |
| 11 | Abtriebselementbasis |
| 20A, 20B | Eintriebselement (Antriebsanordnung) |
| 20C | Koppelmittel |
| 20D | Dreh-Schublager |

| 20E | Verzahnung |
|---|---|
| 21 | Eintriebselementbasis |
| 30 | Rolle |
| 40 | Koppelstange |
| 50 | Feder |
| 60 | Seilzug |
| 100 | Zapfen (Abtriebselement) |
| 100.1 | Dehnhülse/Spannhebel (elastischer/separater Körper) |
| 100.2 | Gewindespindel/Betätigungsstift (Klemmmittel) |
| 100.3 | Elektromotor |
| 100.4 | Spindelmutter |
| 100.5 | Schublager |
| 100.6 | Rastkugeln |
| 100.7 | Zwischenelement(anordnung) |
| 100.8 | Käfighülse |
| 200 | Kupplungsbuchse mit Aussparung (Eintriebselement) |
| 200.1 | Druckfeder |
| 1000 | Kipphebel (Ab-/Eintriebselement) |
| 2000 | Kupplungsteil (Ein-/Abtriebselement) |
| 2000.1 | Schublager |

In den Figuren 34 bis 36:

| 2.1, 2.2 | Klinge (Endeffektor) |
|---|---|
| 3 | Steuermittel |
| 4 | Sterilbarriere |
| 5 | Feder |
| 10 | Antriebsmodul |
| 11, 12 | Stößel ((antriebsmodulsseitige) Antriebsstrang(anordnung)) |
| 13 | Elektromotor (Antrieb) |
| 20 | Instrumentenschaft |
| 21, 22 | Stößel ((instrumentenschaftseitige) Antriebsstrang(anordnung)) |
| 31-34 | Dehnmessstreifen (Messmittel/-anordnung) |
| $F_{E1}$, $F_{E2}$ | Klemmkraft |
| $F_{S1}$, $F_{S2}$ | Instrumentenschaft-Stößel-Kraft |
| $F_1$, $F_2$ | Antriebsmodul-Stößel-Kraft |
| $q_1$ | (Dreh)Freiheitsgrad |
| S1 | Verfahrensschritt |
| $U_A$ | Brücken-Ausgangsspannung |
| $U_E$ | Speisespannung |

In den Figuren 37 bis 46:

| 30 | Antriebseinheit |
|---|---|
| 31 | Instrumentenschaft |
| 32 | (flexible) Sterilbarriere |
| 34, 35 | Abtriebsstößel |
| 37, 38 | Eintriebstößel |
| 40 | Roboter |
| 41 | (sterile) Hülle |
| 42 | Schnittstelle |
| 44; 44a, 44b | Elektromotor |
| 45a - 45d | Stößelpaar |
| 45.1 | Abtriebsgliedanordnung |
| 45.2 | Eintriebsgliedanordnung |
| 46; 46a, 46b | Federmittel (Druckfeder) |
| 47; 47a, 47b | Antriebsmodul |
| 47.1 | Gehäuse des Antriebsmoduls |

| | |
|---|---|
| 49 | Gehäuse |
| 53 | Instrumentenschaftgehäuse |
| 54 | Rohr |
| 55 | Drehlager |
| 56a, 56b | Schnittstelle |
| 57.1, 57.2, 57a - 57d | Zugtrumme |
| 58 | Getrieberad |
| 59 | Wippe |
| 60 | (mechanischer) Anschlag |
| 61, 167 | Riegel |
| 80 | Aufnahme |
| 100, 103 | Elektromagnet |
| 101, 104 | Permanentmagnet |
| 102 | Abstandselement |
| 105 | Abstandselement |
| 106 | Gegenanschlag |
| 140 | Einführöffnung |
| 142, 143, 164 | Vertiefung |
| 150 | Aussparung |
| 151, 153 | Vorsprung |
| 152, 160, 166 | Führungsnut |
| 161, 165 | Führungsrippe |
| 170 | Klappe |
| 180a, 108b | Abschnitt / konvergierende Fläche |
| 181a, 181b | bewegliche Rolle |

In den Figuren 47 bis 55:

| | |
|---|---|
| 20; 20'; 120 | Instrumentenschaft |
| 21 | Endeffektor |
| 22 | Rohr |
| 23 | Instrumentengehäuse |
| 24 | Schnittstelle |
| 25; 25'; 125 | Antriebsmodul |
| 30; 30'; 30"; 130; 330 | Führungselement |
| 31 | Abtriebsglied |
| 31'; 31"; 131 | Elektromotor-Getriebe-Einheit (Aktuator) |
| 32; 32'; 32"; 132; 132'; 132 | Eintriebsglied |
| 32.1"; 32.2" | Stirnseite |
| 33; 33', 33" | Nut |
| 34; 34' | Schublager |
| 35 | Sterilbarriere |
| 36; 136 | Zugseil/Druckstange |
| 37.1,37.2 | Endanschlag |
| 130.1 | Laufring (Wälzkörper) |
| 130.2 | Laufring (Toleranzelement) |
| 130' | Zapfen |

| 132.3 | Toleranzelement |
| 132.4 | Federmittel |
| 138 | Kulisse |
| 139 | Rahmen |
| 140 | Radiallager |

| 230.3 | Innenring |
| 230.4 | Federmittel |
| 233.1, 233.2 | Schrägen |

| 330.1, 330.2 | Wälzlager |
| 333.1 | Schenkel (integrales Toleranzelement) |
| 333.2 | Nut |
| 333.3 | Hohlraum |
| 340 | Schublager |

A; A'; A''; A'''      Drehbewegung

B; B'; B''; B'''; B$^{IV}$      Verschiebeachse

**Patentansprüche**

1. Instrumentenschaft (31) mit einer Eintriebsgliedanordnung (45.2) mit wenigstens einem beweglichen Eintriebsglied (37, 38) zum Aktuieren eines Freiheitsgrades des Instrumentenschaftes, welches, wenigstens im Wesentlichen, senkrecht zu einer Längsachse des Instrumentenschaftes zu einer Aufnahme (80) des Instrumentenschaftes für eine Antriebseinheit (31) hin vorsteht, wobei die Aufnahme eine Führung (152, 153; 160, 161; 165, 166) und/oder eine, insbesondere verschließbare (170), Einführöffnung (140) zum Einführen einer Antriebseinheit (30) in einer Einführrichtung aufweist, welche insbesondere, wenigstens im Wesentlichen, senkrecht oder parallel zur Längsachse des Instrumentenschaftes ist.

2. Instrumentenschaft nach dem vorhergehenden Anspruch, **dadurch gekennzeichnet, dass** die Aufnahme relativ zu einer Längsachse des Instrumentenschafts beweglich, insbesondere verschwenkbar, ist.

3. Instrumentenschaft nach einem der vorhergehenden Ansprüche, **dadurch gekennzeichnet, dass** die Eintriebsgliedanordnung in einer Vertiefung (142; 164) angeordnet ist.

4. Chirurgisches Instrument mit

     - einem Instrumentenschaft (31) nach einem der vorhergehenden Ansprüche, und
     - einer lösbar mit dem Instrumentenschaft verbindbaren Antriebseinheit (30) mit einem Gehäuse (49) und wenigstens einem Antriebsmodul (47; 47a, 47b) mit einem Antrieb und einer Abtriebsgliedanordnung (45.1) mit wenigstens einem beweglichen Abtriebsglied (34, 35) zum Aktuieren eines Freiheitsgrades des Instrumentenschaftes (31).

5. Chirurgisches Instrument nach dem vorhergehenden Anspruch, **dadurch gekennzeichnet, dass** die Koppelrichtung, in der das Antriebsmodul in dem Gehäuse der Antriebseinheit beweglich gelagert und vorgespannt ist, mit einer Längsachse des Instrumentenschaftes einen Winkel einschließt, dessen Betrag größer als 0°, insbesondere größer als 45°, insbesondere wenigstens im Wesentlichen 90° ist.

6. Chirurgisches Instrument nach einem der vorhergehenden Ansprüche 4 bis 5, **gekennzeichnet durch** eine Antriebseinheit-Verriegelungsanordnung (150, 151; 152, 153; 167; 170) zum Verriegeln der Antriebseinheit in einer Aufnahme (80) des Instrumentenschaftes.

7. Chirurgisches Instrument nach einem der vorhergehenden Ansprüche 4 bis 6, **gekennzeichnet durch** eine Schnittstelle zum Befestigen an einem Roboter (40).

8. Chirurgisches Instrument nach einem der vorhergehenden Ansprüche 4 bis 7, **dadurch gekennzeichnet, dass**

43

das Antriebsmodul (47; 47a, 47b) in dem Gehäuse in einer Koppelrichtung beweglich gelagert und vorgespannt ist.

9.  Chirurgisches Instrument nach einem der vorhergehenden Ansprüche 4 bis 8, **dadurch gekennzeichnet, dass** die Antriebseinheit eine Magnetanordnung (100, 101, 103, 104) zum Vorspannen des Antriebsmoduls in der Koppelrichtung aufweist.

10. Chirurgisches Instrument nach einem der vorhergehenden Ansprüche 4 bis 9, **dadurch gekennzeichnet, dass** die Antriebseinheit eine, insbesondere mechanische und/oder magnetische, Rückzuganordnung (59, 60) zum Zurückziehen des Antriebsmoduls gegen die Vorspannung aufweist.

11. Chirurgisches Instrument nach dem vorhergehenden Anspruch, **dadurch gekennzeichnet, dass** die Antriebseinheit eine Antriebsmodul-Verriegelungsanordnung (61; 103, 104) zum Verriegeln des zurückgezogenen Antriebsmoduls aufweist.

12. Chirurgisches Instrument nach einem der vorhergehenden Ansprüche 10 bis 11, **dadurch gekennzeichnet, dass** die Rückzuganordnung (59, 60) durch einen Antrieb des Antriebsmoduls aktuierbar ist.

13. Chirurgisches Instrument nach einem der vorhergehenden Ansprüche 4 bis 12, **dadurch gekennzeichnet, dass** die Antriebseinheit ein, insbesondere konvergentes und/oder bewegliches, Verdrängungsmittel (180a; 181a, 181b) zum Verdrängen einer Eintriebsgliedanordnung (45.2) eines Instrumentenschafts (31) bei einem Einführen der Antriebseinheit in eine Aufnahme (80) des Instrumentenschaftes aufweist.

14. Chirurgisches Instrument nach einem der vorhergehenden Ansprüche 4 bis 13, **dadurch gekennzeichnet, dass** die Abtriebsgliedanordnung (45.1) in einer Vertiefung (143) angeordnet ist.


**Claims**

1.  An instrument shaft (31) with an input drive member assembly (45.2) comprising at least one movable input drive member (37, 38) for actuating a degree of freedom of the instrument shaft which movable input drive member (37, 38) projects, at least substantially, perpendicularly to a longitudinal axis of the instrument shaft towards a receptacle (80) of the instrument shaft for a drive unit (31), wherein the receptacle comprises a guide (152, 153; 160, 161; 165, 166) and / or an insertion opening (140), in particular a closable (170) insertion opening (140), for insertion of a drive unit (30) in an insertion direction which is in particular, at least substantially, perpendicular or parallel to the longitudinal axis of the instrument shaft.

2.  The instrument shaft according to the preceding claim, **characterised in that** the receptacle is movable, in particular pivotable, relative to a longitudinal axis of the instrument shaft.

3.  The instrument shaft according to any one of the preceding claims, **characterised in that** the input drive member assembly is arranged in a recess (142; 164).

4.  A surgical instrument comprising

    - an instrument shaft (31) according to any one of the preceding claims, and
    - a drive unit (30) which can be releasably connected to the instrument shaft and which comprises a housing (49) and at least one drive module (47; 47a, 47b) with a drive and an output drive member assembly (45.1) with at least one movable output drive member (34, 35) for actuating a degree of freedom of the instrument shaft (31).

5.  The surgical instrument according to the preceding claim, **characterised in that** the coupling direction, in which the drive module is movably supported in the housing of the drive unit and in which it is biased, includes an angle with a longitudinal axis of the instrument shaft, the absolute value of which angle is greater than 0°, in particular greater than 45°, in particular at least substantially 90°.

6.  The surgical instrument according to any one of the preceding claims 4 to 5, **characterised by** a drive unit locking assembly (150, 151; 152, 153; 167; 170) for locking the drive unit in a receptacle (80) of the instrument shaft.

7.  The surgical instrument according to any one of the preceding claims 4 to 6, **characterised by** an interface for attachment to a robot (40).

8. The surgical instrument according to any one of the preceding claims 4 to 7, **characterised in that** the drive module (47; 47a, 47b) is movably supported and biased in the housing in a coupling direction.

9. The surgical instrument according to any one of the preceding claims 4 to 8, **characterised in that** the drive unit comprises a magnet assembly (100, 101, 103, 104) for biasing the drive module in the coupling direction.

10. The surgical instrument according to any one of the preceding claims 4 to 9, **characterised in that** the drive unit comprises a retraction assembly (59, 60), in particular a mechanical and / or magnetic retraction assembly (59, 60), for retracting the drive module against the bias.

11. The surgical instrument according to the preceding claim, **characterised in that** the drive unit comprises a drive module locking assembly (61; 103, 104) for locking the retracted drive module.

12. The surgical instrument according to any one of the preceding claims 10 to 11, **characterised in that** the retraction assembly (59, 60) can be actuated by a drive of the drive module.

13. The surgical instrument according to any one of the preceding claims 4 to 12, **characterised in that** the drive unit comprises a displacement means (180a; 181a, 181b), in particular a convergent and / or a movable displacement means (180a; 181a, 181b), for displacing an input drive member assembly (45.2) of an instrument shaft (31) upon insertion of the drive unit into a receptacle (80) of the instrument shaft.

14. The surgical instrument according to any one of the preceding claims 4 to 13, **characterised in that** the output drive member assembly (45.1) is arranged in a recess (143).


**Revendications**

1. Arbre d'instrument (31) avec un agencement d'organe d'enfoncement (45.2) avec au moins un organe d'enfoncement mobile (37, 38) pour l'actionnement d'un degré de liberté de l'arbre d'instrument, lequel fait saillie, au moins sensiblement, perpendiculairement à l'axe longitudinal de l'arbre d'instrument vers un logement (80) de l'arbre d'instrument pour une unité d'entraînement (31), dans lequel le logement présente un guidage (152, 153 ; 160, 161 ; 165, 166) et/ou une ouverture d'introduction (140), en particulier pouvant être fermée (170), pour l'introduction d'une unité d'entraînement (30) dans une direction d'introduction, laquelle est en particulier, au moins sensiblement, perpendiculaire ou parallèle à l'axe longitudinal de l'arbre d'instrument.

2. Arbre d'instrument selon la revendication précédente, **caractérisé en ce que** le logement est mobile, en particulier pivotant, par rapport à un axe longitudinal de l'arbre d'instrument.

3. Arbre d'instrument selon l'une quelconque des revendications précédentes, **caractérisé en ce que** l'agencement d'organe d'enfoncement est agencé dans un évidement (142 ; 164).

4. Instrument chirurgical avec

   - un arbre d'instrument (31) selon l'une quelconque des revendications précédentes, et
   - une unité d'entraînement (30) pouvant être reliée de manière amovible à l'arbre d'instrument avec un boîtier (49) et au moins un module d'entraînement (47 ; 47a, 47b) avec un entraînement et un agencement d'organe de sortie (45.1) avec au moins un organe de sortie mobile (34, 35) pour l'actionnement d'un degré de liberté de l'arbre d'instrument (31).

5. Instrument chirurgical selon la revendication précédente, **caractérisé en ce que** la direction de couplage, dans laquelle le module d'entraînement est logé mobile dans le boîtier de l'unité d'entraînement et précontraint, forme avec un axe longitudinal de l'arbre d'instrument un angle dont la valeur est supérieure à 0°, en particulier supérieure à 45°, en particulier au moins sensiblement 90°.

6. Instrument chirurgical selon l'une quelconque des revendications précédentes 4 à 5, **caractérisé par** un agencement de verrouillage d'unité d'entraînement (150, 151 ; 152, 153 ; 167 ; 170) pour le verrouillage de l'unité d'entraînement

EP 2 942 027 B1

dans un logement (80) de l'arbre d'instrument.

7. Instrument chirurgical selon l'une quelconque des revendications précédentes 4 à 6, **caractérisé par** une interface pour la fixation à un robot (40).

8. Instrument chirurgical selon l'une quelconque des revendications précédentes 4 à 7, **caractérisé en ce que** le module d'entraînement (47 ; 47a, 47b) est logé mobile dans le boîtier dans une direction de couplage et précontraint.

9. Instrument chirurgical selon l'une quelconque des revendications précédentes 4 à 8, **caractérisé en ce que** l'unité d'entraînement présente un agencement d'aimant (100, 101, 103, 104) pour la précontrainte du module d'entraînement dans la direction de couplage.

10. Instrument chirurgical selon l'une quelconque des revendications précédentes 4 à 9, **caractérisé en ce que** l'unité d'entraînement présente un agencement de retrait, en particulier mécanique et/ou magnétique, (59, 60) pour le retrait du module d'entraînement contre la précontrainte.

11. Instrument chirurgical selon la revendication précédente, **caractérisé en ce que** l'unité d'entraînement présente un agencement de verrouillage de module d'entraînement (61 ; 103, 104) pour le verrouillage du module d'entraînement retiré.

12. Instrument chirurgical selon l'une quelconque des revendications précédentes 10 à 11, **caractérisé en ce que** l'agencement de retrait (59, 60) est actionnable par un entraînement du module d'entraînement.

13. Instrument chirurgical selon l'une quelconque des revendications précédentes 4 à 12, **caractérisé en ce que** l'unité d'entraînement présente un moyen de déplacement, en particulier convergent et/ou mobile, (180a ; 181a ; 181b) pour le déplacement d'un agencement d'organe d'enfoncement (45.2) d'un arbre d'instrument (31) lors d'une introduction de l'unité d'entraînement dans un logement (80) de l'arbre d'instrument.

14. Instrument chirurgical selon l'une quelconque des revendications précédentes 4 à 13, **caractérisé en ce que** l'agencement d'organe de sortie (45.1) est agencé dans un évidement (143).

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 17

Fig. 16

Fig. 18

Fig. 19

Fig. 20

Fig. 21

Fig. 22

Fig. 23

Fig. 24

(a)                    (b)                    (c)

200
100
3

100.1

100.2

100.6

**Fig. 25**

(a)                              2

10A (100)

1

(c)
1                                2

10A (100)

(b)

2
10A (100)                        1

**Fig. 26**

(a)    (b)    (c)

**Fig. 27**

**Fig. 28**

**Fig. 29**

(a)                    (b)                    (c)

20A
(200; 2000)

3.2

3

10A
(100; 1000)

20A
(200; 2000)

3.3

3

10A
(100; 1000)

20A
(200; 2000)

3

3.4

10A
(100; 1000)

## Fig. 30

(a)                    (b)                    (c)

20A
(200; 2000)

3.5

10A
(100; 1000)

20A
(200; 2000)

3.5

10A
(100; 1000)

20A
(200; 2000)

3.5

3.5

10A
(100; 1000)

## Fig. 31

(a)

(b)

11 (21)

11 (21)

3.7

3.7

3

3

3.6

3.6

10A
(100; 1000;
20A;
200; 2000)

## Fig. 32

1

3

4

100

3.3

3(1)

4

2

2

## Fig. 33

Fig. 35

Fig. 36

Fig. 34

Fig. 37

Fig.38

Fig. 33

Fig. 40A

EP 2 942 027 B1

Fig. 40B

Fig. 41

EP 2 942 027 B1

Fig. 4

Fig. 43

Fig. 4

(a)

(b)

Fig. 45

Fig. 46

# Fig. 47

# Fig. 48A

# Fig. 48B

# Fig. 49

## Fig. 50

## Fig. 51A

## Fig. 51B

# Fig. 52

# Fig. 53A

# Fig. 53B

# Fig. 54

## Fig. 55A

32

34'

33'

31'

30'

340

## Fig. 55B

34'

340

32'

30'

31'

Fig. 55C

Fig. 55D

**EP 2 942 027 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011143022 A1
- WO 2010123231 A2
- WO 2011037394 A
- EP 1015068 A1
- DE 102009060987 A1
- WO 2009079301 A1